# EUROPEAN PATENT SPECIFICATION

(11) **EP 1 742 642 B1**
(45) Date of publication and mention of the grant of the patent: **15.10.2008**
(21) Application number: 05804767.1
(22) Date of filing: 11.04.2005
(51) Int. Cl.: A61K 31/66, A61K 31/662, A61K 31/675, A61K 31/665, A61P 31/18, C07D 471/04, C07D 487/04, C07F 9/6561

(54) **PHOSPHONATE ANALOGS OF HIV INTEGRASE INHIBITOR COMPOUNDS**
PHOSPHONATANALOGA VON HIV-INTEGRASEHEMMERVERBINDUNGEN
ANALOGUES DE PHOSPHONATE DE COMPOSES INHIBITEURS DE L'INTEGRASE DU VIH

(30) Priority: 14.04.2004 US 562678 P
(43) Date of publication of application: 17.01.2007
(73) Proprietor: GILEAD SCIENCES, INC., Foster City, California 94404 (US); Cai, Zhenhong R., Foster City CA 94404 (US); Chen, Xiaowu, San Mateo, CA 94403 (US); Fardis, Maria, San Carlos, CA 94070 (US); Jabri, Salman Y., San Francisco CA 94127 (US); Jin, Haolun, Foster City CA 94404 (US); Kim, Choung U., San Carlos CA 94070 (US); Metobo, Sanuel E., Newark CA 94560 (US); Mish, Michael R., Foster City CA 94404 (US); Pastor, Richard M., San Francisco CA 94110 (US)
(72) Inventor: CAI, Zhenhong, R., Foster City, CA 94404 (US); CHEN, Xiaowu, San Mateo, California 94403 (US); FARDIS, Maria, San Carlos, California 94070 (US); JABRI, Salman, Y., San Francisco, CA 94127 (US); JIN, Haolun, Foster City, CA 94404 (US); KIM, Choung, U., San Carlos, California 94070 (US); METOBO, Sanuel, E., Newark, CA 94560 (US); MISH, Michael, R., Foster City, CA 94404 (US); PASTOR, Richard, M., San Francisco, CA 94110 (US)
(74) Representative: Reitstötter - Kinzebach
(86) International application number: PCT/US2005/012520
(87) International publication number: WO 2005/117904

(56) References cited:
- WO-A-03/090690
- WO-A-03/091264
- WO-A-20/04035576
- WO-A-20/04035577
- DE-A1- 19 738 005
- ABU SHEIKA G ET AL: "EFFECT OF ACYCLIC NUCLEOSIDE PHOSPHONATES ON THE HIV-1 INTEGRASE IN VITRO" NUCLEOSIDES & NUCLEOTIDES, MARCEL DEKKER, INC, US, vol. 18, no. 4/5, April 1999 (1999-04), pages 849-851, XP008055903 ISSN: 0732-8311
- TRAMONTANO E ET AL: "INHIBITION OF HIV-1 INTEGRASE BY ACYCLIC NUCLEOSIDE PHOSPHONATES" PROCEEDINGS OF THE 90TH ANNUAL MEETING OF THE AMERICAN ASSOCIATION FOR CANCER RESEARCH. PHILADELPHIA, PA, APRIL 10 - 14, 1999, PROCEEDINGS OF THE ANNUAL MEETING OF THE AMERICAN ASSOCIATION FOR CANCER RESEARCH, PHILADELPHIA, PA : AACR, US, vol. VOL. 40, March 1999 (1999-03), page 19, XP008055902

## Description

### FIELD OF THE INVENTION

The invention relates generally to phosphonate compounds with antiviral activity and more specifically with anti-HIV integrase properties.

### BACKGROUND OF THE INVENTION

AIDS is a major public health problem worldwide. Despite the unprecedented successes in the therapy of HIV infection, AIDS remains a major world health problem being the first cause of death in Africa and the fourth leading cause of death worldwide. Rapid emergence of drug-resistant HIV variants and severe side effects limit the efficacy of existing therapies. Although drugs targeting HIV viruses are in wide use and have shown effectiveness, toxicity and development of resistant strains have limited their usefulness. Assay methods capable of determining the presence, absence or amounts of HIV viruses are of practical utility in the search for inhibitors as well as for diagnosing the presence of HIV.

Human immunodeficiency virus (HIV) infection and related disease is a major public health problem worldwide. The retrovirus human immunodeficiency virus type 1 (HIV-1), a member of the primate lentivirus family (De Clercq E (1994) Annals of the New York Academy of Sciences, 724:438-456; Barre-Sinoussi F (1996) Lancet, 348:31-35), is generally accepted to be the causative agent of acquired immunodeficiency syndrome (AIDS) Tarrago et al FASEB Journal 1994, 8:497-503). AIDS is the result of repeated replication of HIV-1 and a decrease in immune capacity, most prominently a fall in the number of CD4+ lymphocytes. The mature virus has a single stranded RNA genome that encodes 15 proteins (Frankel et al (1998) Annual Review of Biochemistry, 67:1-25; Katz et al (1994) Annual Review of Biochemistry, 63:133-173), including three key enzymes: (i) protease (Prt) (von der Helm K (1996) Biological Chemistry, 377:765-774); (ii) reverse transcriptase (RT) (Hottiger et al (1996) Biological Chemistry Hoppe-Seyler, 377:97-120), an enzyme unique to retroviruses; and (iii) integrase (Asante et al (1999) Advances in Virus Research 52:351-369; Wlodawer A (1999) Advances in Virus Research 52:335-350; Esposito et al (1999) Advances in Virus Research 52:319-333). Protease is responsible for processing the viral precursor polyproteins, RT is the key enzyme in the replication of the viral genome, and integrase, a viral encoded protein, is responsible for the integration of the double stranded DNA form of the viral genome into host DNA.

Until 1995, the only drugs approved in the United States were nucleoside inhibitors of RT (Smith et al (1994) Clinical Investigator, 17:226-243). Since then, two new classes of agents, protease inhibitors (PI) and non-nucleoside RT inhibitors (NNRTI), and more than a dozen new drugs have been approved (Johnson et al (2000) Advances in Internal Medicine, 45 (1-40; Porche DJ (1999) Nursing Clinics of North America, 34:95-112). There are now three classes of drugs available: (1) the original nucleoside RT inhibitors, (2) protease inhibitors (PI), and (3) the non-nucleoside RT inhibitors (NNRTI).

An essential step in HIV infection is the integration of the viral genome into the host cell chromosomes within the nucleus. Unlike other retroviruses, HIV can transport its genetic material, in the form of the large nucleoprotein pre-integration complex (PIC), into the nucleus through the intact nuclear envelope and infect non-dividing cells such as macrophages and microglial cells. Although several different components of the PIC have been implicated in its nuclear import, the mechanism of nuclear entry remains unclear (Piller et al (2003) Current Drug Targets 4:409-429; Debyser et al (2002) Antiviral Chemistry & Chemotherapy 13:1-15). Specifically inhibiting PIC nuclear import would likely block HIV infection in non-dividing cells, this important step of HIV replication is of great interest as a drug target. The identification of compounds unambiguously affecting HIV replication by targeting integrase supports the potential of this crucial viral enzyme as a drug target. Certain HIV integrase inhibitors have been disclosed which block integration in extracellular assays and exhibit antiviral effects against HIV-infected cells (Anthony, et al WO 02/30426; Anthony, et al WO 02/30930; Anthony, et al WO 02/30931; WO 02/055079 A2 A3; Zhuang, et al WO 02/36734; US 6395743; US 6245806; US 6271402; Fujishita, et al WO 00/039086; Uenaka et al WO 00/075122; Selnick, et al WO 99/62513; Young, et al WO 99/62520; Payne, et al WO 01/00578; Parrill, A.L. (2003) Current Medicinal Chemistry 10:1811-1824; Gupta et al (2003) Current Medicinal Chemistry 10:1779-1794; Maurin et al (2003) Current Medicinal Chemistry 10:1795-1810; Jing, et al Biochemistry (2002) 41:5397-5403; Pais, et al Jour. Med Chem. (2002) 45:3184-94; Goldgur, et al Proc. Natl. Acad Sci. U.S.A. (1999) 96:13040-13043; Espeseth, et al Proc. Natl. Acad. Sci. U.S.A. (2000) 97:11244-11249 ;WO 2004/035576; DE 19738005 A1; Nucleosides 8 Nucleotides, 18 (485), 849-851(1999); E.Tramontana et al., Proceeding of the Annuals Meeting of the American Association for Cancer Research, Philadelphia PA: AACR, US, Vol. 40, March 1999, page 19, XP 0080 55902). For review, see: Neamati (2002) Expert Opin. Ther. Patents 12(5):709-724; Pommier et al (1999) Advances in Virus Research 52:427-458; Young (2001) Current Opinion in Drug Discovery & Development 4(4):402-410; Neamati et al (2001) "Human Immunodeficiency Virus type 1 Integrase Targeted Inhibitor Design", Antiretroviral Therapy, E. De Clercq, Ed., ASM Press, Washington, DC; Pani et al (2000) Current Pharm. Design 6:569-584; Pommier et al (2000) Antiviral Res. 47(3):139-148; De Clercq E. (2002) Medicinal Research Reviews 22(6):531-565.

HIV integrase inhibitory compounds with improved antiviral and pharmacokinetic properties are desirable; including enhanced activity against development of HIV resistance, improved oral bioavailability, greater potency and extended effective half-life *in vivo* (Nair, V. "HIV integrase as a target for antiviral chemotherapy" Reviews in Medical Virology (2002) 12(3):179-193). Three-dimensional quantitative structure-activity relationship studies and docking simulations (Buolamwini, et al Jour. Med. Chem. (2002) 45:841-852) of conformationally-restrained cinnamoyl-type integrase inhibitors (Artico, et al Jour. Med. Chem. (1998) 41:3948-3960) have correlated hydrogen-bonding interactions to the inhibitory activity differences among the compounds.

Phase II clinical studies candidate, S-1360 (Shionogi-GlaxoSmithKline Pharmaceuticals LLC) is the furthest advanced HIV integrase inhbitor to date. Animal toxicity studies have been reported for other candidates, L-731,988 and L-708,906, by Merck.

There is a need for anti-HIV therapeutic agents, i.e, drugs having improved antiviral and pharmacokinetic properties with enhanced activity against development of HIV resistance, improved oral bioavailability, greater potency and extended effective half-life *in vivo.* New HIV inhibitors should be active against mutant HIV strains, have distinct resistance profiles, fewer side effects, less complicated dosing schedules, and orally active. In particular, there is a need for a less onerous dosage regimen, such as one pill, once per day. Although drugs targeting HIV protease are in wide use and have shown effectiveness, particularly when employed in combination, toxicity and development of resistant strains have limited their usefulness (Palella, et al N. Engl. J. Med. (1998) 338:853-860; Richman, D. D. Nature (2001) 410:995-1001).

Combination therapy with HIV inhibitors has proven to be highly effective in suppressing viral replication to unquantifiable levels for a sustained period of time. Also, combination therapy with RT and protease inhibitors have shown synergistic effects in suppressing HIV replication. Unfortunately, many patients currently fail combination therapy due to the development of drug resistance, non-compliance with complicated dosing regimens, pharmacokinetic interactions, toxicity, and lack of potency. Therefore, there is a need for HIV integrase inhibitors that are synergistic in combination with other HIV inhibitors, or show chemical stability in combination formulations.

Improving the delivery of drugs and other agents to target cells and tissues has been the focus of considerable research for many years. Though many attempts have been made to develop effective methods for importing biologically active molecules into cells, both *in vivo* and *in vitro,* none has proved to be entirely satisfactory. Optimizing the association of the inhibitory drug with its intracellular target, while minimizing intercellular redistribution of the drug, e.g. to neighboring cells, is often difficult or inefficient.

Most agents currently administered to a patient parenterally are not targeted, resulting in systemic delivery of the agent to cells and tissues of the body where it is unnecessary, and often undesirable. This may result in adverse drug side effects, and often limits the dose of a drug (e.g., cytotoxic agents and other anti-cancer or anti-viral drugs) that can be administered. By comparison, although oral administration of drugs is generally recognized as a convenient and economical method of administration, oral administration can result in either (a) uptake of the drug through the cellular and tissue barriers, e.g. blood/brain, epithelial, cell membrane, resulting in undesirable systemic distribution, or (b) temporary residence of the drug within the gastrointestinal tract. Accordingly, a major goal has been to develop methods for specifically targeting agents to cells and tissues. Benefits of such treatment includes avoiding the general physiological effects of inappropriate delivery of such agents to other cells and tissues, such as uninfected cells. Intracellular targeting may be achieved by methods and compositions, including prodrugs (Krise et al (1996) Advanced Drug Delivery Reviews 19:287-310), which allow accumulation or retention of biologically active agents inside cells.

### SUMMARY OF THE INVENTION

The present invention provides novel compounds with HIV integrase activity, i.e. novel human retroviral integrase inhibitors. Therefore, the compounds of the invention may inhibit retroviral integrases and thus inhibit the replication of the virus. They are useful for treating human patients infected with a human retrovirus, such as human immunodeficiency virus (strains of HIV-1 or HIV-2) or human T-cell leukemia viruses (HTLV-I or HTLV-II) which results in acquired immunodeficiency syndrome (AIDS) and/or related diseases. The present invention includes novel phosphonate HIV integrase inhibitor compounds and phosphonate analogs of known experimental integrase inhibitors. The compounds of the invention optionally provide cellular accumulation as set forth below.

The present invention relates generally to the accumulation or retention of therapeutic compounds inside cells. The invention is more particularly related to attaining high concentrations of phosphonate-containing molecules in HIV infected cells. Intracellular targeting may be achieved by methods and compositions which allow accumulation or retention of biologically active agents inside cells. Such effective targeting may be applicable to a variety of therapeutic formulations and procedures.

Compositions of the invention include new HIV integrase inhibitor compounds having at least one phosphonate group. The compositions of the invention thus include all known approved, experimental, and proposed HIV integrase inhibitors, that do not already comprise a phosphonate group, with at least one phosphonate group covalently attached. Experimental HIV integrase inhibitors include those reviewed in: Dayam et al (2003) Current Pharmaceutical Design 9:1789-1802; De Clercq E. (2002) Biochimica et Biophysica Acta 1587(2-3):258-275; Nair, V. (2002) Reviews in Medical Virology 12(3):179-193; Neamati, N. (2002) Expert Opinion on Therapeutic Patents 12(5):709-724; Asante-Appiah et al (1997) Antiviral Res. 36:139-156; Dubrovsky et al (1995) Mol. Med. 1:217-230; Popov et al (1998) EMBO J. 17:909-917; Farnet et al (1996) AIDS 10(Suppl. A):S3-S 11; Hansen et al (1998) Genet. Eng. 20:41-61; Bukrinsky, M. (1997) Drugs Future 22:875-883; Neamati et al (2000) Adv. Pharmacol. 49:147-163; Pommier et al (1999) Adv. Virus Res. 52:427-458; Neamati et al (1997) Drug Discovery Today 2:487-498; Nicklaus et al (1997) J. Med Chem. 40:920-929; Pommier et al (1997) Antivir. Chem. Chemother. 8:483-503; Robinson J. (1998) Infect. Med. 15:129-137; Thomas et al (1997) Trends Biotechnol. 15:167-172.

The invention includes the following HIV integrase inhibitors

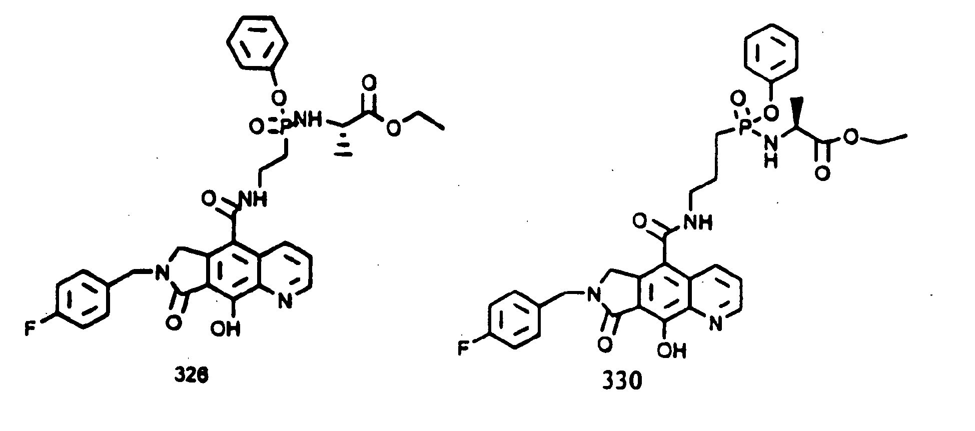

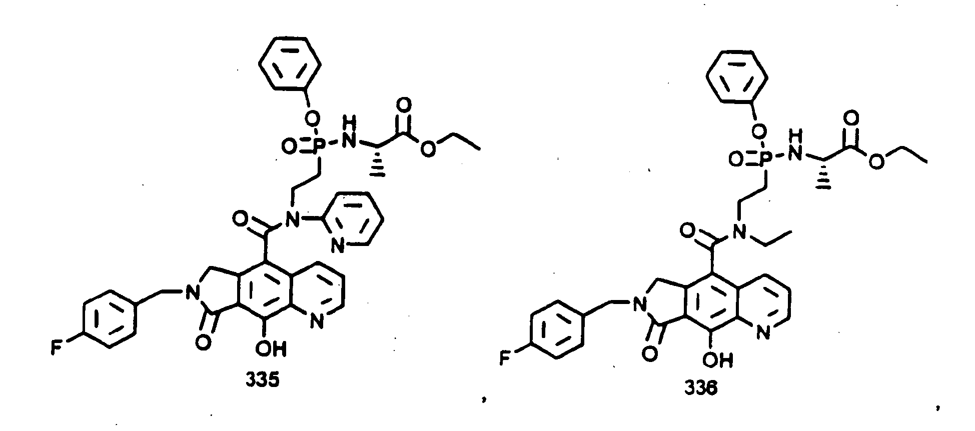

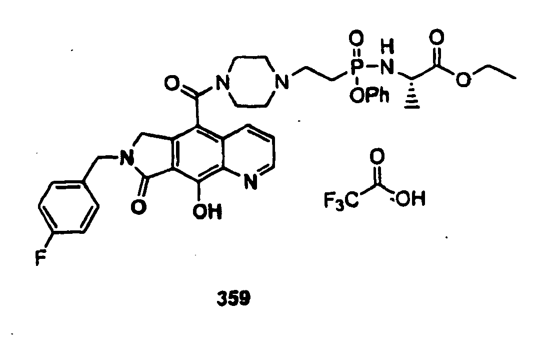

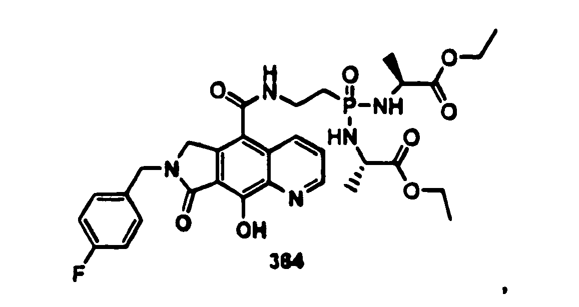

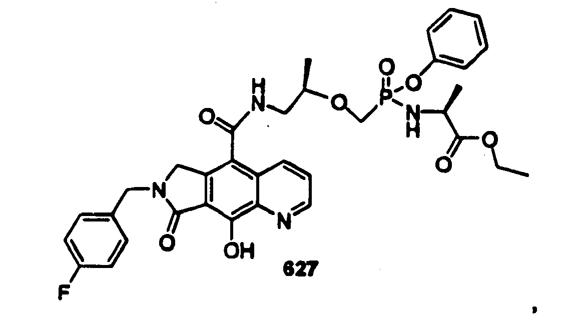

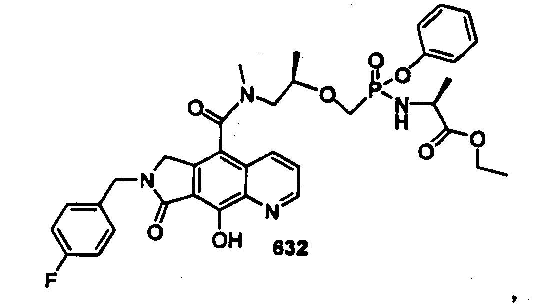

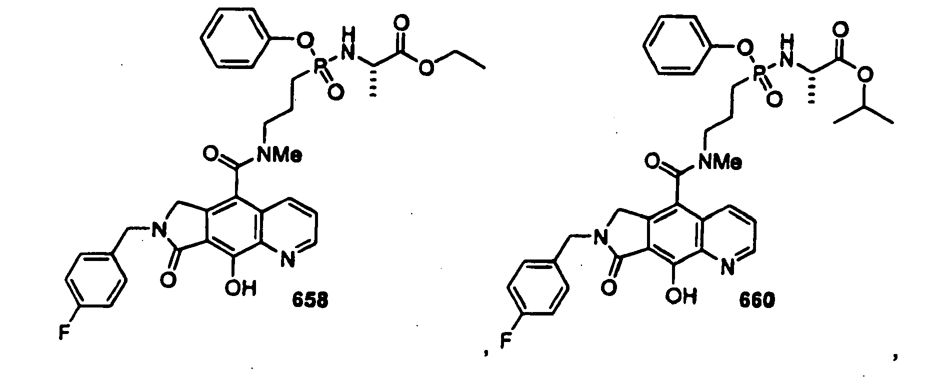

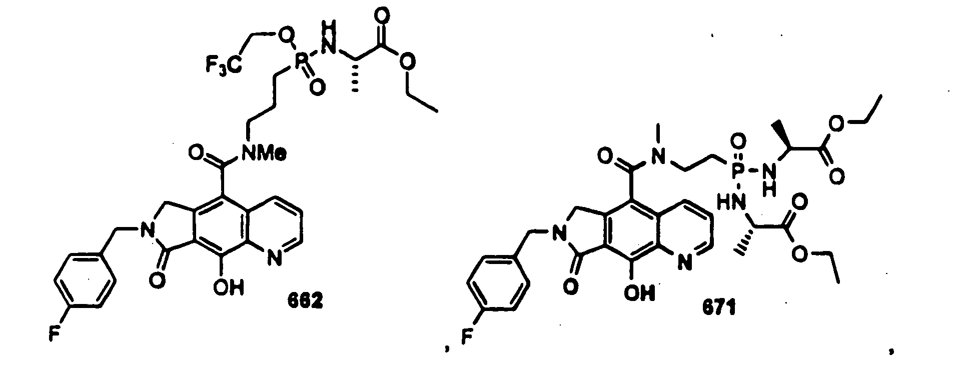

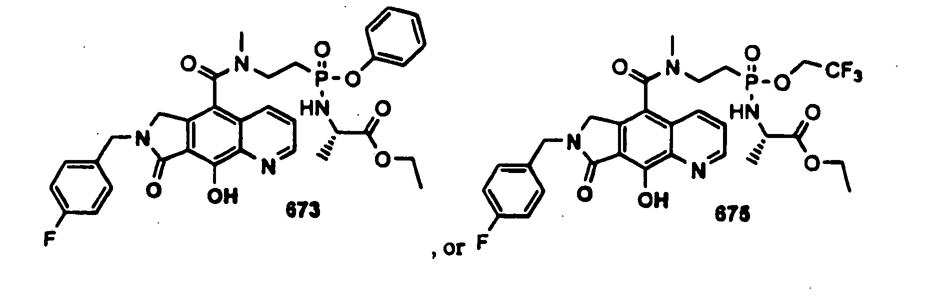

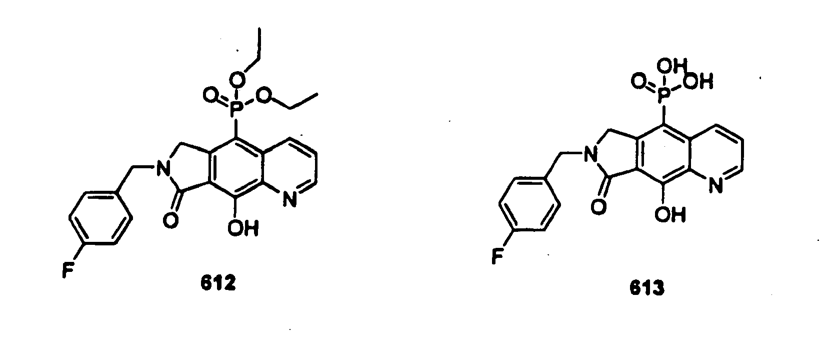

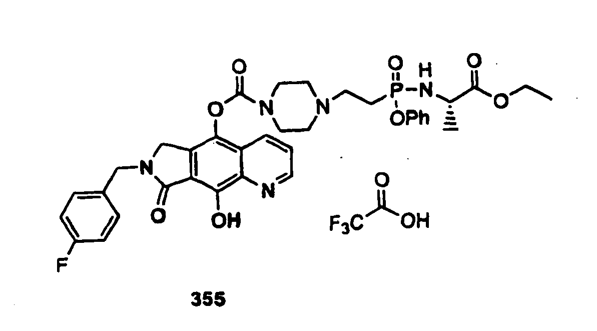

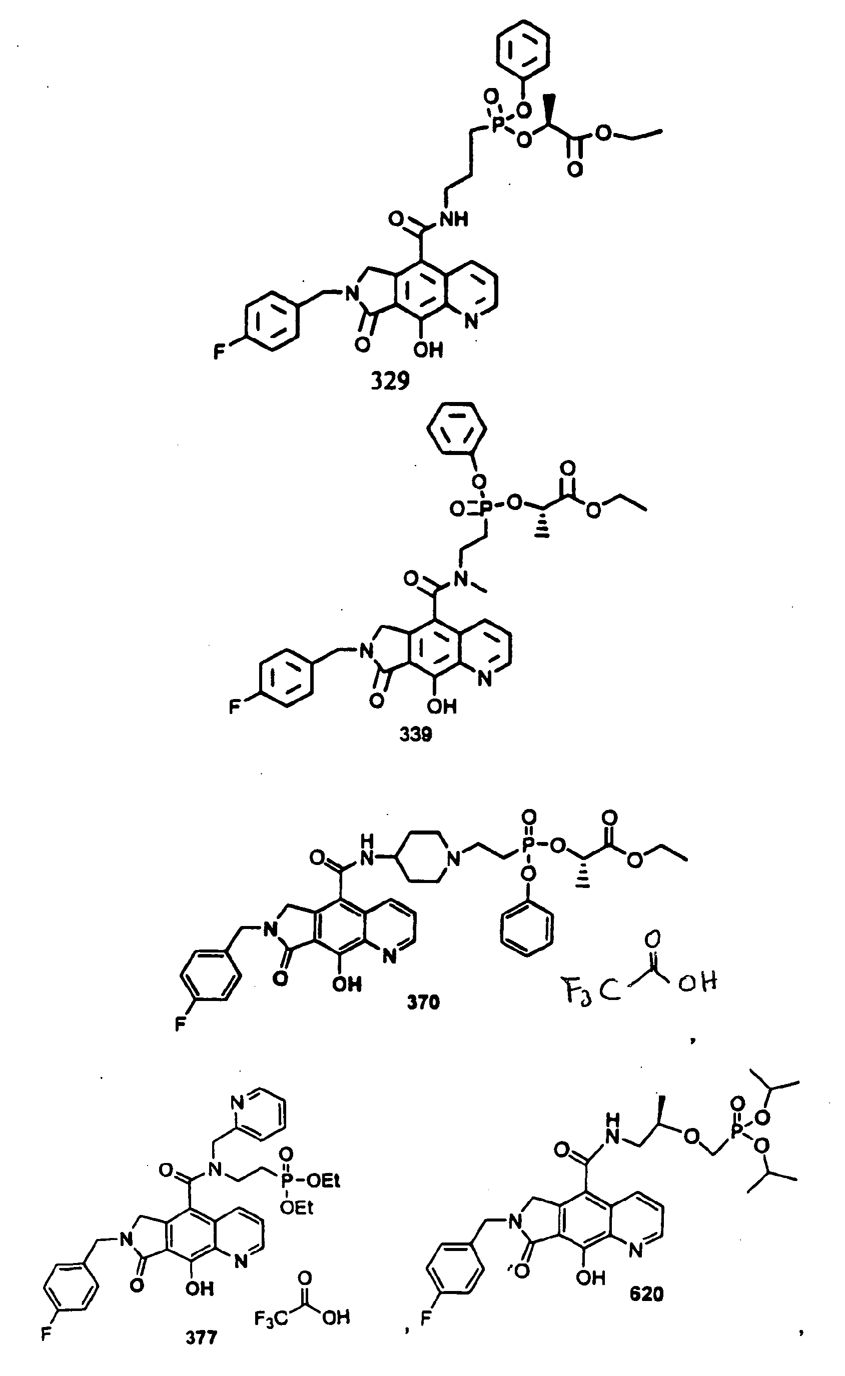

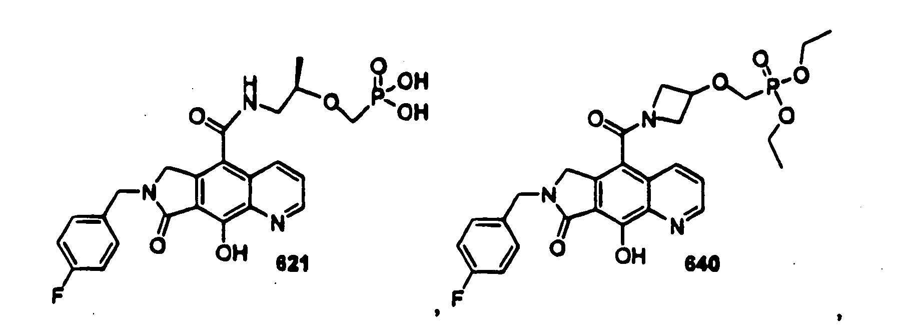

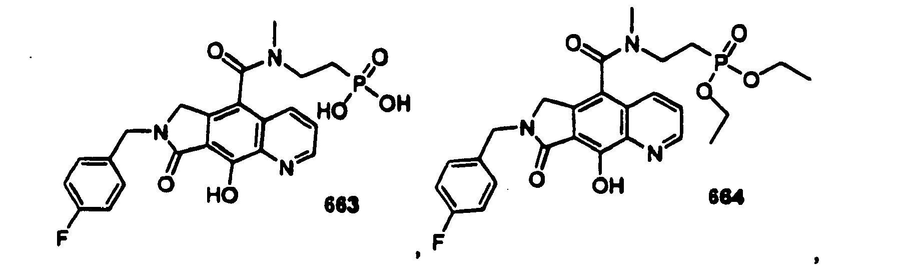

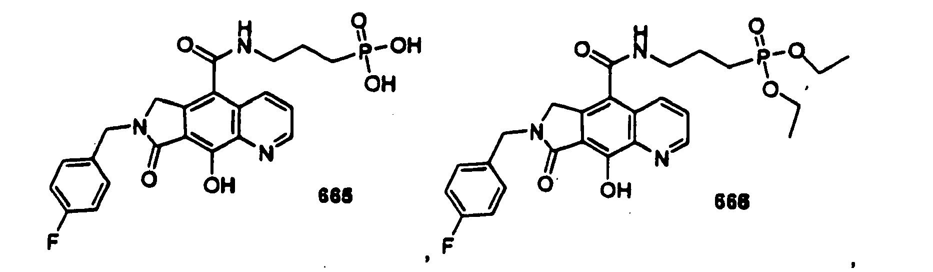

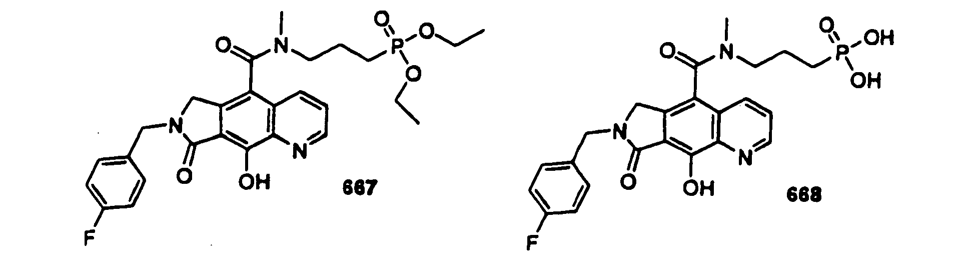

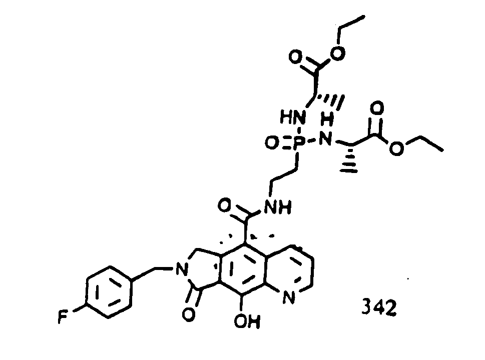

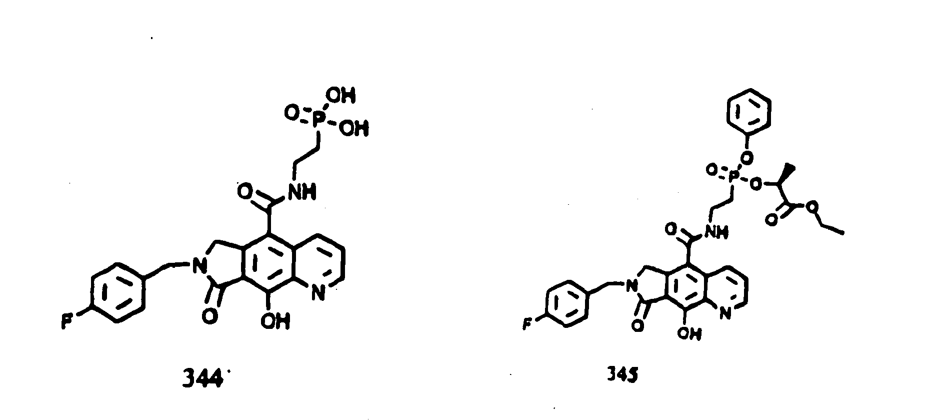

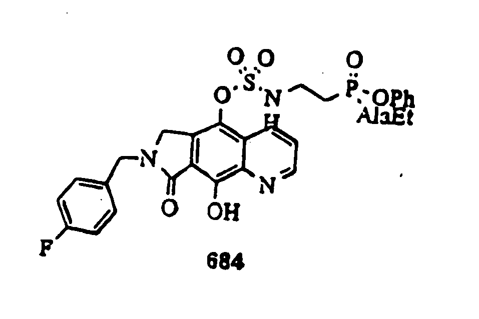

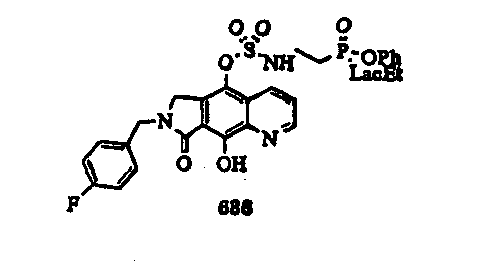

The invention includes pharmaceutically acceptable salts of said compounds and all enol and tautomeric resonance isomers thereof. Except where the stereochemistry is explicit, the compounds of the invention include all stereoisomers; i.e. each enantiomer, diastereomer, and atropisomer in purified form, or racemic and isomerically enriched mixtures.

The invention provides a pharmaceutical composition comprising an effective amount of said compounds, or a pharmaceutically acceptable salt thereof, in a formulation, i.e. in combination with a pharmaceutically acceptable excipient, diluent or carrier.

The invention includes combination formulations including the compounds of the invention, with other active ingredients that treat or prevent HIV infections. Such combination formulations may be a fixed dose of two or more active ingredients, including at least one compound of the invention.

The use of the compounds of the invention in an HIV infected patient, or in a sample suspected of containing HIV, anticipates all metabolites of the compounds so administered which occur by solvolysis, hydrolysis, photolysis, or by enzymatic action which converts or degrades the administered compound into, e.g. an activated form, an incorporated form, a cleaved form, or a metabolite for excretion.

The invention also provides a compound as defined above for use in medical therapy, as well as the use of such a compound for the manufacture of a medicament useful for (1) the treatment of AIDS or ARC (AIDs related complex); or (2) the prophylaxis of infection by HIV.

Other aspects of the invention are formulation compositions of the compounds of the invention, as well as methods of formulating the compositions.

### DETAILED DESCRIPTION OF EXEMPLARY EMBODIMENTS

Reference will now be made in detail to certain embodiments of the invention, examples of which are illustrated in the accompanying structures and formulas.

### DEFINITIONS

Unless stated otherwise, the following terms and phrases as used herein are intended to have the following meanings:

When tradenames are used herein, applicants intend to independently include the tradename product and the active pharmaceutical ingredient(s) of the tradename product.

The terms "phosphonate" and "phosphonate group" mean a functional group or moiety within a molecule that comprises at least one phosphorus-carbon bond, and at least one phosphorus-oxygen double bond. The phosphorus atom is further substituted with oxygen, sulfur, or nitrogen substituents. These substituents may be part of a prodrug moiety. As defined herein, "phosphonate" and "phosphonate group" include moieties with phosphonic acid, phosphonic monoester, phosphonic diester, phosphonamidate, and phosphonthioate functional groups.

The term "prodrug" as used herein refers to any compound that when administered to a biological system generates the drug substance, i.e. active ingredient, as a result of spontaneous chemical reaction(s), enzyme catalyzed chemical reaction(s), photolysis, and/or metabolic chemical reaction(s). A prodrug is thus a covalently modified analog or latent form of a therapeutically-active compound. For a review of phosphorus prodrugs, see: Krise et al (1996) Advanced Drug Delivery Reviews 19:287-310.

"Pharmaceutically acceptable prodrug" refers to a compound that is metabolized in the host, for example hydrolyzed or oxidized, by either enzymatic action or by general acid or base solvolysis, to form an active ingredient, Typical examples of prodrugs of the compounds of the invention have biologically labile protecting groups on a functional moiety of the compound. Prodrugs include compounds that can be oxidized, reduced, aminated, deaminated, esterified, deesterified, alkylated, dealkylated, acylated, deacylated, phosphorylated, dephosphorylated, photolyzed, hydrolyzed, or other functional group change or conversion involving forming or breaking chemical bonds on the prodrug.

"Prodrug moiety" means a labile functional group which separates from the active inhibitory compound during metabolism, systemically, inside a cell, by hydrolysis, enzymatic cleavage, or by some other process (Bundgaard, Hans, "Design and Application of Prodrugs" in Textbook of Drog Design and Development (1991), P. Krogsgaard-Larsen and H. Bundgaard, Eds. Harwood Academic Publishers, pp. 113-191). Enzymes which are capable of an enzymatic activation mechanism with the phosphonate prodrug compounds of the invention include, but are not limited to, amidases, esterases, microbial enzymes, phospholipases, cholinesterases, and phosphases. Prodrug moieties can serve to enhance solubility, absorption and lipophilicity to optimize drug delivery, bioavailability and efficacy.

Exemplary prodrug moieties include the hydrolytically sensitive or labile acyloxymethyl esters -CH₂OC(=O)R⁹ and acyloxymethyl carbonates -CH₂OC(=O)OR⁹ where R⁹ is C₁-C₆ alkyl, C₁-C₆ substituted alkyl, C₆-C₂₀ aryl or C₆-C₂₀ substituted aryl. The acyloxyalkyl ester was first used as a prodrug strategy for carboxylic acids and then applied to phosphates and phosphonates by Farquhar et al (1983) J. Pharm. Sci. 72: 324; also US Patent Nos. 4816570, 4968788, 5663159 and 5792756. In certain compounds of the invention, a prodrug moiety is part of a phosphonate group. Subsequently, the acyloxyalkyl ester was used to deliver phosphonic acids across cell membranes and to enhance oral bioavailability. A close variant of the acyloxyalkyl ester, the alkoxycarbonyloxyalkyl ester (carbonate), may also enhance oral bioavailability as a prodrug moiety in the compounds of the combinations of the invention. An exemplary acyloxymethyl ester is pivaloyloxymethoxy, (POM) -CH₂OC(=O)C(CH₃)₃. Exemplary acyloxymethyl carbonate prodrug moieties are pivaloyloxymethylcarbonate (POC) -CH₂OC(=O)OC(CH₃)₃ and (pivoxil) -CH₂OC(=O)OCH(CH₃)₂.

The phosphonate group may be a phosphonate prodrug moiety. The prodrug moiety may be sensitive to hydrolysis, such as, but not limited to a pivaloyloxymethyl carbonate (POC) or POM group. Alternatively, the prodrug moiety may be sensitive to enzymatic potentiated cleavage, such as a lactate ester or a phosphonamidate-ester group.

Aryl esters of phosphorus groups, especially phenyl esters, are reported to enhance oral bioavailability (DeLambert et al (1994) J. Med. Chem. 37: 498). Phenyl esters containing a carboxylic ester ortho to the phosphate have also been described (Khamnei and Torrence, (1996) J. Med Chem. 39:4109-4115)*.* Benzyl esters are reported to generate the parent phosphonic acid. In some cases, substituents at the *ortho-*or *para-*position may accelerate the hydrolysis. Benzyl analogs with an acylated phenol or an alkylated phenol may generate the phenolic compound through the action of enzymes, e.g. esterases, oxidases, etc., which in turn undergoes cleavage at the benzylic C-O bond to generate the phosphoric acid and the quinone methide intermediate. Examples of this class of prodrugs are described by Mitchell et al (1992) J. Chem. Soc. Perkin Trans. I2345; Brook et al WO 91/19721. Still other benzylic prodrugs have been described containing a carboxylic ester-containing group attached to the benzylic methylene (Glazier et al WO 91/19721). Thio-containing prodrugs are reported to be useful for the intracellular delivery of phosphonate drugs. These proesters contain an ethylthio group in which the thiol group is either esterified with an acryl group or combined with another thiol group to form a disulfide. Deesterification or reduction of the disulfide generates the free thio intermediate which subsequently breaks down to the phosphoric acid and episulfide (Puech et al (1993) Antiviral Res., 22:155-174; Benzaria et al (1996) J. Med Chem. 39:4958). Cyclic phosphonate esters have also been described as prodrugs of phosphorus-containing compounds (Erion et al, US Patent No. 6312662).

"Protecting group" refers to a moiety of a compound that masks or alters the properties of a functional group or the properties of the compound as a whole. The chemical substructure of a protecting group varies widely. One fimction of a protecting group is to serve as intermediates in the synthesis of the parental drug substance. Chemical protecting groups and strategies for protection/deprotection are well known in the art. See: "Protective Groups in Organic Chemistry", Theodora W. Greene (John Wiley & Sons, Inc., New York, 1991. Protecting groups are often utilized to mask the reactivity of certain functional groups, to assist in the efficiency of desired chemical reactions, e.g. making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

Protected compounds may also exhibit altered, and in some cases, optimized, properties *in vitro* and *in vivo*, such as passage through cellular membranes and resistance to enzymatic degradation or sequestration. In this role, protected compounds with intended therapeutic effects may be referred to as prodrugs. Another function of a protecting group is to convert the parental drug into a prodrug, whereby the parental drug is released upon conversion of the prodrug in *vivo.* Because active prodrugs may be absorbed more effectively than the parental drug, prodrugs may possess greater potency *in vivo* than the parental drug. Protecting groups are removed either *in vitro,* in the instance of chemical intermediates, or *in vivo,* in the case of prodrugs. With chemical intermediates, it is not particularly important that the resulting products after deprotection, e.g. alcohols, be physiologically acceptable, although in general it is more desirable if the products are pharmacologically innocuous.

Any reference to any of the compounds of the invention also includes a reference to a physiologically acceptable salt thereof. Examples of physiologically acceptable salts of the compounds of the invention include salts derived from an appropriate base, such as an alkali met al (for example, sodium), an alkaline earth (for example, magnesium), ammonium and NX₄⁺ (wherein X is C₁-C₄ alkyl). Physiologically acceptable salts of an hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound of an hydroxy group include the anion of said compound in combination with a suitable cation such as Na⁺ and NX₄⁺ (wherein X is independently selected from H or a C₁-C₄ alkyl group).

For therapeutic use, salts of active ingredients of the compounds of the invention will be physiologically acceptable, i.e. they will be salts derived from a physiologically acceptable acid or base. However, salts of acids or bases which are not physiologically acceptable may also find use, for example, in the preparation or purification of a physiologically acceptable compound. All salts, whether or not derived form a physiologically acceptable acid or base, are within the scope of the present invention.

### Intracellular Targetting

The phosphonate derivatives of the invention may cleave *in vivo* in stages after they have reached the desired site of action, i.e. inside a cell. One mechanism of action inside a cell may entail a first cleavage, e.g. by esterase, to provide a negatively-charged "locked-in" intermediate. Cleavage of a terminal ester grouping thus affords an unstable intermediate which releases a negatively charged "locked in" intermediate.

After passage inside a cell, intracellular enzymatic cleavage or modification of the phosphonate prodrug compound may result in an intracellular accumulation of the cleaved or modified compound by a "trapping" mechanism. The cleaved or modified compound may then be "locked-in" the cell by a significant change in charge, polarity, or other physical property change which decreases the rate at which the cleaved or modified compound can exit the cell, relative to the rate at which it entered as the phosphonate prodrug. Other mechanisms by which a therapeutic effect are achieved may be operative as well. Enzymes which are capable of an enzymatic activation mechanism with the phosphonate prodrug compounds of the invention include, but are not limited to, amidases, esterases, microbial enzymes, phospholipases, cholinesterases, and phosphatases.

In selected instances in which the drug is of the nucleoside type, such as is the case of zidovudine and numerous other antiretroviral agents, it is known that the drug is activated *in vivo* by phosphorylation. Such activation may occur in the present system by enzymatic conversion of the "locked-in" intermediate with phosphokinase to the active phosphonate diphosphate and/or by phosphorylation of the drug itself after its release from the "locked-in" intermediate as described above. In either case, the original nucleoside-type drug will be convened, via the derivatives of this invention, to the active phosphorylated species.

### Cellular Accumulation Embodiment

Another embodiment of the invention is directed toward compounds capable of accumulating in human PBMC (peripheral blood mononuclear cells). PBMC refer to blood cells having round lymphocytes and monocytes. Physiologically, PBMC are critical components of the mechanism against infection. PBMC may be isolated from heparinized whole blood of normal healthy donors or buffy coats, by standard density gradient centrifugation and harvested from the interface, washed (e.g. phosphate-buffered saline) and stored in freezing medium. PBMC may be cultured in multi-well plates. At various times of culture, supernatant may be either removed for assessment, or cells may be harvested and analyzed (Smith R. etal (2003) Blood 102(7):2532-2540).

Optionally, the compounds of this embodiment demonstrate improved intracellular half-life of the compounds or intracellular metabolites of the compounds in human PBMC when compared to analogs of the compounds not having the phosphonate or phosphonate prodrug. Typically, the half-life is improved by at least about 50%, more typically at least in the range 50-100%, still more typically at least about 100%, more typically yet greater than about 100%.

In another embodiment, the intracellular half-life of a metabolite of the compound in human PBMCs is improved when compared to an analog of the compound not having the phosphonate or phosphonate prodrug. In such embodiments, the metabolite may be generated intracellularly, e.g. generated within human PBMC. The metabolite may be a product of the cleavage of a phosphonate prodrug within human PBMCs. The phosphonate prodrug may be cleaved to form a metabolite having at least one negative charge at physiological pH. The phosphonate prodrug may be enzymatically cleaved within human PBMC to form a phosphonate having at least one active hydrogen atom of the form P-OH.

### Stereoisomers

The compounds of the invention may have chiral centers, e.g. chiral carbon, sulfur, or phosphorus atoms. The compounds of the invention thus include racemic mixtures of all stereoisomers, including enantiomers, diastereomers, and atropisomers. In addition, the compounds of the invention include enriched or resolved optical isomers at any or all asymmetric, chiral atoms. In other words, the chiral centers apparent from the depictions are provided as the chiral isomers or racemic mixtures. Both racemic and diastereomeric mixtures, as well as the individual optical isomers isolated or synthesized, substantially free of their enantiomeric or diastereomeric partners, are all within the scope of the invention. The racemic mixtures are separated into their individual, substantially optically pure isomers through well-known techniques such as, for example, the separation of diastereomeric salts formed with optically active adjuncts, e.g., acids or bases followed by conversion back to the optically active substances. In most instances, the desired optical isomer is synthesized by means of stereospecific reactions, beginning with the appropriate stereoisomer of the desired starting material.

The compounds of the invention can also exist as tautomeric isomers in certain cases. Although only one delocalized resonance structure may be depicted, all such forms are contemplated within the scope of the invention. For example, ene-amine tautomers can exist for purine, pyrimidine, imidazole, guanidine, amidine, and tetrazole systems and all their possible tautomeric forms are within the scope of the invention.

### Salts and Hydrates

the compositions of this invention optionally comprise salts of the compounds herein, especially pharmaceutically acceptable non-toxic salts containing, for example, Na⁺, Li⁺, K⁺, Ca⁺² and Mg⁺². Such salts may include those derived by combination of appropriate cations such as alkali and alkaline earth met al ions or ammonium and quaternary amino ions with an acid anion moiety, typically a carboxylic acid. Monovalent salts are preferred if a water soluble salt is desired.

Met al salts typically are prepared by reacting the met al hydroxide with a compound of this invention. Examples of met al salts which are prepared in this way are salts containing Li⁺, Na⁺, and K⁺. A less soluble met al salt can be precipitated from the solution of a more soluble salt by addition of the suitable met al compound.

In addition, salts may be formed from acid addition of certain organic and inorganic acids, e.g., HCl, HBr, H₂SO₄, H₃PO₄ or organic sulfonic acids, to basic centers, typically amines, or to acidic groups. Finally, it is to be understood that the compositions herein comprise compounds of the invention in their un-ionized, as well as zwitterionic form, and combinations with stoichiometric amounts of water as in hydrates.

Also included within the scope of this invention are the salts of the parental compounds with one or more amino acids. Any of the amino acids described above are suitable, especially the naturally-occurring amino acids found as protein components, although the amino acid typically is one bearing a side chain with a basic or acidic group, e.g., lysine, arginine or glutamic acid, or a neutral group such as glycine, serine, threonine, alanine, isoleucine, or leucine.

### Methods of Inhibition of HIV Integrase

Another aspect of the invention relates to methods of inhibiting the activity of HIV integrase comprising the step of treating a sample suspected of containing HIV with a composition of the invention.

Compositions of the invention may act as inhibitors of HIV integrase, as intermediates for such inhibitors or have other utilities as described below. The inhibitors will bind to locations on the surface or in a cavity of HIV integrase having a geometry unique to HIV integrase. Compositions binding HIV integrase may bind with varying degrees of reversibility. Those compounds binding substantially irreversibly are ideal candidates for use in this method of the invention. Once labeled, the substantially irreversibly binding compositions are useful as probes for the detection of HIV integrase. Accordingly, the invention relates to methods of detecting HIV integrase in a sample suspected of containing HIV integrase comprising the steps of: treating a sample suspected of containing HIV integrase with a composition comprising a compound of the invention bound to a label; and observing the effect of the sample on the activity of the label. Suitable labels are well known in the diagnostics field and include stable free radicals, fluorophores, radioisotopes, enzymes, chemiluminescent groups and chromogens. The compounds herein are labeled in conventional fashion using functional groups such as hydroxyl or amino.

Within the context of the invention, samples suspected of containing HIV integrase include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing an organism which produces HIV integrase, frequently a pathogenic organism such as HIV. Samples can be contained in any medium including water and organic solvent\water mixtures. Samples include living organisms such as humans, and man made materials such as cell cultures.

The treating step of the invention comprises adding the composition of the invention to the sample or it comprises adding a precursor of the composition to the sample. The addition step comprises any method of administration as described above.

If desired, the activity of HIV integrase after application of the composition can be observed by any method including direct and indirect methods of detecting HIV integrase activity. Quantitative, qualitative, and semiquantitative methods of determining HIV integrase activity are all contemplated. Typically one of the screening methods described above are applied, however, any other method such as observation of the physiological properties of a living organism are also applicable.

Organisms that contain HIV integrase include the HIV virus. The compounds of this invention are useful in the treatment or prophylaxis of HIV infections in animals or in man.

However, in screening compounds capable of inhibiting human immunodeficiency viruses, it should be kept in mind that the results of enzyme assays may not correlate with cell culture assays. Thus, a cell based assay should be the primary screening tool.

### Screens for HIV integrase Inhibitors.

Compositions of the invention are screened for inhibitory activity against HIV integrase by any of the conventional techniques for evaluating enzyme activity. Within the context of the invention, typically compositions are first screened for inhibition of HIV integrase *in vitro* and compositions showing inhibitory activity are then screened for activity *in vivo.* Compositions having *in vitro* Ki (inhibitory constants) of less then about 5 X 10⁻⁶ M, typically less than about 1 X 10⁻⁷ M and preferably less than about 5 X 10⁻⁸ M are preferred for *in vivo* use.

Useful *in vitro* screens have been described in detail and will not be elaborated here. However, the examples describe suitable *in vitro* assays.

### Pharmaceutical Formulations

The compounds of this invention are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as those set forth in the Handbook of Pharmaceutical Excipients (1986). Excipients include ascorbic acid and other antioxidants, chelating agents such as EDTA, carbohydrates such as dextrin, hydroxyalkylcellulose, hydroxyalkylmethylcellulose, stearic acid and the like. The pH of the formulations ranges from about 3 to about 11, but is ordinarily about 7 to 10.

While it is possible for the active ingredients to be administered alone it may be preferable to present them as pharmaceutical formulations. The formulations, both for veterinary and for human use, of the invention comprise at least one active ingredient, as above defined, together with one or more acceptable carriers therefor and optionally other therapeutic ingredients. The carrier(s) must be "acceptable" in the sense of being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be administered as a bolus, electuary or paste.

A tablet is made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient wherefrom.

For infections of the eye or other external tissues e.g. mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc.), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the invention include Tween^{®} 60, Span^{®} 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils are used.

Pharmaceutical formulations according to the present invention comprise a combination according to the invention together with one or more pharmaceutically acceptable carriers or excipients and optionally other therapeutic agents. Pharmaceutical formulations containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration may be prepared according to conventional methods and may be delivered with other therapeutic agents such as compounds heretofore used in the treatment or prophylaxis of HIV infections as described below.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulations of this invention may include other agents conventional in the art having regard to the type of formulation in question, for example those suitable for oral administration may include flavoring agents.

The invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds of the invention are used to provide controlled release pharmaceutical formulations containing as active ingredient one or more compounds of the invention ("controlled release formulations.") in which the release of the active ingredient are controlled and regulated to allow less frequency dosing or to improve the pharmacokinetic or toxicity profile of a given active ingredient.

Effective dose of active ingredient depends at least on the nature of the condition being treated, toxicity, whether the compound is being used prophylactically (lower doses) or against an active viral infection, the method of delivery, and the pharmaceutical formulation, and will be determined by the clinician using conventional dose escalation studies. It can be expected to be from about 0.0001 to about 100 mg/kg body weight per day. Typically, from about 0.01 to about 10 mg/kg body weight per day. More typically, from about .01 to about 5 mg/kg body weight per day. More typically, from about .05 to about 0.5 mg/kg body weight per day. For example, the daily candidate dose for an adult human of approximately 70 kg body weight will range from 1 mg to 1000 mg, preferably between 5 mg and 500 mg, and may take the form of single or multiple doses.

Active ingredients of the invention are also used in combination with other active ingredients. Such combinations are selected based on the condition to be treated, cross-reactivities of ingredients and pharmaco-properties of the combination. For example, when treating viral infections the compositions of the invention are combined with other antivirals such as other protease inhibitors, nucleoside reverse transcriptase inhibitors, non-nucleoside reverse transcriptase inhibitors or HIV integrase inhibitors.

### Routes of Administration

One or more compounds of the invention (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the recipient. An advantage of the compounds of this invention is that they are orally bioavailable and can be dosed orally.

### Combination Therapy

It is also possible to combine any compound of the invention with one or more other active ingredients in a unitary dosage form for simultaneous or sequential administration to an HIV infected patient. The combination therapy may be administered as a simultaneous or sequential regimen. When administered sequentially, the combination may be administered in two or more administrations. Second and third active ingredients in the combination may have anti-HIV activity. Exemplary active ingredients to be administered in combination with compounds of the invention are protease inhibitors, nucleoside reverse transcriptase inhibitors, non-nucleoside reverse transcriptase inhibitors, and HIV integrase inhibitors.

The combination therapy may provide "synergy" and "synergistic", i.e. the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially, e.g. in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together. A synergistic anti-viral effect denotes an antiviral effect which is greater than the predicted purely additive effects of the individual compounds of the combination.

It is also possible to combine a compound of the invention with a second or third active ingredient in a unitary dosage form for simultaneous or sequential administration. When administered sequentially, the combination may be administered in two or three administrations. The second or third active ingredient may have anti-HIV activity and include protease inhibitors (PI), nucleoside reverse transcriptase inhibitors (NRTI), non-nucleoside reverse transcriptase inhibitors (NNRTI), and integrase inhibitors. Exemplary second or third active ingredients to be administered in combination with a compound of the invention are shown in Table C.

**Table C**

| |
|---|
| 5,6 dihydro-5-azacytidine |
| 5-aza 2'deoxycytidine |
| 5-azacytidine |
| 5-yl-carbocyclic 2'-deoxyguanosine (BMS200,475) |
| 9 (arabinofuranosyl)guanine; 9-(2' deoxyribofuranosyl)guanine |
| 9-(2'-deoxy 2'fluororibofuranosyl)-2,6-diaminopurine |
| 9-(2'-deoxy 2'fluororibofuranosyl)guanine |
| 9-(2'-deoxyribofuranosyl)-2,6 diaminopurine |
| 9-(arabinofuranosyl)-2,6 diaminopurine |
| Abacavir, Ziagen® |
| Acyclovir, ACV; 9-(2-hydroxyethoxylmethyl)guanine |
| Adefovir dipivoxil, Hepsera® |
| amdoxivir, DAPD |
| Amprenavir, Agenerase® |
| araA; 9-b-D-arabinofuranosyladenine (Vidarabine) |
| atazanivir sulfate (Reyataz®) |
| AZT; 3'-azido-2',3'-dideoxythymdine, Zidovudine, (Retrovir®) |
| BHCG; (.+-.)-(1a,2b,3a)-9-[2,3-bis(hydroxymethyl)cyclobutyl]guanine |
| BMS200,475; 5-yl-carbocyclic 2'-deoxyguanosine |
| Buciclovir; (R) 9-(3,4-dihydroxybutyl)guanine |
| BvaraU; 1-b-D-arabinofuranosyl-E-5-(2-bromovinyl)uracil (Sorivudine) |
| Calanolide A |
| Capravirine |
| CDG; carbocyclic 2'-deoxyguanosine |
| Cidofovir, HPMPC; (S)-9-(3-hydroxy-2-phosphonylmethoxypropyl)cytosine |
| Clevudine, L-FMAU; 2'-Fluoro-5-methyl-β-L-arabino-furanosyluracil |
| Combivir® (lamivudine/zidovudine) |
| Cytallene; [1-(4'-hydroxy-1',2'-butadienyl)cytosine] |
| d4C; 3'-deoxy-2',3'-didehydrocytidine |
| DAPD; (-)-β-D-2,6-diaminopurine dioxolane |
| ddA; 2',3'-dideoxyadenosine |
| ddAPR; 2,6-diaminopurine-2',3'-dideoxyriboside |
| ddC; 2',3'-dideoxycytidine (Zalcitabine) |
| ddI; 2',3'-dideoxyinosine, didanosine, (Videx®, Videx® EC) |
| Delavirdine, Rescriptor® |
| Didanosine, ddI, Videx®; 2',3'-dideoxyinosine |
| DXG; dioxolane guanosine |
| E-5-(2-bromovinyl)-2'-deoxyuridine |
| Efavirenz, Sustiva® |
| Emtricitabine; (-)-cis FTC (Emtriva^{™}) |
| Enfuvirtide (Fuzeon®) |
| F-ara-A; fluoroarabinosyladenosine (Fludarabine) |
| FDOC; (-)-β-D-5-fluoro-1-[2-(hydroxymethyl)-1,3-dioxolane]cytosine |
| FEAU; 2'-deoxy-2'-fluoro-1-β-D-arabinofuranosyl-5-ethyluracil |
| FIAC; 1-(2-deoxy-2-fluoro-β-D-arabinofuranosyl)-5-iodocytosine |
| FIAU; 1-(2-deoxy-2-fluoro-β-D-arabinokranosyl)-5-iodouridine |
| FLG; 2',3'-dideoxy-3'-fluoroguanosine |
| FLT; 3'-deoxy-3'-fluorothymidine |
| Fludarabine; F-ara-A; fluoroarabinosyladenosine |
| FMAU; 2'-Fluoro-5-methyl-b-L-arabino-furanosyluracil |
| FMdC |
| Foscamet; phosphonoformic acid, PFA |
| FPMPA; 9-(3-fluoro-2-phosphonylmethoxypropyl)adenine |
| Gancyclovir, GCV; 9-(1,3-dihydroxy-2-propoxymethyl)guanine |
| GS-7340; 9-[*R*-2-[[(*S*)-[[(*S*)-1-(isopropoxycarbonyl)ethyl]amino]-phenoxyphosphinyl]methoxy]propyl]adenine |
| HPMPA; (S)-9-(3-hydroxy-2-phosphonylmethoxypropyl)adenine |
| HPMPC; (S)-9-(3-hydroxy-2-phosphonylmethoxypropyl)cytosine (Cidofovir) Hydroxyurea, Droxia® |
| Indinavir, Crixivan® |
| Kaletra® (lopinavir/ritonavir) |
| Lamivudine, 3TC, Epivir^{™}; (2*R*, 5*S*, cis)-4-amino-1-(2-hydroxymethyl-1,3-oxathiolan-5-yl)-(1H)-pyrimidin-2-one |
| L-d4C; L-3'-deoxy-2',3'-didehydrocytidine |
| L-ddC; L-2',3'-dideoxycytidine |
| L-Fd4C; L-3'-deoxy-2',3'-didehydro-5-fluorocytidine |
| L-FddC; L-2',3'-dideoxy-5-fluorocytidine |
| Lopinavir |
| Nelfinavir, Viracept® |
| Nevirapine, Viramune® |
| Oxetanocin A; 9-(2-deoxy-2-hydroxymethyl-beta-D-erythro-oxetanosyl)adenine |
| Oxetanocin G; 9-(2-deoxy-2-hydroxymethyl-β-D-erythro-oxetanosyl)guanine |
| Penciclovir PMEDAP; 9-(2-phosphonylmethoxyethyl)-2,6-diaminopurine |
| PMPA, tenofovir; (R)-9-(2-phosphonylmethoxypropyl)adenine |
| PPA; phosphonoacetic acid |
| Ribavirin; 1-β-D-ribofuranosyl-1,2,4-triazole-3-carboxamide |
| Ritonavir, Norvir® |
| Saquinavir, Invirase®, Fortovase® |
| Sorivudine, BvaraU; 1-β-D-arabinofuranosyl-E-5-(2-bromovinyl)uracil |
| Stavudine, d4T, Zerit®; 2',3'-didehydro-3'-deoxythymidine |
| Tenofovir disoproxil fumarate (TDF, Viread®) |
| Trifluorothymidine, TFT; Trifluorothymidine |
| Trizivir® (abacavir sulfate/lamivudine/zidovudine) |
| Vidarabine, araA; 9-β-D-arabinofuranosyladenine |
| Zalcitabine, Hivid®, ddC; 2',3'-dideoxycytidine |
| Zidovudine, AZT, Retrovir®; 3'-azido-2',3'-dideoxythymdine |
| Zonavir; 5-propynyl-1-arabinosyluracil |

### Metabolites of the Compounds of the Invention

The *in vivo* metabolic products the compounds described herein may result for example from the oxidation, reduction, hydrolysis, amidation, esterification and the like of the administered compound, primarily due to enzymatic processes. Accordingly, the compounds are produced by a process comprising contacting a compound of this invention with a mammal for a period of time sufficient to yield a metabolic product thereof. Such products typically are identified by preparing a radiolabelled (e.g. C¹⁴ or H³) compound of the invention, administering it parenterally in a detectable dose (e.g. greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g. by MS or NMR analysis. In general, analysis of metabolites is done in the same way as conventional drug metabolism studies well-known to those skilled in the art. The conversion products, so long as they are not otherwise found *in vivo*, are useful in diagnostic assays for therapeutic dosing of the compounds of the invention even if they possess no HIV integrase inhibitory activity of their own.

Recipes and methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37 °C. Simply because the compounds are stable to the gastrointestinal tract does not mean that they cannot be hydrolyzed *in vivo.* The phosphonate prodrugs typically will be largely stable in the digestive system but substantially hydrolyzed to the parental drug in the digestive lumen, liver or other metabolic organ, or within cells in general.

### EXEMPLARY METHODS OF MAKING THE COMPOUNDS OF THE INVENTION.

General aspects of exemplary methods are described below and in the Examples for the making, i.e. preparation, synthesis, of compounds of the invention. Each of the products of the following processes is optionally separated, isolated, and/or purified prior to its use in subsequent processes.

The compounds of the invention may be prepared by a variety of synthetic routes and methods known to those skilled in the art. The invention also relates to methods of making the compounds of the invention. The compounds are prepared by any of the applicable techniques of organic synthesis. Many such techniques are well known in the art. However, many of the known techniques are elaborated in: Compendium of Organic Synthetic Methods, John Wiley & Sons, New York, Vol. 1, Ian T. Harrison and Shuyen Harrison, 1971; Vol. 2, Ian T. Harrison and Shuyen Harrison, (1974); Vol. 3, Louis S. Hegedus and Leroy Wade, 1977; Vol. 4, Leroy G. Wade, jr., 1980; Vol. 5, Leroy G. Wade, Jr., 1984; and Vol. 6, Michael B. Smith; as well as March, J., Advanced Organic Chemistry, Third Edition, John Wiley & Sons, New York, 1985; Comprehensive Organic Synthesis. Selectivity, Strategy & Efficiency in Modern Organic Chemistry (9 Volume set) Barry M. Trost, Editor-in-Chief, Pergamon Press, New York, 1993.

Generally, the reaction conditions such as temperature, reaction time, solvents, work-up procedures, and the like, will be those common in the art for the particular reaction to be performed. The cited reference material, together with material cited therein, contains detailed descriptions of such conditions. Typically the temperatures will be -100 °C to 200 °C, solvents will be aprotic or protic, and reaction times will be 10 seconds to 10 days. Work-up typically consists of quenching any unreacted reagents followed by partition between a water/organic layer system (extraction) and separating the layer containing the product.

Oxidation and reduction reactions are typically carried out at temperatures near room temperature (about 20 °C), although for met al hydride reductions frequently the temperature is reduced to 0 °C to -100 °C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0 °C to -100 °C) are also common. Solvents can be either protic (common in equilibrating reactions) or aprotic (common in kinetically controlled reactions).

Standard synthetic techniques such as azeotropic removal of reaction byproducts and use of anhydrous reaction conditions (e.g. inert gas environments) are common in the art and will be applied when applicable.

The terms "treated", "treating", "treatment", and the like, mean contacting, mixing, reacting, allowing to react; bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two.

"Treating" indicates the reasonable and usual manner in which organic chemicals are allowed to react. Normal concentrations (0.01M to 10M, typically 0.1M to 1M), temperatures (-100 °C to 250 °C, typically -78 °C to 150 °C, more typically -78 °C to 100 °C, still more typically 0 °C to 100 °C), reaction vessels (typically glass, plastic, met al), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic synthesis selects conditions and apparatus reasonably expected to successfully carry out the chemical reactions of the described processes based on the knowledge in the art.

Modifications of each of the exemplary schemes above and in the examples (hereafter "exemplary schemes") leads to various analogs of the specific exemplary materials produce. The above cited citations describing suitable methods of organic synthesis are applicable to such modifications.

In each of the exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium, and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

A single stereoisomer, e.g. an enantiomer, substantially free of its stereoisomer may be obtained by resolution of the racemic mixture using a method such as formation of diastereomers using optically active resolving agents (Stereochemistry of Carbon Compounds, (1962) by E. L. Eliel, McGraw Hill; Lochmuller, C. H., (1975) J. Chromatogr., 113:(3) 283-302). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Drug Stereochemistry, Analytical Methods and Pharmacology, Irving W. Wainer, Ed., Marcel Dekker, Inc., New York (1993).

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (Eliel, E. and Wilen, S. (1994) Stereochemistry of Organic Compounds, John Wiley & Sons, Inc., p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, such as a menthyl ester, e.g. (-) menthyl chloroformate in the presence of base, or Mosher ester, α-methoxy-α-(trifluoromethyl)phenyl acetate (Jacob III. (1982) J. Org. Chem. 47:4165), of the racemic mixture, and analyzing the NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111). By method (3), a racemic mixture of two enantiomers can be separated by chromatography using a chiral stationary phase (Chiral Liquid Chromatography (1989) W. J. Lough, Ed. Chapman and Hall, New York; Okamoto, (1990) J. of Chromatogr. 513:375-378). Enriched or purified enantiomers can be distinguished by methods used to distinguish other chiral molecules with asymmetric carbon atoms, such as optical rotation and circular dichroism.

A number of exemplary methods for the preparation of the compounds, of the invention are provided herein. These methods are intended to illustrate the nature of such preparations.

Deliberate use may be made of protecting groups to mask reactive functionality and direct reactions regioselectively (Greene, etal (1991) "Protective Groups in Organic Synthesis", 2nd Ed., John Wiley & Sons). For example, useful protecting groups for the 8-hydroxyl group and other hydroxyl substituents include methyl, MOM (methoxymethyl), trialkylsilyl, benzyl, benzoyl, trityl, and tetrahydropyranyl. Certain aryl positions may be blocked from substitution, such as the 2-position as fluorine.

Exemplary methods of synthesis of compounds of the invention are described below in Schemes 1-10. One method of synthesis of compounds of the invention is cyclization of a succinimide compound with a pyridine dicarboxylate compound to give tricyclic compounds (Murray and Semple, Synthesis (1996) 11:80-82; Jones and Jones, Jour. Chem. Soc., Perkin Transactions I (1973) 26-32), according to Scheme 1.

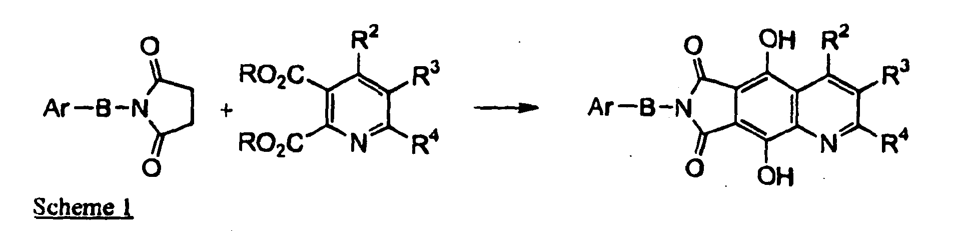

Alternatively, a succinimide with a labile protecting group (P) on the nitrogen may be reacted with a pyridine dicarboxylate compound. P may be an acid-labile protecting group, such as trialkylsilyl. Trialkylsilyl groups may also be removed with fluoride reagents. After P is removed, a variety of Ar-L groups may be covalently attached, according to Scheme 2.

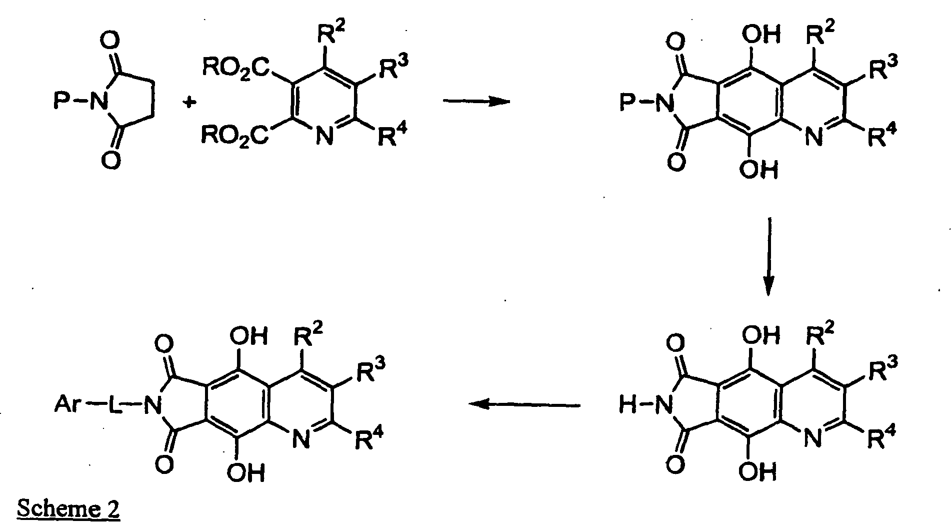

Imide compounds can be reduced with dissolving metal reducing agents, e.g. Zn, or hydride reagents, e.g. NaBH₄, to form a lactam. An exemplary regioselective conversion is shown in Scheme 3 :

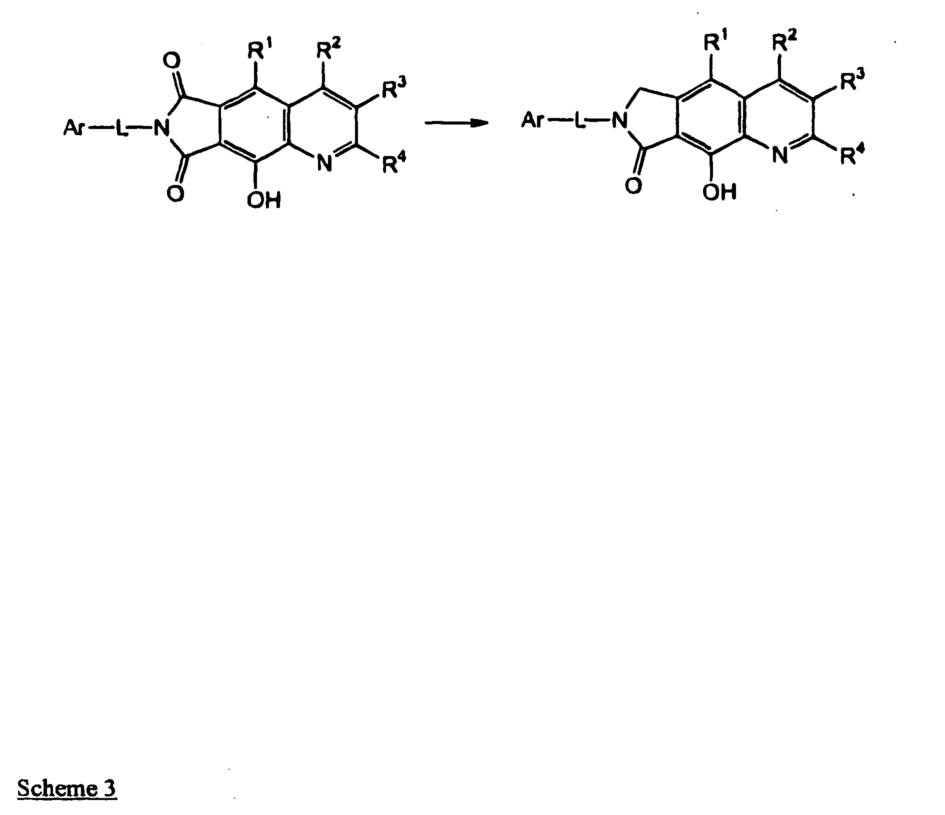

Imide compounds may also be reduced to the hydroxylactam under mild conditions. Reductions with sodium borohydride and cerium or samarium salts have been shown to proceed with regioselectivity on asymmetric imides (Mase, etal J. Chem. Soc. Perkin Communication 1 (2002) 707-709), as in Scheme 4, upper. Grignard reagents and acetylenic anions (Chihab-Eddine, etal Tetrahedron Lett. (2001) 42:573-576) may also add with regioselectivity to an imide carbonyl to form alkyl-hydroxylactam compounds, as in Scheme 4, lower). The phenolic oxygen groups may be protected and deprotected as necessary to furnish yield reactions.

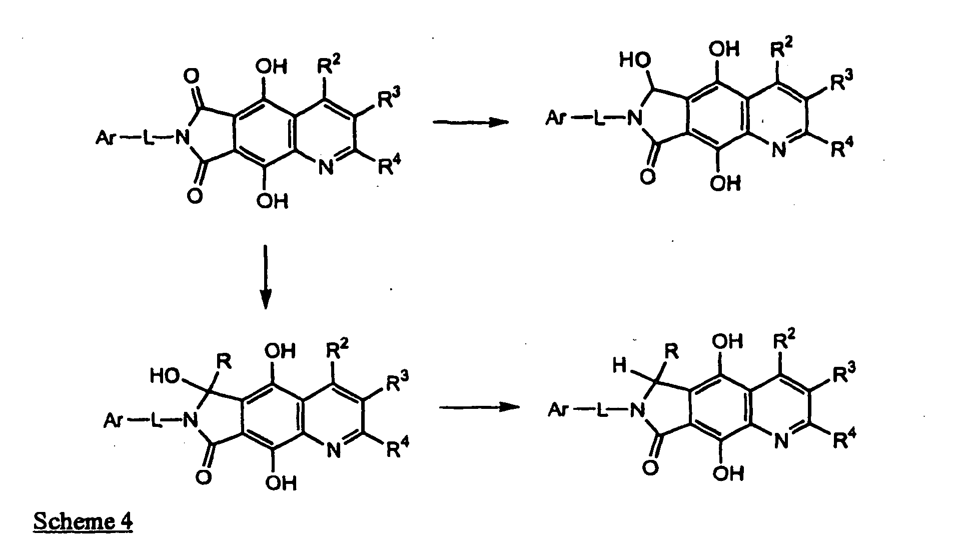

Another synthetic route to the compounds of the invention proceeds through substituted quinoline intermediates (Clemence, etal U.S. Patent No. 5,324,839; Billhardt-Troughton, etal U.S. Patent No. 5,602,146; Matsumura, J. Amer. Chem. Soc. (1935) 57:124-128) having the general formula: 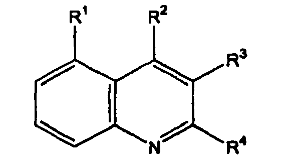 5,8-Dihydroxy quinoline compounds may be elaborated according to Scheme 5:

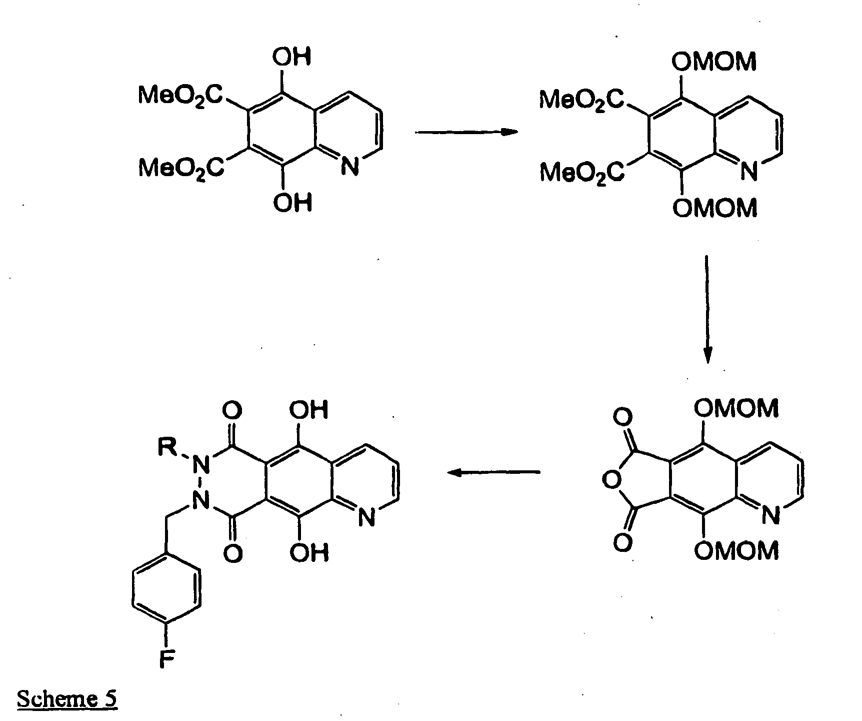

The cyclic anhydride below may be regioselectively esterified to give the compounds of the invention, for example via the route in Scheme 6 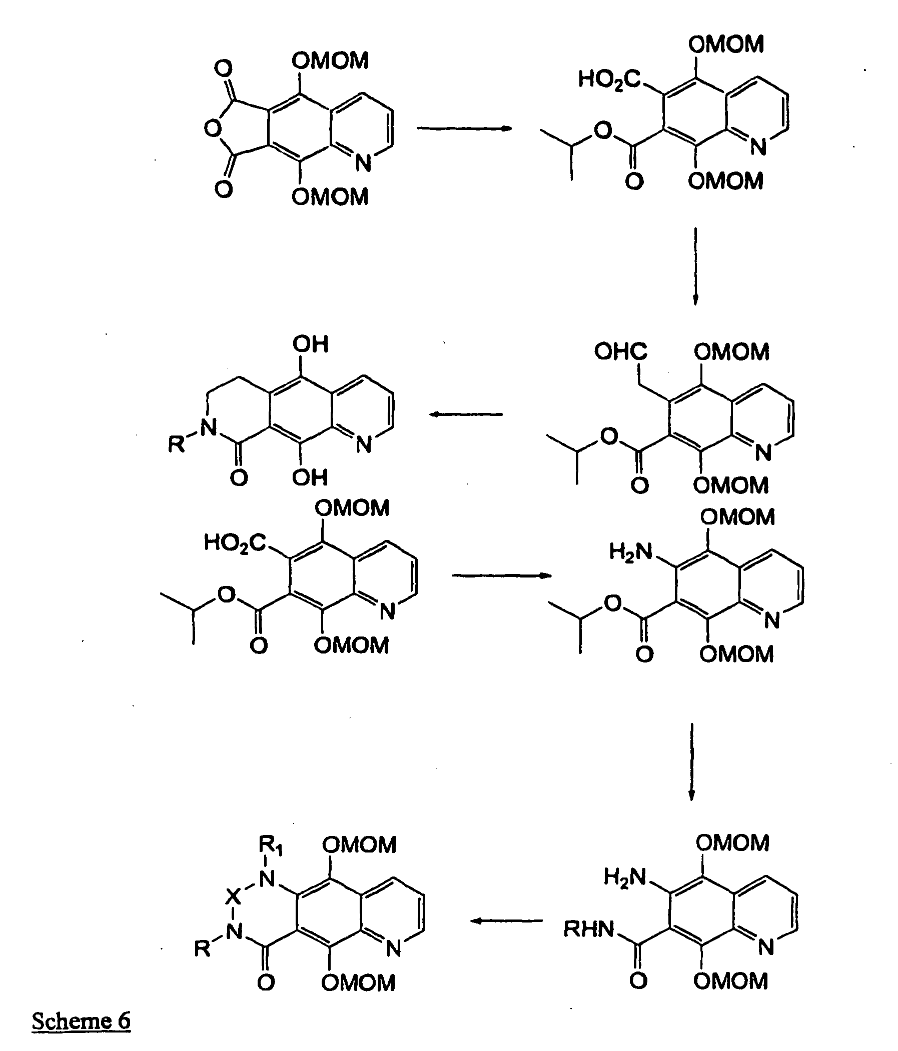 where MOM is methoxymethyl and X is, for example, C(=O), CRC(=O), C(=O)C(=O), and SO₂. See Ornstein, etal Jour. Med Chem. (1989) 32:827-833. The same chemistry can be applied to the 5-membered lactam synthesis to control the regiochemistry as in Scheme 7:

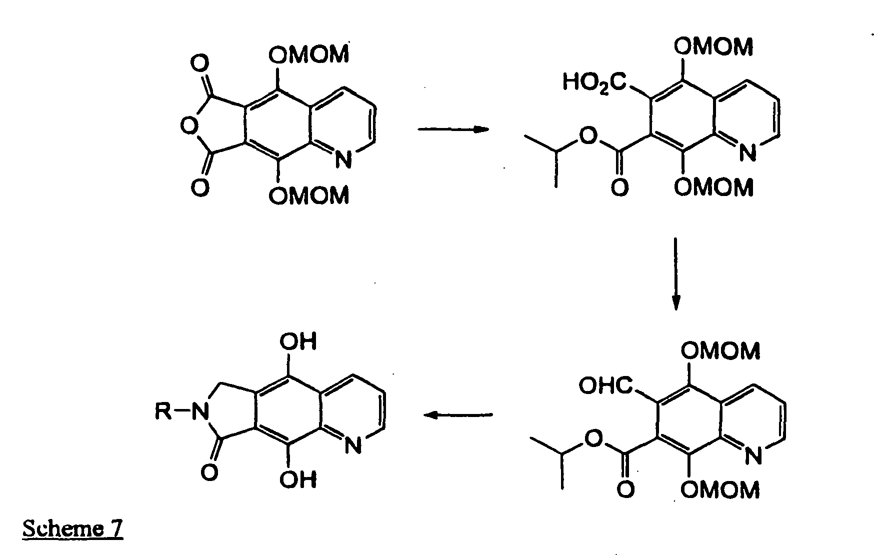

A cyclic imide may be conveniently alkylated, acylated, or otherwise reacted to form a broad array of compounds with Ar-L groups:

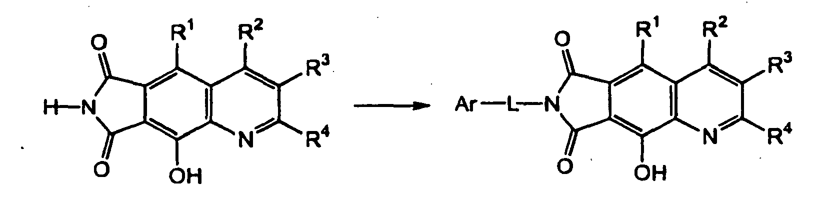

The Ar-L group may be attached as one reactant group, for example as an alkylating reagent like benzyl bromide (Ar = phenyl, L = CH₂) or a sulfonating reagent, like 4-methoxyphenyl sulfonyl chloride (Ar = 4-methoxyphenyl, L = S(=O)₂. Alternatively, the Ar-L group may be attached by a multi step process. For example, the imide nitrogen may react with a sulfurizing reagent such as 2,2-dipyridyl disulfide to form an N-sulfide intermediate (Ar = 2-pyridyl, L = S). Such an intermediate may be further elaborated to a variety of Ar-L groups where L is S, S(=O) or S(=O)₂.

Another synthetic route to the compounds of the invention proceeds through 7-substituted, 8-quinolinol intermediates (Zhuang, etal WO 02/36734; Vaillancourt, etal U.S. Patent No. 6,310,211; Hodel, U.S. Patent No. 3,113,135) having the general formulas, including aryl substituted compounds:

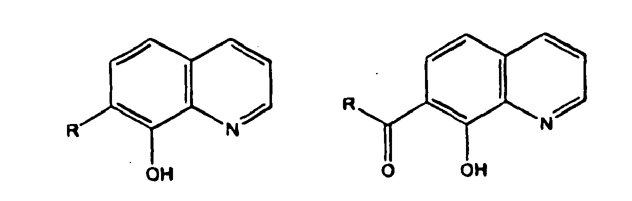

Annulation of the third, 5-7 membered ring can be conducted by appropriate selection of aryl substituents on the quinoline ring system, utilizing known synthetic transformations to give compounds of Formula I. For example, methods for coupling carboxylic acids and other activated acyl groups with amines to form carboxamides are well known in the art (March, J. Advanced Organic Chemistry, 3rd Edition, John Wiley & Sons, 1985, pp. 370-376). An exemplary cyclization includes the following:

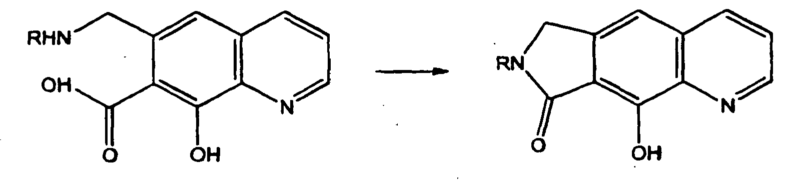

Scheme 8 below shows another synthetic route to compounds of the invention, i.e. Formula I. This route proceeds by cyclization of a 2-O-protected, 3 halo-aniline compound with an α,β-unsaturated carbonyl compound to give a functionalized quinoline. The α,β-unsaturated carbonyl compound may be, for example, an aldehyde (X=H), ketone (X=R), ester (X=OR), amide (X=NR2), acyl halide (X=Cl), or anhydride. Carbonylation via palladium catalysis can give an ester which may be elaborated to the amide functionality and cyclization to form a 5, 6, or 7 membered ring. The R group of phenolic oxygen may be a labile protecting group, e.g. trialkylsilyl or tetrahydropyranyl, which may be removed at a step in the synthetic route, or it may be a substituent which is retained in the putative integrase inhibitor compound.

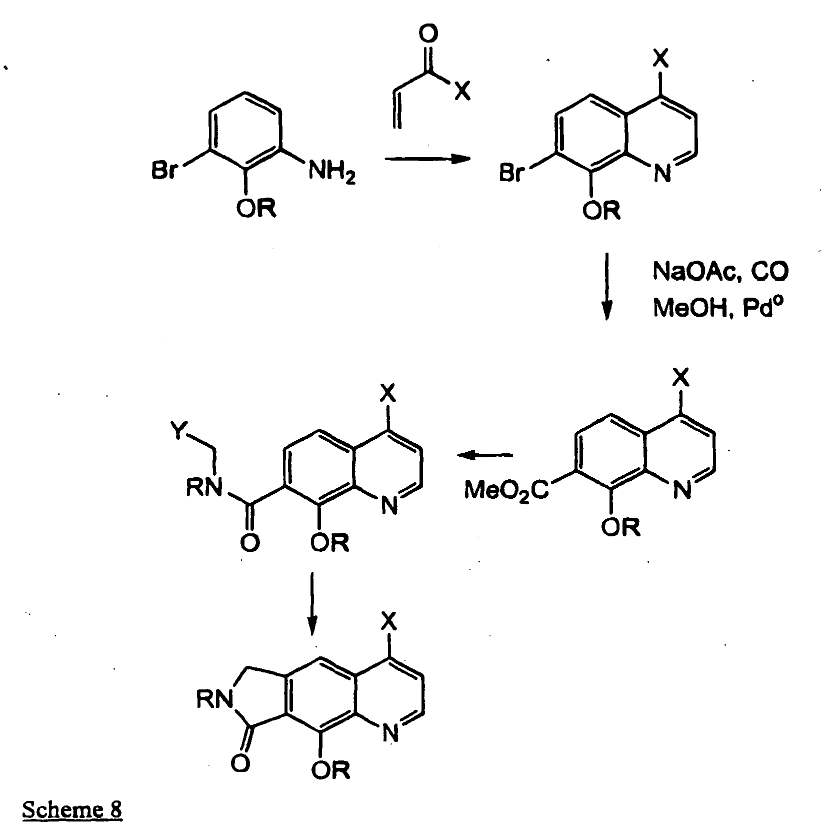

Halo quinoline intermediates may undergo a flexible array of nucleophilic aromatic substitutions and Suzuki-type reactions, as shown in Scheme 9 below. Suzuki coupling of aryl halide compounds with acetylenic and vinylic palladium complexes are carbon-carbon bond forming reactions under relatively mild conditions. In some instances it may be necessary to block the 2 position to direct reaction at the desired aryl position.

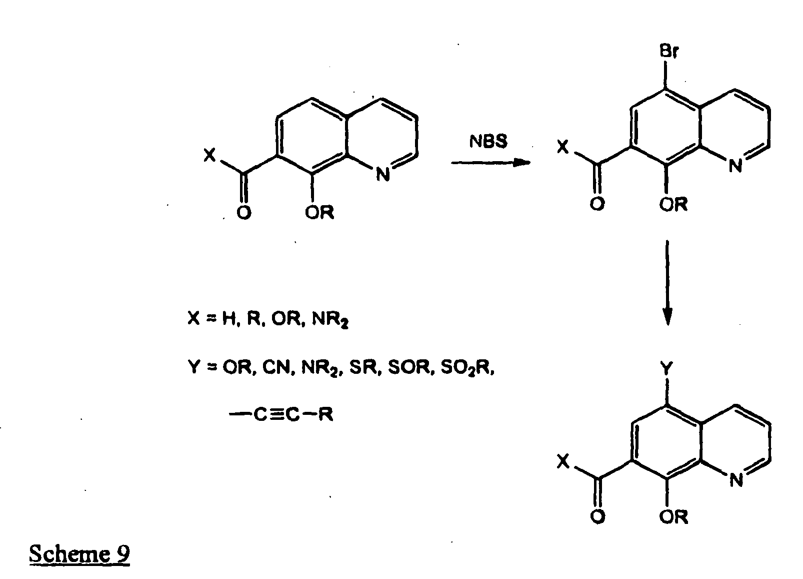

Formula I compounds with a 5,9-dihydroxy-pyrrolo[3,4-g]quinoline-6,8-dione were prepared by selective protection of the C9 phenol in 5,9-dihydroxy-pyrrolo[3,4-g]quinoline-6,8-dione. The C9 phenol was protected with a TIPS group and the C5 phenol could then be alkylated or acylated (Scheme 10).

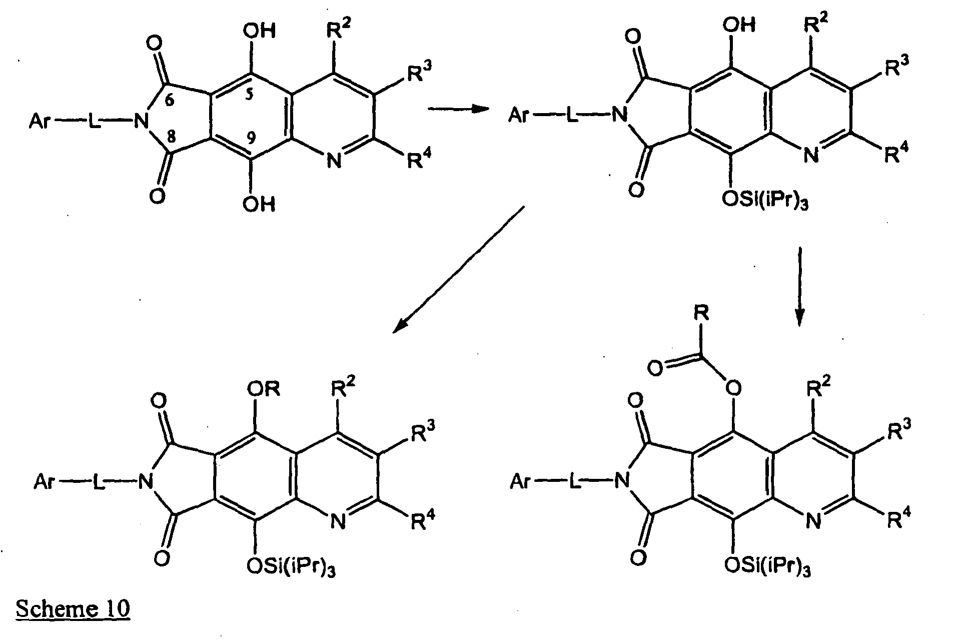

Scheme **A10** depicts the preparation of phosphonate esters in which the phosphonate group is attached by means of an amide linkage and a carbon link R, in which the group R is an acyclic or cyclic saturated or unsaturated alkylene, or aryl, aralkyl or heteroaryl moiety In this sequence, the aldehyde **A10.1**, prepared, for example, as shown in Example 16 oxidized to the corresponding carboxylic acid **A10.2**. The conversion of an aldehyde to the corresponding carboxylic acid is described in Comprehensive Organic Transformations, by R. C. Larock, VCH, 1989, p. 838. The reaction is effected by the use of various oxidizing agents such as, for example, potassium permanganate, ruthenium tetroxide, silver oxide or sodium chlorite. The carboxylic acid is then coupled, as described in Scheme **A5**, with an amine **A10.3** to afford the amide, which upon deprotection gives the phenolic amide **A10.4.**

For example, 9-benzhydryloxy-7-(4-chloro-benzyl)-6,8-dioxo-7,8-dihydro-6H-pyrrolo[3,4-g]quinoline-5-carbaldehyde **A10.5**, prepared using the methods described in Example 49, is treated with silver oxide in acetonitrile, as described in Tet. Lett., 5685, 1968, to produce the corresponding carboxylic acid 9-benzhydryloxy-7-(4-chloro-benzyl)-6,8-dioxo-7,8-dihydro-6H-pyrrolo[3,4-g]quinoline-5-carboxylic acid **A10.6**. This material is then coupled, in dimethylformamide solution, with one molar equivalent of a dialkyl aminoethyl phosphonate **A10.7** (Aurora) and dicyclohexyl carbodiimide, to afford the amide, which upon deprotection gives the phenolic product **A10.8**.

Using the above procedures, but employing, in place of the aldehyde **A10.5**, different aldehydes **A10.1**, and/or different amines **A10.3**, the corresponding products **A10.4** are obtained.

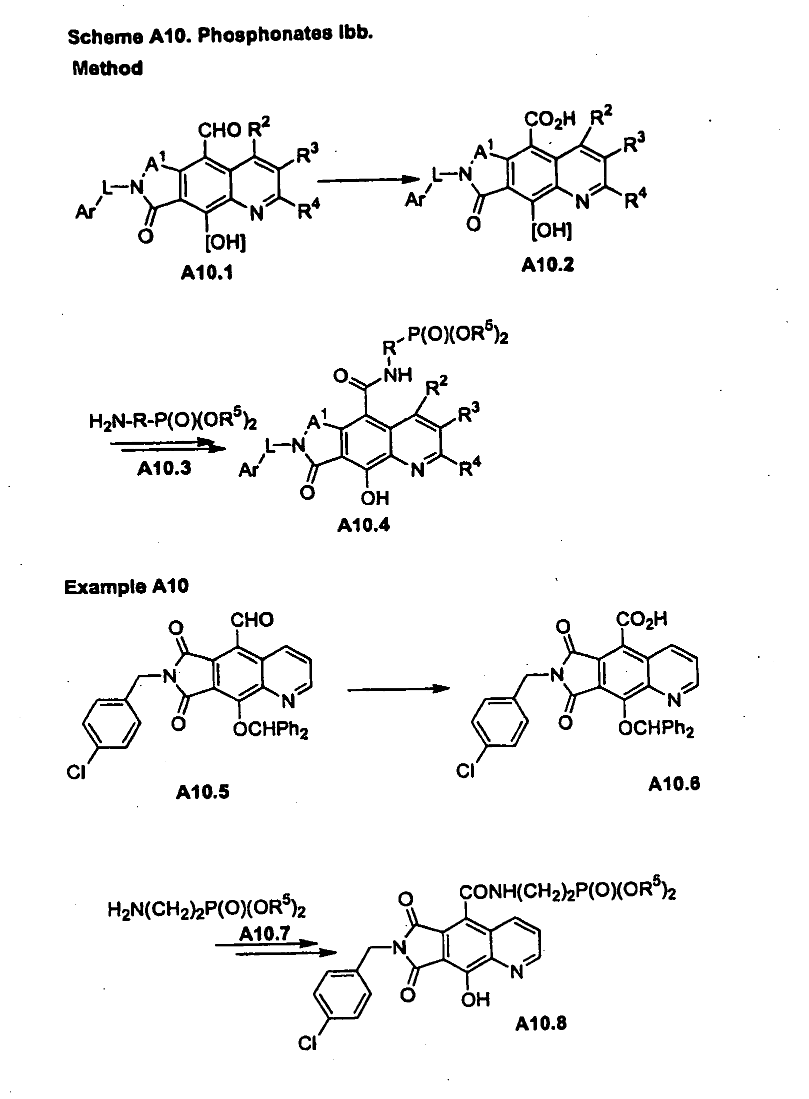

### EXAMPLE GENERAL SECTION

Some Examples have been performed multiple times. In repeated Examples, reaction conditions such as time, temperature, concentration, and the like, and yields were within normal experimental ranges. In repeated Examples where significant modifications were made, these have been noted where the results varied significantly from those described. In Examples where different starting materials were used, these are noted. When the repeated Examples refer to a "corresponding" analog of a compound, such as a "corresponding ethyl ester", this intends that an otherwise present group, in this case typically a methyl ester, is taken to be the same group modified as indicated.

### SYNTHESIS OF HIV-INTEGRASE INHIBITOR COMPOUNDS

### EXAMPLES (compounds not included in the claims do not form part of the invention)

### Example 1 N-4-fluorobenzyl-succinimide 1

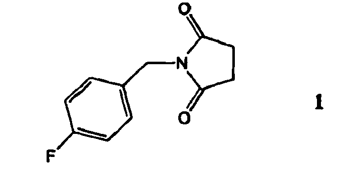

Freshly ground potassium carbonate, K₂CO₃ (31 g, 225 mmol) was added to dry acetone (200ml) in a 3-necked flask equipped with drying tube, condenser, and mechanical stirrer. Succinimide (7.43 g, 75mmol) and 4-fluorobenzylbromide (11.21 mL, 90 mmol) were added. The mixture was refluxed for 19 hours and filtered through Celite. Acetone was removed under vacuum, diluted with EtOAc, washed with saturated aqueous sodium bicarbonate and also with brine, dried (MgSO₄), filtered and concentrated to give crude. Crude product was chromatographed (EtOAc/Hexane) on silica gel to give N-4-fluorobenzyl-succinimide **1** as white solid (13.22 g, 85%). ¹H NMR (CDCl₃) δ 7.4 (dd, 2H), 7.0 (t, 2H), 4.6 (s, 1H), 2.7 (s, 4 H). Example 2 5,8-Dihydroxy-[6,7]-N-(4-fluorobenzyl)-succinimido-quinoline **2**

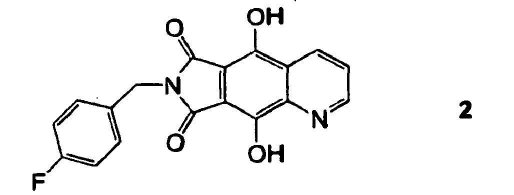

N-4-fluorobenzyl-succinimide 1 (8 g, 38.6 mmol) and 2,3-pyridine carboxylic acid dimethyl ester (7.9 g, 40.6 mmol) were dissolved in dry tetrahydrofuran (THF, 78 mL) and dry methanol (MeOH, 1.17 mL) in a 3-necked flask with mechanical stirrer and condenser. Sodium hydride (NaH, 60% in mineral oil, 3.4g, 85 mmol) was added slowly in four portions. The mixture was stirred until bubbling ceased, then refluxed for 24 hours. HCl (30 mL 6 M) was then added to the mixture while in an ice bath, with stirring for 15 minutes. Diethylether (100 mL) was added. The precipitate was filtered, washed with diethylether and H₂O, and dried under vacuum at 100 °C. Crude product was then recrystallized from 1 L refluxing dioxane and dried under vacuum at 100 ° C to give solid 5,8-Dihydroxy-[6,7]-N-(4-fluorobenzyl)-succinimido-quinoline **2** (8.6 g, 66%). ¹H NMR (CD₃SOCD₃) δ 9.05 (d, 1H), 8.75 (d, 1H), 7.79 (dd, 1H), 7.37 (dd, 2H), 7.17 (t, 2H), 4.73 (s, 2H). mp: 281.9-284.0.

### Example 3 5-O-Propanoate, 8-hydroxy-[6,7]-N-(4-fluorobenzyl)-succinimidoquinoline 3

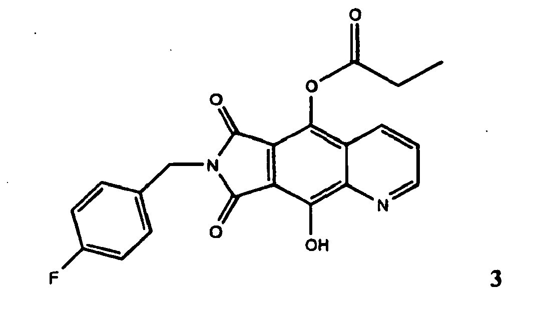

5,8-Dihydroxy-[6,7]-N-(4-fluorobenzyl)-succinimido-quinoline **2** is acylated with propanoyl chloride to give 5-O-propanoate, 8-hydroxy-[6,7]-N-(4-fluorobenzyl)-succinimido-quinoline **3**.

### Example 4 Carbonic acid ethyl ester 7-(4-fluoro-benzyl)-9-hydroxy-6,8-dioxo-7,8-dihydro-6H-pyrrolo[3,4-g]quinolin-5-yl ester 4

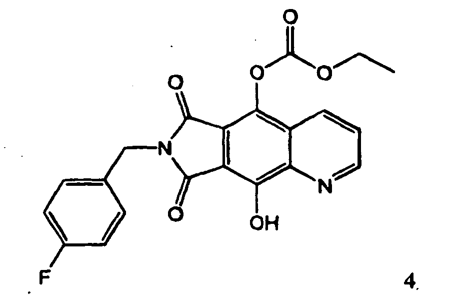

5,8-Dihydroxy-[6,7]-N-(4-fluorobenzyl)-succinimido-quinoline (300mg, 0.887 mmol) **2** was suspended in 1,4 dioxane (5 mL) and water (20 mL). An aqueous solution of NaOH (0.567 M, 3.1 mL) was added slowly to form red solution which was then cooled in an ice-water bath. Ethyl chloroformate (0.093 mL, 0.975 mmol) was added and the mixture was stirred at room temperature for 30 minutes. Dichloromethane and 1N aqueous HCl were added to the mixture in a separate. The aqueous layer was extracted with dichloromethane two more times. The combined organic solution was washed with brine, dried (MgS04) and concentrated. The crude product was crystallized from EtOAc to give carbonic acid ethyl ester 7-(4-fluorobenzyl)-9-hydroxy-6,8-dioxo-7,8-dihydro-6H-pyrrolo[3,4-g]quinolin-5-yl ester **4** (136 mg, 37%) as a yellow solid. ¹H NMR (CDCl₃) δ 9.0 (d, 1H), 8.5 (d, 1H), 7.7 (dd, 1H),7.5 (t, 2H), 7.4 (t, 2H), 7.0 (t, 2H), 4.8 (s, 2H), 4.5 (q, 2H), 1.5 (t, 3H); MS: 409 (M-1)

### Example 5 Diphenyldiazomethane 38

Benzophenone hydrazone (25 g, 122.3 mmol) and sodium sulfate (anhydrous) (26 g, 183.5 mmol) were suspended in ether (anhydrous, 400 mL). To this mixture, a potassium hydroxy (powder) saturated ethanol solution (10 mL) was added, followed by mercury oxide (66.2g 305.8 mmol) to form a red solution. This solution was shaken at RT for 1.5 hours. The solid was filtered off. The filtrate was concentrated to a residue, which was redissolved in 200 mL of hexane and placed in a cold room overnight. The solidified solution was evaporated to dryness, which gave diphenyldiazomethane **38** as a red solid (24.7 g, 99.7 %).

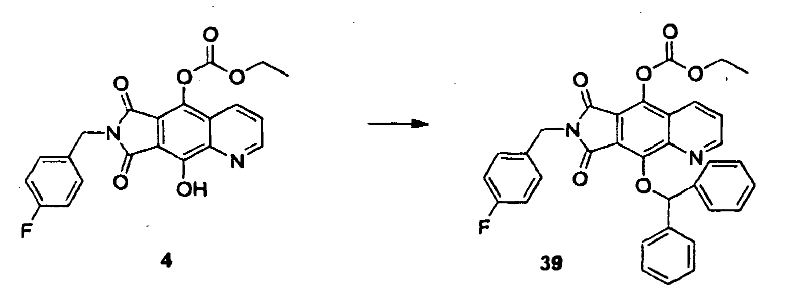

### Example 6

Mono carbonate **4** (8.9 g, 21.7 mmol) was dissolved in 1,2-dichloroethane (400 mL). Diphenyldiazomethane **38** (8.4g, 43.4 mmol) was added in one portion. The mixture was stirred at 70°C for 3 hours. The reaction was monitored by TLC (EtOAc/Hexane = 3/7). After completion of the reaction, the solution was cooled down to room temperature. The solvent was evaporated. The crude product is chromatographed on a silica gel column, eluting with EtOAc/hexane to give the product **39** as a white solid (10.1g, 80%). ¹H NMR (CDCl₃): δ 9.1 (d, 1H), 8.4 (d, 1H), 8.0 (s, 1H), 7.6 (dd, 1H), 7.6 (d, 4H), 7.4 (dd, 2H), 7.2-7.3 (m, 6H), 7.0 (t, 2H), 4.8 (s, 2H), 4.4 (q, 2H), 1.4 (t, 3H). MS: 577 (M+1), 599 (M+23).

The reaction was repeated, where mono-carbonate **4** (.2 g, 0.4878 mmol) was dissolved in 9 mL of dichloroethane. To this was added diphenyldiazomethane (0.189 g, 0.9756 mmol) and stirred at 70 °C for two hours. After starting material consumed, concentrated off some solvent, and chromatographed (25% ethylacetate/hexanes) to give product **39** (0.2653 g, .4598 mmol, 94%.) ¹H NMR (CDCl₃) δ 9.14 (d, 1H), 8.47 (d, 1H), 7.99 (s, 1H), 7.61 (m, 5H), 7.43 (dd, 2H), 7.27 (m, 6H), 7.02 (dd, 2H), 4.82 (s, 2H), 4.45 (q, 2H), 1.47 (t, 3H.) MS: 577 (M+1)

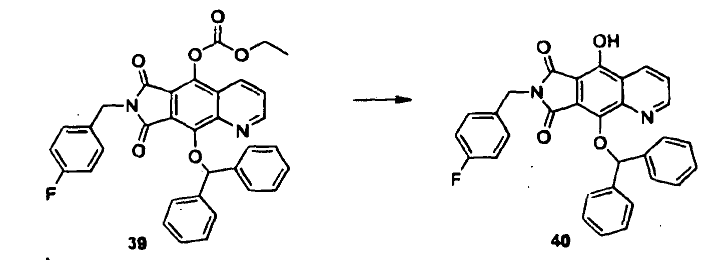

### Example 7

A solution of K₂CO₃ (24.2 g, 175.2 mmol) in water (120 mL) and 4-dimethylaminopyridine (4.24 g, 35.0 mmol) was added to the ethyl carbonate **39** (10.1 g, 17.5 mmol) in THF (180 mL). The mixture is stirred at room temperature under nitrogen atmosphere overnight. Most of THF is removed under reduced pressure at 30-40°C and the remaining solution is diluted with dichloromethane. To this, it is acidified with 1N HCl to pH about 4. The organic phase was separated and washed with brine, dried (MgSO₄) and concentrated to give a yellow solid crude product **40** (9.9 g, 100%). ¹H NMR (CDCl₃): δ 9.1 (d, 1H), 8.6 (d, 1H), 8.4 (s, 1H, (OH)), 7.8 (s, 1H), 7.6 (dd, 1H), 7.6 (dd, 4H), 7.4 (d, 2H), 7.2-7.3 (m, 6H), 7.0 (t, 2H), 4.8 (s, 2H). LC/MS: 527 (M+23).

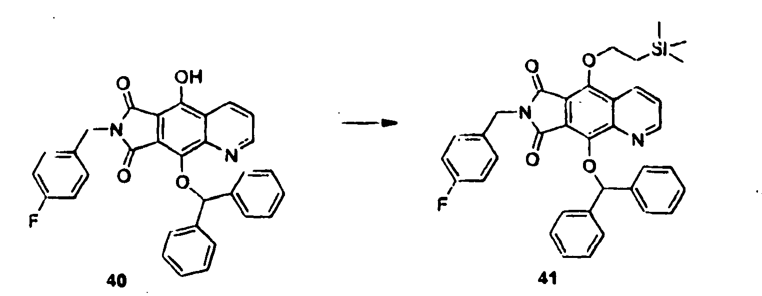

### Example 8

2-(Trimethylsilyl) ethanol (2.4 mL, 16.7 mmol), triphenylphosphine (3.5 g, 13.4 mmol) and diethyl azodicarboxylate (92.1.mL, 13.4 mmol) was added to the phenol **40** (3.37 g, 6.7 mmol) in anhydrous THF (70 mL). The solution was stirred at room temperature for 3 hours under nitrogen. TLC indicated the completion of the reaction. The solvent was evaporated and the residue oil was purified by silica gel chromatography, eluting with EtOAc/hexane to afford the product **41** (3.3 g, 82%). ¹H NMR (CDCl₃): δ 9.1 (d, 1H), 8.6 (d, 1H), 7.9 (s, 1H), 7.6 (dd, 1H), 7.6 (d, 4H), 7.4 (d, 2H). 7.2-7.3 (m, 6H), 7.0 (t, 2H), 4.8 (s, 2H), 4.6 (t, 2H), 1.2 (t, 2H). MS: 605 (M+1), 627 (M+23).

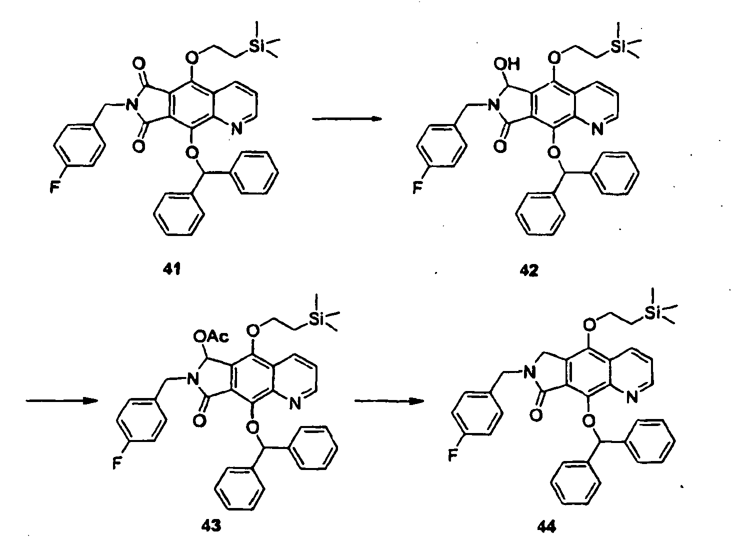

### Example 9

Compound **41** (3.3 g, 5.46 mmol) was dissolved in the mixture of THF (40 mL), isopropanol (20 mL) and water (10 mL) and chilled to 0 °C in an ice-bath. To this was added lithium borohydride (373.0 mg, 16.4 mmol) slowly. The mixture was stirred at 0°C for 1 hour and at room temperature for 1 hour under nitrogen. TLC indicated the completion of the reaction. A solution of 1N HCl (30 mL) was added and the mixture was extracted twice with CH₂Cl₂ (2x50 mL). The organic layer was washed with saturated NaHC0₃ and dried over Mg₂SO₄ and evaporated to dryness to give **42** as an oil (3.3 g).

### Example 10

Crude product **42** was dissolved in anhydrous dichloromethane (50mL). *N-*dimethylaminopyridine (66.7 mg, 0.546 mmol), *N*,*N*-diisopropylethylamine (2.85 mL, 16.4 mmol) and acetic anhydride (1.03 mL, 109 mmol) were added. The mixture was stirred at room temperature under nitrogen overnight. TLC indicated the completion of the reaction. The reaction was quenched with 1N HCl (30 mL) and extracted with CH₂Cl₂ twice (2x50 mL). The organic layer was washed with saturated NaHCO₃, dried (Mg₂SO₄) and concentrated to give crude product **43** (3.5 g).

### Example 11

Crude product **43** was dissolved in anhydrous dichloromethane (60mL) under nitrogen. To this solution was added 2,6-lutidine (3.2 mL, 23.7 mmol), triethylsiliane (10 mL), then trimethylsilyl triflate (1.5 mL, 8.2 mmol) slowly. The mixture was stirred at room temperature for 3 hours. TLC indicated the completion of the reaction. It was quenched with 1N HCl (30 mL) and extracted with CH₂Cl₂ twice (2x50 mL). The organic layer was washed with saturated NaHCO₃, dried (Mg₂SO₄) and concentrated. The residue was chromatographed on a silica gel column, eluting with EtOAc/Hexane to afford **44** (1.4 g, 43.4% in 3 steps from **41**). ¹H NMR (CDCl₃): δ 9.0 (d, 1H), 8.4 (d, 1H), 8.0 (s, 1H), 7.7 (d, 4H), 7.4 (dd, 1H), 7.1-7.3 (m, 8H), 7.0 (t, 2H), 4.8 (s, 2H), 4.2 (s, 2H), 4.1 (t, 2H), 1.1 (t, 2H), 0.1 (s, 9H). MS: 591 (M+1).

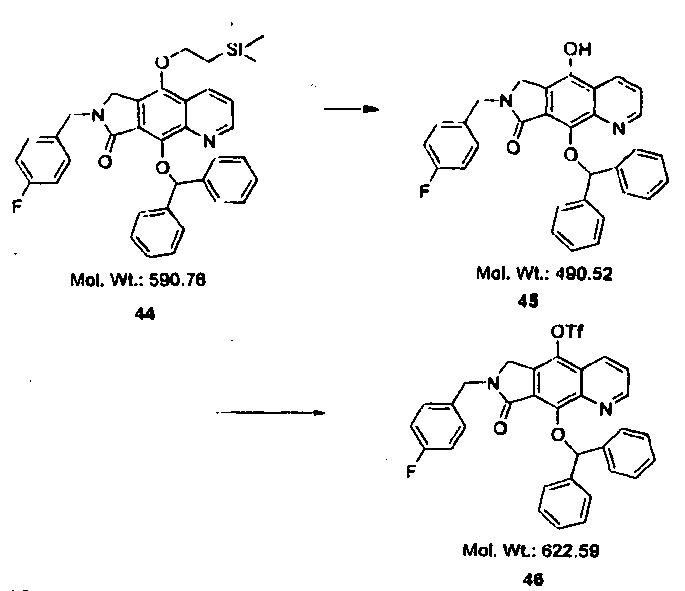

### Example 12

To 9-benzhydryloxy-7-(4-fluoro-benzyl)-5-(2-trimethylsilanyl-ethoxy)-6,7-dihydro-pyrrolo[3,4-g]quinolin-8-one **44** (300.8 mg, 0.509 mmol) in anhydrous THF (20 mL), was added tetrabutylammonium fluoride hydrate (500 mg, 1.02 mmol). The reaction mixture turned to red and was stirred at room temperature under nitrogen for 1 hour. The reaction was monitored by TLC (EtOAc/Hexane = 3/7). After completion of the reaction, it was diluted with EtOAc (50 mL) and washed with 1N HCl, saturated NaHCO₃ and brine. The organic layer was dried (MgSO₄) and concentrated to give a crude product **45** (280 mg).

The reaction was repeated whereby, to a solution of lactam **44** (0.026g, 0.044 mmol) in THF (0.441 mL) was added triethylamine (0.025 mL, 0.176 mmol) and tetrabutylammonium fluoride in 1M THF (0.066 mL). The reaction mixture was stirred at room temperature under an inert atmosphere for 30 minutes, monitored to completion by MS. The mixture was diluted with dichloromethane, washed with saturated NH₄Cl, dried (MgSO₄), and concentrated *in vacuo*. The crude material **45** was taken forward immediately with no further purification or characterization: MS: 491 (M+1).

Alternatively, to a solution of 9-benzhydryloxy-7-(4-fluoro-benzyl)-5-(2-trimethylsilanyl-ethoxy)-6,7-dihydro-pyrrolo[3,4-g]quinolin-8-one **44** (30 mg, 0.051 mmol) dissolved in THF (1 mL) was added tetrabutylammonium fluoride hydrate (1M in THF, 150 µl). The reaction mixture turned to red and was stirred at room temperature for 1/2 hours under an inert atmosphere, which generated 9-benzhydryloxy-7-(4-fluoro-benzyl)-5-hydroxy-6,7-dihydro-pyrrolo[3,4-g]quinolin-8-one **45**. TLC was used to monitor the reaction.

### Example 13

Crude compound **45** was dissolved in dichloromethane (20 mL). To this was added cesium carbonate (200 mg, 0.611 mmol) and *N-*phenyltrifluoromethane sulfonimide (220 mg, 0.611 mmol). The mixture was stirred at room temperature under nitrogen for 16 hours. The reaction was monitored by TLC (EtOAc/Hexane = 3/7). After completion of the reaction, it was diluted with EtOAc (50 mL) and washed with 1N HCl, saturated NaHCO₃ and brine. The organic layer was dried (MgSO₄) and concentrated. The residue was chromatographed on a silica gel column, eluting with EtOAc/Hcxanc to afford the clean product **46** (135 mg, 42.6% in 2 steps). ¹H NMR (CDCl₃): δ 9.1 (d, 1H), 8.3 (d, 1H), 8.0 (s, 1H), 7.7 (d, 4H), 7.6 (dd, 1H), 7.2-7.4 (m, 8H), 7.1 (t, 2H), 4.8 (s, 2H), 4.4 (s, 2H). MS: 623 (M+1), 645(M+23).

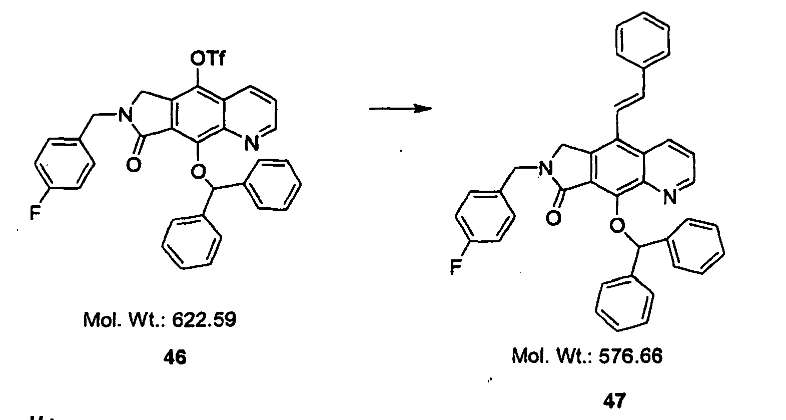

### Example 14

To the triflate **46** (66.6 mg, 0.107 mmol) in toluene (2.8 mL)/ethanol (1.2 mL)/water (0.8 mL) was added potassium carbonate (37 mg, 0.268 mmol), transphenylvinylboronic acid (24.5 mg, 0.160 mmol) and tetrakis(triphenylphosphine)-palladium (0) (18.5 mg, 0.016 mmol). The mixture in the flask was flushed with argon three times and heated to 120 °C under argon for 3 hours. The mixture was cooled to room temperature, diluted with EtOAc and washed with 1N HCl, saturated NaHCO₃ and brine. The organic phase was dried (MgSO₄) and concentrated. The residue was chromatographed on a silica gel column, eluting with EtOAc/Hexane to afford the product **47** (51.4 mg, 83%). ¹H NMR (CDCl₃): δ 9.0 (d, 1H), 8.4 (d, 1H), 8.1 (s, 1H), 7.7 (d, 4H), 7.2-7.5 (m, 14H), 7.1 (d, 1H), 7.0 (dd, 2H), 6.8 (d, 1H), 4.8 (s, 2H), 4.4(s, 2H). MS: 577 (M+1), 599(M+23).

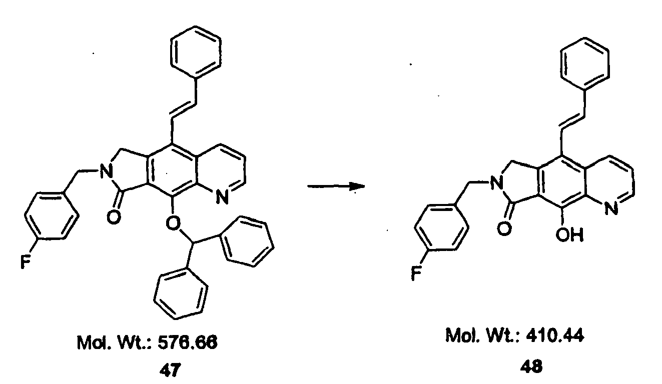

### Example 15

The compound **47** (12 mg, 0.02 mmol) was dissolved in dichloromethane (1 mL) at room temperature under nitrogen. Triethylsilane (200 J.1L) was added followed by TFA (100 µL) slowly. The mixture became smoke and dark. It was stirred at room temperature for 30 min. The solvent was removed under reduced pressure. The crude product was triturated in diethylether/hexane to afford a yellow solid **48** (9 mg, 90%).
¹H NMR (CDCl₃): δ 9.0 (d, 1H), 8.6 (d, 1H), 7.5 (m, 3H), 7.2-7.4 (m, 6H), 7.1 (m, 2H), 6.8 (d, 1H), 4.8 (s, 2H), 4.5(s, 2H). MS: 411 (M+1).

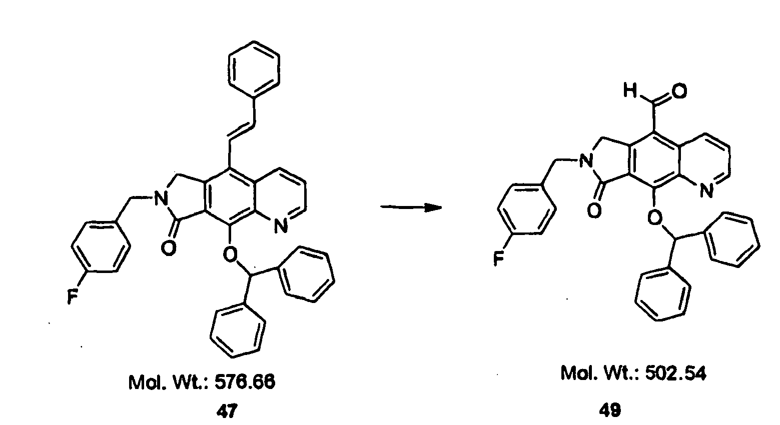

### Example 16

Compound **47** (405 mg, 0.7 mmol) in dichloromethane (150 mL) was chilled to -78 °C. Ozone (O₃) was passed slowly into the solution over 30 min. TLC indicated the completion of the reaction. Nitrogen was bubbled into the mixture for 10 min to expel excess O₃. Dimethyl sulfate (10 mL) was then added the mixture at - 78 °C and the mixture was warmed to room temperature slowly with stirring. After 16 hours, the mixture was evaporated to dryness and the residue was purified by chromatography on a silica gel column, eluting with methanol/dichloromethane to give product of **49** (166.5 mg) and its hydrate form (122 mg), total yield of 80.8%. ¹H NMR (CDCl₃): δ 10.7 (s, 1H, CHO), 9.1 (m, 2H), 8.4 (s, 1H), 7.7 (d, 4H), 7.6 (dd, 2H), 7.2-7.4 (m, 8H), 7.0 (t, 2H), 4.8 (s, 2H), 4.6 (s, 2H). MS: 503 (M+1), 525(M+23).

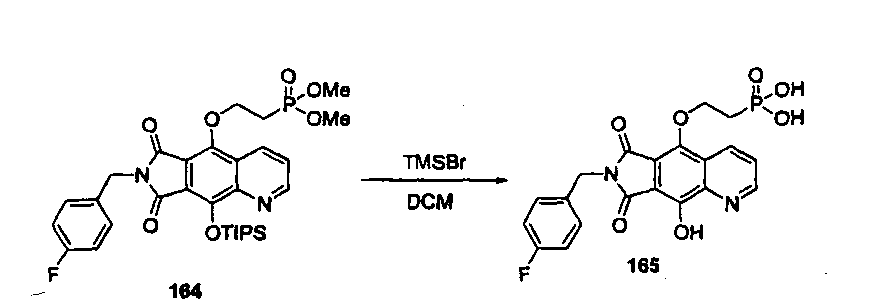

### Example 17

A solution of the phosphonate **164** (0.025 g, 0.040 mmol) in dichloromethane (0.397 mL) was treated with trimethylsilane bromide (0.0314 mL, 0.24 mmol). The reaction mixture was stirred at room temperature under an inert atmosphere overnight. The volatiles were removed *in vacuo* with methanol. The solid was washed with dichloromethane to afford the diacid **165** (0.0094 g, 53%): ¹H NMR (CD₃OD) δ 9.4 (dd, 1H), 9.1 (dd, 1H), 8.05 (dd, 1H), 7.5 (dd, 2H), 7.1 (t, 2H), 4.9 (s, 2H), 4.8 (m, 2H), 2.45 (m, 2H); ³¹P NMR (CD₃OD) δ 24.7; MS: 447 (M+1), 445 (M-1).

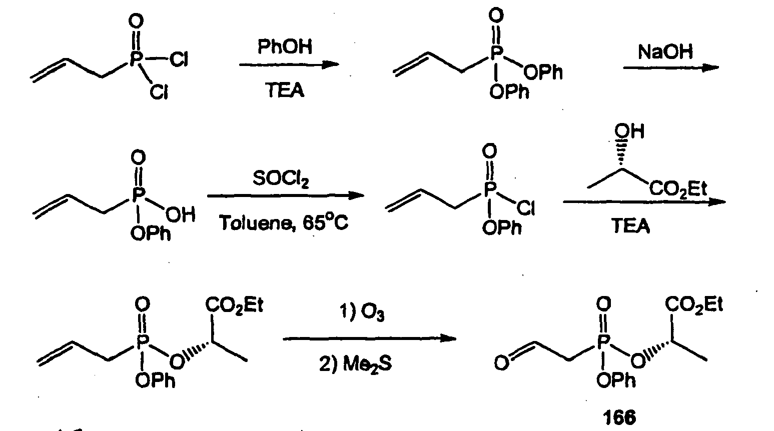

### Example 18

To a solution of allylphosphonic dichloride (4 g, 25.4 mmol) and phenol (5.2 g, 55.3 mmol) in CH₂Cl₂ (40 mL) at 0 °C was added triethylamine (TEA, 8.4 mL, 60 mmol). After stirring at room temperature for 1.5 h, the mixture was diluted with hexane-ethylacetate and washed with HCl (0.3 N) and water. The organic phase was dried over MgSO₄, filtered and concentrated under reduced pressure. The residue was filtered through a pad of silica gel (eluted with 2:1 hexane-ethyl acetate) to afford crude product diphenol allylphosphonate (7.8 g, containing the excessive phenol) as an oil which was used directly without any further purification. The crude material was dissolved in CH₃CN (60 mL), and NaOH (4.4N, 15 mL) was added at 0 °C. The resulting mixture was stirred at room temperature for 3 h, then neutralized with acetic acid to pH = 8 and concentrated under reduced pressure to remove most of the acetonitrile. The residue was dissolved in water (50 mL) and washed with CH₂Cl₂ (three 25 mL portions). The aqueous phase was acidified with concentrated HCl at 0 °C and extracted with ethyl acetate. The organic phase was dried over MgSO₄, filtered, evaporated and co-evaporated with toluene under reduced pressure to yield desired monophenol allylphosphonate (4.75 g. 95%) as an oil.

To a solution of monophenol allylphosphonate (4.75 g, 24 mmol) in toluene (30 mL) was added SOCl₂ (5 mL, 68 mmol) and DMF (0.05 mL). After stirring at 65 °C for 4 h, the reaction was complete as shown by ³¹P NMR. The reaction mixture was evaporated and co-evaporated with toluene under reduced pressure to give the mono chloride (5.5 g) as an oil. To a solution of the mono chloride in CH₂Cl₂ (25 mL) at 0 °C was added ethyl (*S*)-lactate (3.3 mL, 28.8 mmol), followed by TEA. The mixture was stirred at 0 °C for 5 min, then at room temperature for 1 h, and concentrated under reduced pressure. The residue was partitioned between ethylacetate and HCl (0.2N), the organic phase was washed with water, dried over MgSO₄, filtered and concentrated under reduced pressure. The residue was purified by chromatography on silica gel to afford the allyl monolactate (5.75 g, 80%) as an oil (2:1 mixture of two isomers): ¹H NMR (CDCl₃) δ 7.1-7.4 (m, 5H), 5.9 (m, 1H), 5.3 (m, 2H), 5.0 (m, 1H), 4.2 (m, 2H), 2.9 (m, 2H), 1.6; 1.4 (d, 3H), 1.25 (m, 3H); ³¹P NMR (CDCl₃) δ 25.4, 23.9.

A solution of the allyl monolactate (2.5 g, 8.38 mmol) in CH₂Cl₂ (30 mL) was bubbled with ozone air at -78 °C until the solution became blue, then bubbled with nitrogen until the blue color disappeared. Methyl sulfide (3 mL) was added at -78 °C. The mixture was warmed up to room temperature, stirred for 16 h and concentrated under reduced pressure to give desired aldehyde 166 (3.2 g, as a 1:1 mixture of DMSO): ¹H NMR (CDCl₃) δ 9.8 (m, 1H), 7.1-7.4 (m, 5H), 5.0 (m, 1H), 4.2 (m, 2H), 3.4 (m, 2H), 1.6; 1.4 (d, 3H), 1.25 (m, 3H). ³¹P NMR (CDCl₃) δ 17.7, 15.4.

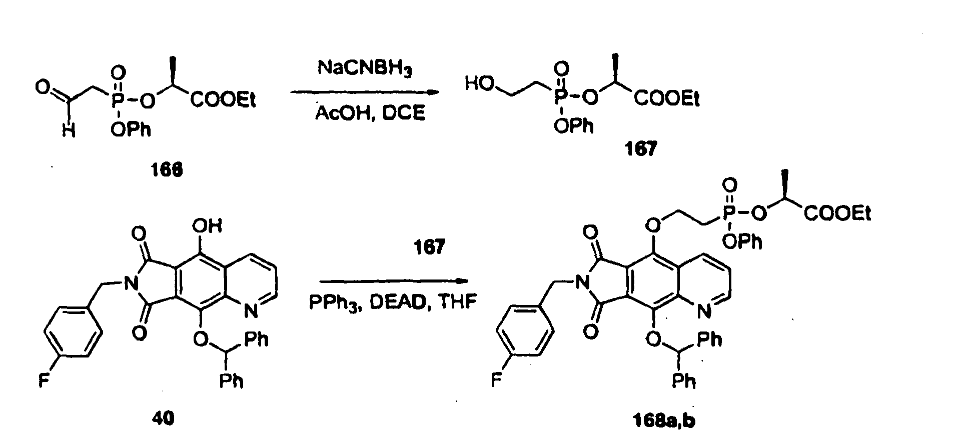

### Example 20

To a solution of 2-[(2-oxo-ethyl)-phenoxy-phosphinoyloxy]-propionic acid ethyl ester; aldehyde **166** (0.082 g, 0.218 mmol) in a 1:1 mixture of DMSO and 1,2-dichloroethane was added acetic acid (0.050 mL, 0.870 mmol) then sodium cyanoborohydride (0.027 g, 0.435 mmol). The reaction mixture stirred at room temperature for three hours under an inert atmosphere. Saturated NaHCO₃ was added to the reaction mixture and was stirred for five more minutes. The mixture was concentrated *in vacuo* to remove most of the dichloroethane. Brine was added and then the crude product was extracted into ethylacetate. The organic phase was dried (MgSO₄) and concentrated. The residue was purified by silica gel chromatography (5/95 - methanol/dichloromethane) to afford the product **167** (0.047 g, 73%), an oil as a mixture of two diastereomers: ¹H NMR (CDCl₃) δ 7.1-7.4 (m, 5H), 5.1 (m, 1H), 4.25 (m, 2H), 4.1 (m, 2H), 2.3 (m, 4H), 1.6 & 1.4 (d, 3H),1.25 (m, 3H); ³¹P NMR (CDCl₃) δ 29.0, 26.8.

### Example 21

To a solution of phenol **40** (0.033 g,0.065 mmol) dissolved in THF (0.34 mL) was added ethyl-lactate phosphonate alcohol **167** (0.029 g, 0.097 mmol), triphenylphosphine (0.043 g, 0.162 mmol), and diethyl azodicarboxylate (0.015 mL, 0.097 mmol). The reaction mixture stirred at room temperature under an inert atmosphere overnight. The residue was purified directly by silica gel chromatography (50/50 - ethylacetate/hexane) to afford the product **168** (0.027 g, 53%). Separation of the diastereomers by chromatography allowed for characterization of **168a** (0.016g): ¹H NMR (CDCl₃) δ 9.1 (dd, 1H), 8.8 (dd, 1H), 7.9 (s, 1H), 7.6 (m, 4H), 7.55 (m, 1H), 7.4 (dd, 2H), 7.1-7.4 (m, 11H), 7.0 (t, 2H), 5.0 (m, **1**H), 4.9 (s, 2H), 4.8 (m, 2H), 4.1 (q, 3H), 2.75 (m, 2H), 1.4 (d, 3H), 1.2 (t, 3H); ³¹P NMR (CDCl₃) δ 26.05; MS: 790 (M+1) - and **168b** (0.011g): ¹HNMR (CDCl₃) δ 9.1 (dd, 1H), 8.8 (dd, 1H), 7.95 (s, 1H), 7.6 (m, 4H), 7.55 (m, 1H), 7.40 (dd, 2H), 7.1-7.4 (m, 11H), 7.05 (t, 2H), 5.05 (m, 1H), 4.85 (s, 2H), 4.8 (m, 2H), 4.15 (q, 3H), 2.7 (m, 2H), 1.55 (d, 3H), 1.2 (t, 3H); ³¹P NMR (CDCl₃) δ 24.37; MS: 790 (M+1)

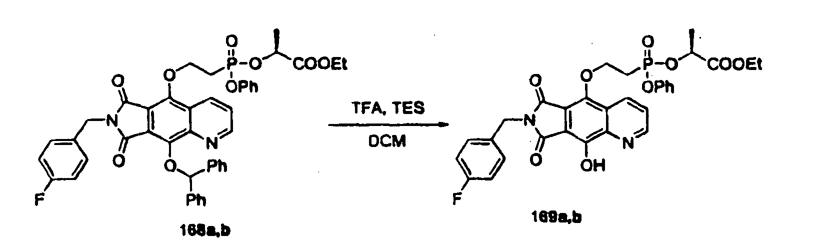

### Example 22a

A solution of the phosphonate **168a** (0.013 g, 0.0165 mmol) in dichloromethane, (0.5 mL) was treated with trifluoroacetic acid (0.1 mL) and triethylsilane (0.2 mL). The reaction mixture was stirred at room temperature under an inert atmosphere for 20 minutes. The volatiles were removed *in vacuo* with toluene. The solid was triturated in diethylether/hexane to afford the product **169a** (0.008 g, 80%) as a TFA salt: ¹H NMR (CDCl₃) δ 8.95 (dd, 1H), 8.9 (dd, 1H), 7.6 (m, 1H), 7.5 (dd, 2H), 7.1-7.4 (m, 5H), 7.0 (t, 2H), 5.0 (m, 1H), 5.0 (m, 2H), 4.85 (s, 2H), 4.15 (q, 3H), 2.8 (m, 2H), 1.4 (d, 3H), 1.25 (t, 3H); ³¹P NMR (CDCl₃) δ 26.13; MS: 623 (M+1), 621 (M-1).

### Example 22b

A solution of the phosphonate **168b** (0.011 g, 0.014 mmol) in dichloromethane (0.5 mL) was treated with trifluoroacetic acid (0.1 mL) and triethylsilane (0.2 mL). The reaction mixture was stirred at room temperature under an inert atmosphere for 20 minutes. The volatiles were removed *in vacuo* with toluene. The solid was triturated in diethylether/hexane to afford the product **169b** (0.005 g, 60%) as a TFA salt: ¹H NMR (CDCl₃) δ 8.95 (dd, 1H), 8.9 (dd, 1H), 7.65 (m, 1H), 7.5 (dd, 2H), 7.1-7.4 (m, 5H), 7.0 (t, 2H), 5. (m, 2H), 4.9 (m, 1H), 4.85 (s, 2H), 4.15(q, 3H), 2.7 (m, 2H), 1.55 (d, 3H), 1.2 (t, 3H); ³¹P NMR (CDCl₃) δ 24.44; MS: 623 (M+1), 621 (M-1).

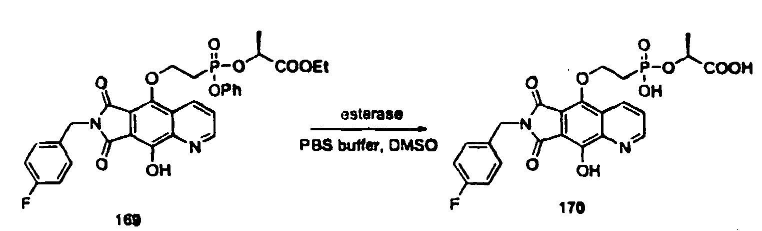

### Example 23

A solution of ethyl-lactate phosphonate **169** (0.021 g, 0.034 mmol) in DMSO (0.675 mL) and phosphate buffer saline (3.38 ml) was heated to 40° C. The reaction mixture was treated with esterase-from porcine liver (0.200 mL) and stirred overnight. Another equivalent of esterase was added the following day and the mixture stirred another day. The mixture was concentrated and purified by reversed phase HPLC to afford the product **170** (0.008 g, 46%) as a solid: ¹H NMR (CD₃OD) δ 8.95 (dd, 1H), 8.9 (dd, 1H), 7.75 (m, 1H), 7.45 (dd, 2H), 7.05 (t, 2H), 4.9 (s, 2H), 4.85 (m, 3H), 2.5 (m, 2H), 1.5 (d, 3H); ³¹P NMR (CD₃OD) δ 26.26; MS: 519 (M+1), 517 (M-1).

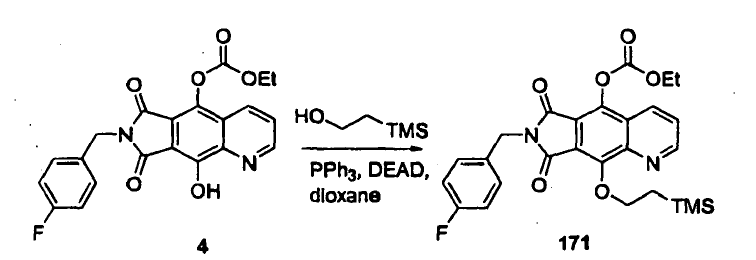

### Example 24

To a solution of phenol **4** (1.14 g, 2.79 mmol) dissolved in dioxane (27.9 mL) was added 2-(trimethylsilyl)-ethanol (0.600 mL, 4.18 mmol), triphenylphosphine (1.46 g, 5.57 mmol), and diethyl azodicarboxylate (0.88 mL, 5.57 mmol). The reaction mixture stirred at room temperature under an inert atmosphere overnight. The residue was purified directly by silica gel chromatography (30/70 - ethylacetate/hexane) to afford the product **171** (0.240 g, 67%): ¹H NMR (CDCl₃) δ 9.1 (dd, 1H), 8.5 (dd, 1H), 7.65 (dd, 1H), 7.45 (dd, 2H), 7.0 (t, 2H), 4.9 (m, 2H), 4.8 (s, 2H), 4.45 (q, 2H), 1.5 (t, 3H), 1.4 (m. 2H), 0.1 (s, 9H); MS: 510 (M+1).

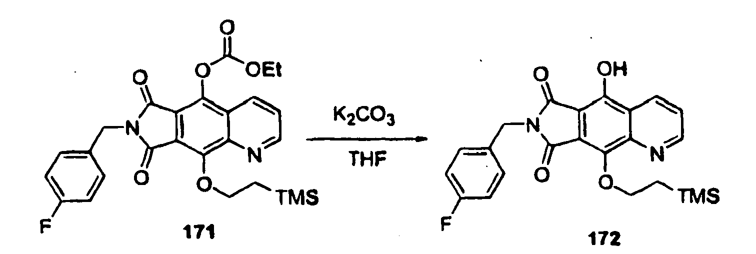

### Example 25

To the ethyl carbonate **171** (0.716 g, 1.4 mmol) in THF (70.2 mL) was added a solution (45 mL) of K₂CO₃ (1.94 g, 14 mmol) in water and 4-dimethylaminopyridine (0.035 g, 0.281 mmol). The yellow solution was stirred at room temperature under an inert atmosphere overnight. Most of THF was removed *in vacuo* and the remaining solution was diluted with dichloromethane, washed with 1N HCl and brine, then dried (MgSO₄) and concentrated. The crude product was triturated in diethylether/hexane to afford the yellow solid product **172** (0.428 g, 70%): ¹H NMR (CDCl₃) δ 9.1 (dd, 1H), 8.65 (dd, 1H), 7.6 (dd, 1H), 7.5 (dd, 2H), 7.0 (t, 2H), 4.85 (s, 2H), 4.85 (m, 2H), 1.35 (m, 2H), 0.1 (s, 9H); MS: 438 (M+1).

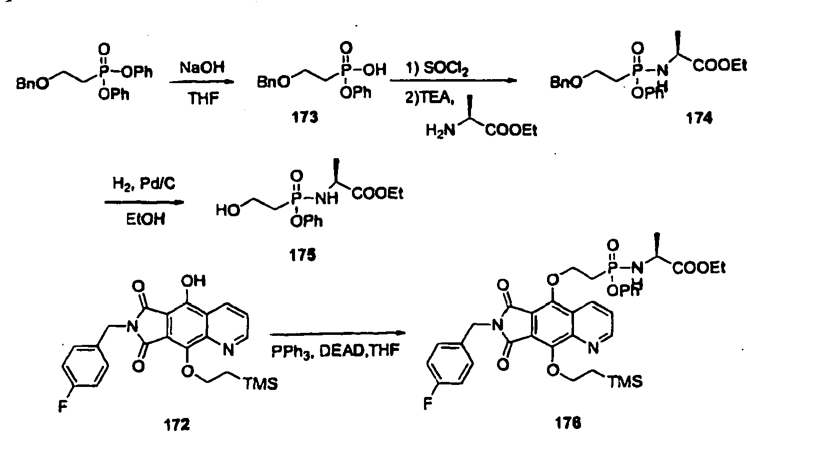

### Example 26

To a solution of (2-benzyloxy-ethyl)-phosphonic acid dibenzyl ester (0.200 g, 0.543 mmol) in THF was added a solution of NaOH (1.36 mL, 1M) in water. The reaction mixture was stirred at room temperature for 3 hours. Most of THF was removed *in vacuo* and the residue was dissolved in water. The aqueous solution was washed with ethylacetate three times then acidified with 1N HCl (to pH=1) then extracted with ethylacetate. The organic phase was dried (MgSO₄), concentrated and co-evaporated with toluene *in vacuo* to afford the mono-acid, (2-benzyloxy-ethyl)-phosphonic acid monobenzyl ester, **173** (0.160 g. 100%) as an oil with no further purification ¹H NMR (CDCl₃) δ 9.25 (bs, 1H), 7.4-7.1 (m, 10H), 4.5 (s, 2H), 3.8 (m, 2H), 2.25 (m, 2H); ³¹P NMR (CDCl₃) δ 28.63.

### Example 27

To a solution of the mono-acid **173** (0.160 g, 0.576 mmol) dissolved in acetonitrile (3.84 mL) was added thionyl chloride (0.42 mL, 5.76 mmol). The reaction mixture was heated to 70 °C and stirred for 3 hours at which point the reaction was completed as shown by ³¹P NMR (CDCl₃) δ 36.7. The reaction mixture was concentrated as such to afford the intermediate mono-chloridate as an oil which was immediately dissolved in dichloromethane (2.88 mL) and treated with triethylamine (0.321 mL, 2.30 mmol). The reaction mixture was cooled to 0 °C and L-alanine ethyl ester (0.265 g, 1.73 mmol) was added. The mixture was stirred overnight at room temperature under an inert atmosphere and then was concentrated *in vacuo.* The residue was partitioned between ethylacetate and saturated NH₄Cl, and the organic phase was washed with brine, dried (MgSO₄) then concentrated *in vacuo*. The residue was purified by chromatography on silica gel washed with methanol prior to use (1/1 - ethylacetate/hexane) to afford the amidate **174** (0.095 g, 45%) as an oil with a 1:1.2 mixture of diastereomers: ¹H NMR (CDCl₃) δ 7.1-7.4 (m, 10H), 4.6 (s, 2H), 4.1 (q, 2H), 3.8 (m, 2H), 3.65 (m, 1H), 2.3 (m, 2H), 1.3 & 1.2 (d, 3H), 1.25 (t, 3H); ³¹P NMR (CDCl₃) δ 29.51, 28.70.

### Example 28

to a solution of the amidate **174** (0.095 g, 0.243 mmol) dissolved in ethanol (4.9 mL) was added palladium (on carbon). The reaction was purged under a vacuum then submitted to hydrogen gas (via balloon attached to the reaction vessel). After several purges between gas and vacuum the reaction mixture was stirred at room temperature for 4 hours. The mixture was filtered with Celite and concentrated *in vacuo* to afford the alcohol **175** (0.74 g, 100%) as an oil with a 1:1.2 mixture of diastereomers without further purification: ¹H NMR (CDCl₃) δ 7.4-7.1 (m, 5H), 4.15 (m, 2H), 3.7 (q, 2H), 3.5 (m, 1H), 2.2 (m, 2H), 1.35 & 1.25 (d, 3H), 1.25 (m, 3H); ³¹P NMR (CDCl₃) δ 30.82, 30.54.

### Example 29

To a solution of phenol **172** (0.073 g, 0.167 mmol) dissolved in THF (1.67 mL) was added the alcohol **175** (0.075 g, 0.25 mmol), triphenylphosphine (0.087 g, 0.33 mmol), and diethyl azodicarboxylate (0.042 mL, 0.33 mmol). The reaction mixture stirred at room temperature under an inert atmosphere overnight. The residue was purified directly by chromatography on silica gel washed with methanol prior to use (80/20 - toluene/acetone) to afford the product **176** (0.065 g, 54%) with a 1:1.2 mixture of diastereomers: ¹H NMR (CDCl₃) δ 9.1 (dd, 1H), 8.8 (dd, 1H), 7.6 (dd, 1H), 7.5 (dd, 2H), 7.4-7.1 (m, 5H), 7.0 (t, 2H), 4.85 (s, 2H), 4.85-4.7 (m, 4H), 4.2 (q, 1H), 4.15 (m, 2H), 4.0-3.8 (m, 1H), 2.65 (m, 2H), 1.4 & 1.25 (d, 3H), 1.3 (m, 2H), 1.2 (m, 3H), 0.10 (s, 9H); ³¹P NMR (CDCl₃) δ 27.84,26.96; MS: 722 (M+1).

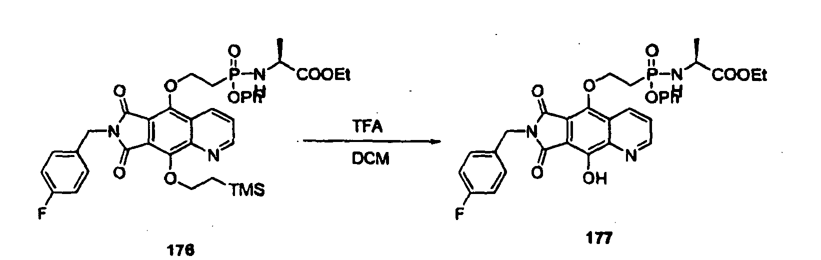

### Example 30

A solution of the phosphonate **176** (0.030 g, 0.042 mmol) in dichloromethane (0.832 mL) was treated with trifluoroacetic acid (0.064 mL, 0.84 mmol). The reaction mixture was stirred at room temperature under an inert atmosphere for 45 minutes. The volatiles were removed *in vacuo* with toluene. The solid was triturated in diethylether/hexane to afford the product **177** (0.022 g, 85%) as a TFA salt with a 1:1.2 mixture of diastereomers:¹H NMR (CDCl₃) δ 9.0 (dd, 1H), 8.85 (dd, 1H), 7.65 (dd, 1H), 7.5 (dd, 2H), 7.4-7.1 (m, 5H), 7.0(t, 2H), 4.85 (s, 2H), 4.85 (m, 2H), 4.15 (m, 1H), 4.15 (m. 1H), 4.1 (m, 2H), 3.8 (m, 1H), 2.65 (m, 2H), 1.35 & 1.30 (d, 3H), 1.2 (m, 3H); ³¹P NMR (CDCl₃) δ 27.86, 27.05; MS: 622 (M+1), 620 (M-1).

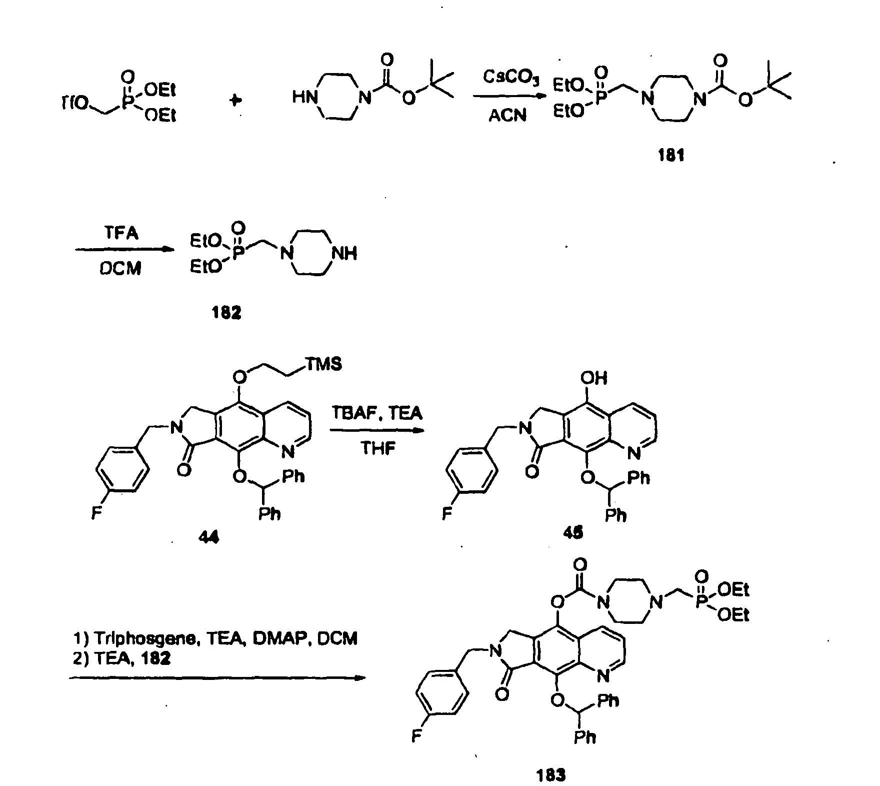

### Example 31

To a solution of 1-BOC-piperazine (0.200 g, 1.08 mmol) in acetonitrile (10.4 mL) was added CsCO₃ (1.05 g, 3.23 mmol) and then cooled to 0 °C. Trifluoromethanesulfonic acid diethoxyphosphorylmethyl ester (0.387 g, 1.29 mmol) dissolved in acetonitrile (5 mL) was added in a dropwise manner. The reaction mixture was stirred at room temperature for 1 hour upon which it was concentrated *in vacuo.* The reaction mixture was taken into ethylacetate then washed with saturated NH₄Cl and brine, dried (MgSO₄), then concentrated *in vacuo.* The residue was purified using silica gel chromatography (3/97 - methanol/dichloromethane) to afford the product **181** (0.310 g, 86%) as an oil: ¹H NMR (CDCl₃) δ 4.15 (m, 4H), 3.45 (t, 4H), 2.8 (d, 2H), 2.6 (m, 4H), 1.45 (s, 9H), 1.35 (t, 6H); ³¹P NMR (CDCl₃) δ 24.03; MS: 337 (M+1).

### Example 32

A solution of the BOC protected piperazine linker phosphonate **181** (0.310 g, 0.923 mmol) in dichloromethane (6.15 mL) was treated with trifluoroacetic acid (0.711 mL, 9.23 mmol). The reaction mixture was stirred at room temperature under an inert atmosphere overnight. The volatiles were removed *in vacuo* with toluene to afford the free piperazine linker phosphonate **182** (0.323 g, 100%) as a TFA salt: ¹H NMR (CDCl₃) δ 11.0 (bs, 1H), 4.2 (m, 4H), 3.45 (t, 4H), 3.35 (m, 4H), 3.2 (d, 2H), 1.4 (t, 6H); ³¹P NMR (CDCl₃) δ 19.16; MS: 237 (M+1).

### Example 33

A solution of the phenol intermediate **45** (0.044 mmol) in dichloromethane (0.441 mL) was treated with triethylamine (0.025 mL, 0.176 mmol) and cat. 4-dimethylaminopyridine. The reaction mixture was cooled to 0 °C then triphosgene (0.026 g, 0.088 mmol) in a 1M solution of dichloromethane was added. The mixture stirred at room temperature under an inert atmosphere for 2 hours, then the free piperazine linker phosphonate **182** (0.046 g, 0.132 mmol) in a 1M solution of dichloromethane treated with triethylamine (0.025 mL, 0.176 mmol) was added, and the mixture was stirred overnight. The mixture was partitioned between dichloromethane and water. The organic phase was washed with saturated NH₄Cl and brine, dried (MgSO₄), and concentrated *in vacuo.* The residue was purified by silica gel chromatography (3/97 - methanol/dichloromethane) to afford the product **183** (0.016 g, 64%): ¹H NMR (CDCl₃) δ 9.05 (dd, 1H), 8.1 (dd, 1H), 8.0 (s, 1H), 7.75 (d, 4H), 7.5 (dd, 1H), 7.4-7.m, 8H), 7.05 (t, 2H), 4.8 (s, 2H), 4.2 (s, 2H), 4.15 (m, 4H), 3.75 (m, 2H), 3.6 (m, 2H), 2.85 (d, 2H), 2.8 (m, 2H), 2.75 (m, 2H), 1.35 (t, 6H); ³¹P NMR (CDCl₃) δ 23.57; MS: 753 (M+1).

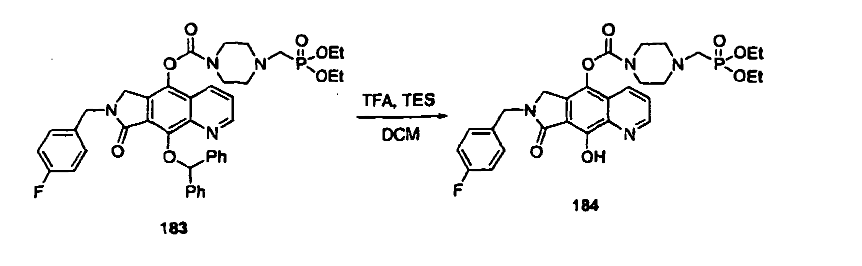

### Example 34

A solution of the phosphonate **183** (0.016 g, 0.021 mmol) in dichloromethane (0.5 mL) was treated with trifluoroacetic acid (0.1 mL) and triethylsilane (0.2 mL). The reaction mixture was stirred at room temperature under an inert atmosphere for 20 minutes. The volatiles were removed *in vacuo* with toluene. The solid was triturated in diethylether/hexane to afford the product **184** (0.0125 g, 100%) as a TFA salt: ¹H NMR (CDCl₃) δ 9.0 (dd, 1H), 8.2 (dd, 1H), 7.6 (dd, 1H), 7.3 (m, 2H), 7.05 (t, 2H), 4.75 (s, 2H), 4.35 (s, 2H), 4.2 (m, 4H), 3.95 (m, 2H), 3.75 (m, 2H), 3.2 (d, 2H), 3.2 (m, 2H), 3.1 (m, 2H), 1.4 (t, 6H); ³¹P NMR (CDCl₃) δ 19.93; MS: 587 (M+1), 585 (M-1).

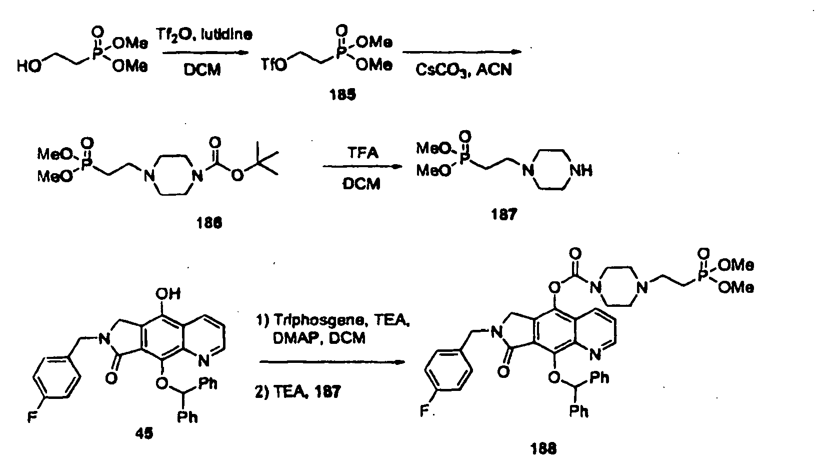

### Example 35

To a solution of (2-hydroxy-ethyl)-phosphonic acid dimethyl ester (0.250 g, 1.62 mmol) in dichloromethane (4 mL) was added 2,6-lutidine (0.284 mL, 2.44 mmol). The reaction mixture was cooled to -40° C and trifluoromethanesulfonic anhydride (0.355 mL, 2.11 mmol) was added. The mixture stirred in the cold bath under an inert atmosphere for 2 hours at which point the reaction was completed as shown by ³¹P NMR (CDCl₃) δ 25.7. The mixture was partitioned between dichloromethane and water both cooled by an ice-water bath. The organic phase was washed with brine, dried (MgSO₄) and concentrated *in vacuo* to afford trifluoromethanesulfonic acid dimethoxy-phosphoryl-2-ethyl ester **185** as an oil which was immediately carried forward with no further purification or characterization.

### Example 36

To a solution of 1-BOC-piperazine (0.252 g, 1.35 mmol) in acetonitrile (14.3 mL) was added CsCO₃ (1.32 g, 4.06 mmol) and then cooled to 0 °C. Trifluoromethanesulfonic acid dimethoxy-phosphoryl-2-ethyl ester **185** (0.464 g, 1.62 mmol) dissolved in acetonitrile (5 mL) was added in a dropwise manner. The reaction mixture was stirred at room temperature overnight upon which it was concentrated *in vacuo.* The reaction mixture was taken into ethylacetate then washed with saturated NH₄Cl and brine, dried (MgSO₄), then concentrated *in vacuo.* The residue was purified using silica gel chromatography (5/95 - methanol/dichloromethane) to afford the BOC protected piperazine linker phosphonate **186** (0.162 g, 31% over 2 steps) as an oil: ¹H NMR (CDCl₃) δ 3.75 (d, 6H), 3.4 (m, 4H), 2.65 (m, 2H), 2.4 (m, 4H), 1.95 (m, 2H), 1.45 (s, 9H); ³¹P NMR (CDCl₃) δ 33.06; MS: 323 (M+1).

### Example 37

A solution of the BOC protected piperazine linker phosphonate **186** (0.162 g, 0.503 mmol) in dichloromethane (3.35 mL) was treated with trifluoroacetic acid (0.388 mL, 5.03 mmol). The reaction mixture was stirred at room temperature under an inert atmosphere overnight. The volatiles were removed *in vacuo* with toluene to afford the free piperazine linker phosphonate **187** (0.169 g, 100%) as a TFA salt: ¹H NMR (CD₃OD) δ 3.8 (d, 6H), 3.45 (m, 4H), 3.2 (m, 4H), 3.15 (m, 2H), 2.3 (m, 2H); ³¹P NMR (CDCl₃) δ 30.92; MS: 223 (M+1).

### Example 38

A solution of the phenol intermediate **45** (0.046 mmol) in dichloromethane (0.458 mL) was treated with triethylamine (0.026 mL, 0.183 mmol) and a catalytic amount of 4-dimethylaminopyridine. The reaction mixture was cooled to 0 °C then triphosgene (0.027 g, 0.092 mmol) in a 1M solution of dichloromethane was added. The mixture was stirred at room temperature under an inert atmosphere for 2 hours, then the free piperazine linker phosphonate **187** (0.046 g, 0.137 mmol) in a 1M solution of dichloromethane treated with triethylamine (0.026 mL, 0.183 mmol) was added dropwise. The mixture was stirred overnight and then partitioned between dichloromethane and water. The organic phase was washed with saturated NH₄Cl and brine, dried (MgSO₄), and concentrated *in vacuo.* The residue was purified by silica gel chromatography (8/92 - methanol/ethylacetate) to afford the product **188** (0.019 g, 56%): ¹H NMR (CDCl₃) δ 9.05 (dd, 1H). 8.1 (dd, 1H), 8.05 (s, 1H), 7.75 (m, 4H), 7.5 (dd, 1H), 7.4-7.1 (m, 8H), 7.1 (t, 2H), 4.8 (s, 2H), 4.2 (s, 2H), 3.8 (d, 6H), 3.6 (m, 4H), 2.75 (m, 2H), 2.55 (m, 4H), 2.1 (m, 2H); ³¹P NMR (CDCl₃) δ 32.65; MS: 739 (M+1).

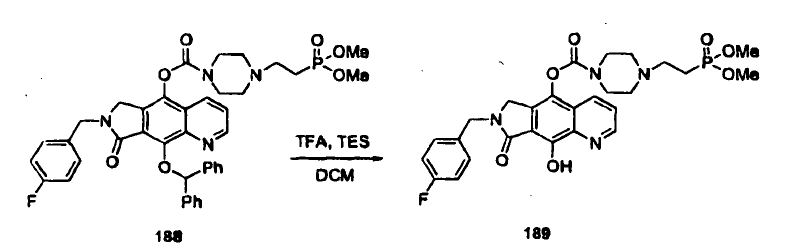

### Example 39

A solution of the phosphonate **188** (0.019 g, 0.026 mmol) in dichloromethane (0.5 mL) was treated with trifluoroacetic acid (0.1 mL) and triethylsilane (0.2 mL). The reaction mixture was stirred at room temperature under an inert atmosphere for 20 minutes. The volatiles were removed *in vacuo* with toluene. The solid was triturated in diethylether/hexane to afford the product **189** (0.013 g, 74%) as a TFA salt: ¹H NMR (CDCl₃) δ 8.9 (dd, 1H), 8.15 (dd, 1H), 7.55 (dd, 1H), 7.35 (m, 2H), 7.05 (t, 2H), 4.75 (s, 2H), 4.35 (s, 2H), 4.2 (m, 2H), 3.95 (m, 2H), 3.8 (d, 6H), 3.4 (m, 4H), 3.35 (m, 2H), 2.4 (m, 2H); ³¹P NMR (CDCl₃) δ 27.31; MS: 573 (M+1).

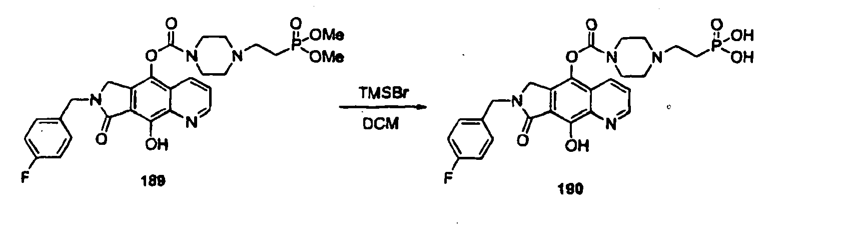

### Example 40

A solution of the phosphonate **189** (0.006 g, 0.009 mmol) in dichloromethane (0.088 mL) was treated with trimethylsilane bromide (0.007 mL, 0.053 mmol). The reaction mixture was stirred at room temperature overnight under an inert atmosphere. The volatiles were removed *in vacuo* with methanol. The solid was washed with dichloromethane to afford the diacid **190** (0.006 g, 100%): ¹H NMR (CD₃OD) δ 9.3 (dd, 1H), 9.2 (dd, 1H), 8.2 (dd, 1H), 7.4 (m, 2H), 7.1 (t, 2H), 4.8 (s, 2H), 4.6 (s, 2H), 3.6-3.2 (m, 10H), 2.35 (m, 2H); ³¹P NMR (CD₃OD) δ 21.43; MS: 545 (M+1), 543 (M-1).

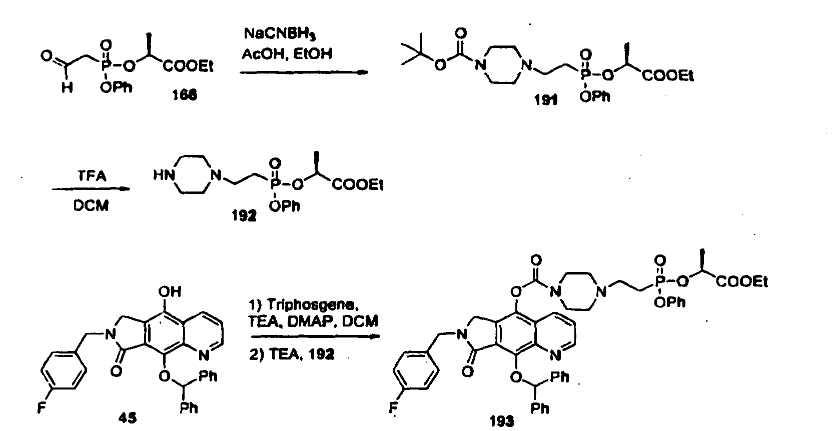

### Example 41

To a solution of 2-[(2-oxo-ethyl)-phenoxy-phosphinoyloxy]-propionic acid ethyl ester, aldehyde **166**, as a 1:1 mixture of DMSO (0.050 g, 0.167 mmol) and 1-BOC-piperazine (0.034 g, 0.183 mmol) dissolved in ethanol (1.67 mL) was added acetic acid (0.038 mL, 0.667 mmol). The reaction mixture was stirred at room temperature for 2.5 hours then sodium cyanoborohydride (0.021 g, 0.333 mmol) was added. The reaction mixture stirred at room temperature overnight. Saturated NaHCO₃ was added to the reaction mixture and was stirred for five more minutes. The mixture was concentrated *in vacuo* to remove most of the ethanol. Brine was added and then the crude product was extracted into ethylacetate. The organic phase was dried (MgSO₄) and concentrated. The residue was purified by silica gel chromatography (5/95 - methanol/dichloromethane) to afford the product **191** (0.050 g, 64%), an oil as a mixture of diastereomers: ¹H NMR (CDCl₃) δ 7.4-7.1 (m, 5H), 5.0 (m, I H), 4.2 (m, 2H), 3.4 (m, 4H), 2.8 (m, 2H). 2.4 (m, 4H), 2.2 (m, 2H), 1.6 & 1.35 (d, 3H), 1.4 (s, 9H), 1.2 (t, 3H); ³¹P NMR (CDCl₃) δ 28.83, 27.18; MS: 471 (M+1).

Alternatively, a solution of 2-[(2-oxo-ethyl)-phenoxy-phosphinoyloxy]-propionic acid ethyl ester **166**, as a 1:1 mixture with DMSO (0.500 g, 1.67 mmol), and piperazine-1-carboxylic acid tert-butyl ester (1-BOC-piperazine, 0.340 g, 1.83 mmol) dissolved in ethanol (1.67 mL) was added 4 A molecular sieves (0.300 g) and acetic acid (0.400 mL, 6.8 mmol). The reaction mixture was stirred at room temperature for 1.5 hours then sodium cyanoborohydride (0.212 g, 3.33 mmol) was added. The reaction mixture stirred at room temperature for 3 hours and was concentrated *in vacuo* then redissolved in chloroform. The mixture was washed with saturated NaHCO₃ and brine, dried (NaSO₄), filtered and concentrated. The residue was treated with diethyl ether. Solid precipitate was filtered off, and the filtrate was concentrated to afford 4-{2-[(1-Ethoxycarbonyl-ethoxy)-phenoxy-phosphoryl]-ethyl}-piperazine-1-carboxylic acid tert-butyl ester **191** (0.600 g, 77%) as an oil (mixture of two diastereomers).

### Example 42

A solution of 4-{2-[(1-ethoxycarbonyl-ethoxy)-phenoxy-phosphoryl]-ethyl}-piperazine-1-carboxylic acid tert-butyl ester **191** (0.050 g, 0.106 mmol) in dichloromethane (0.709 mL) was treated with trifluoroacetic acid (0.082 mL, 1.06 mmol). The reaction mixture was stirred at room temperature under an inert atmosphere for 4 hours. The volatiles were removed *in vacuo* with toluene to afford the free piperazine linker phosphonate **192** (0.051 g, 100%) as a TFA salt (mixture of two diastereomers): ¹H NMR (CDCl₃) δ 10.8 (bs, 1H), 7.5-7.1 (m, 5H), 5.0 (m, 1H), 4.2 (m, 4H), 3.7 (m, 8H), 2.65 (m, 2H), 1.6 & 1.4 (d, 3H), 1.25 (t, 3H); ³¹P NMR (CDCl₃) δ 25.58, 20.86; MS: 371 (M+1).

Alternatively a solution of 4-{2-[(1-ethoxycarbonyl-ethoxy)-phenoxy-phosphoryl]-ethyl}-piperazine-1-carboxylic acid tert-butyl ester **191** (0.100 g, 0.212 mmol) in methylene chloride (2 mL) was treated with trifluoroacetic acid (0.340 mL, 4.41 mmol). The reaction mixture was stirred at room temperature under an inert atmosphere for 6 hours. The volatiles were removed *in vacuo* with ethyl acetate to afford the trifluoroacetate salt of 2-(phenoxy-(2-piperazin-1-yl-ethyl)-phosphinoyloxy]-propionic acid ethyl ester **192** (0.103 g, 100%) (mixture of two diastereomers).

### Example 43

A solution of the phenol intermediate **45** (0.039 mmol) in dichloromethane (0.386 mL) was treated with triethylamine (0.022 mL, 0.155 mmol) and cat. 4-dimethylaminopyridine. The reaction mixture was cooled to 0 °C then triphosgene (0.023 g, 0.077 mmol) in a I M solution of dichloromethane was added. The mixture stirred at room temperature under an inert atmosphere for 2 hours, then the free piperazine linker phosphonate **192** (0.056 g, 0.115 mmol) in a 1M solution of dichloromethane treated with triethylamine (0.022 mL, 0.155 mmol) was added, and the mixture was stirred overnight. The mixture was partitioned between dichloromethane and water. The organic phase was washed with saturated NH₄Cl and brine, dried (MgSO₄), and concentrated *in vacuo.* The residue was purified by silica gel chromatography (5/95 - methanol/dichloromethane) to afford the product **193** (0.013 g, 50%) as a mixture of diastereomers: ¹H NMR (CDCl₃) δ 9.05 (dd, 1H), 8.1 (dd, 1H), 8.05 (s, 1H), 7.75 (d, 4H), 7.5 (dd, 1H), 7.4-7.1 (m, 11H), 7.05 (t, 2H), 5.1 (m, 1H), 4.8 (s, 2H), 4.2 (s, 2H), 4.15 (m, 2H), 3.8-3.4 (m, 4H), 3.0-2.2 (m, 8H), 1.6 & 1.4 (d, 3H), 1.2 (t, 3H); ³¹P NMR (CDCl₃) δ 28.30, 26.59; MS: 887 (M+1).

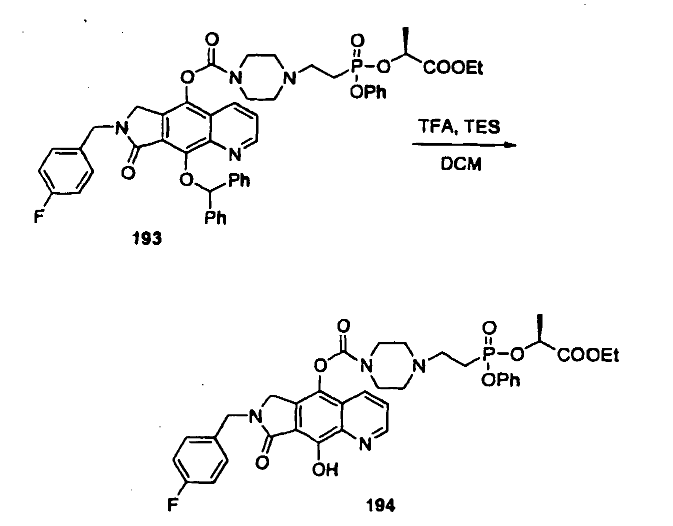

### Example 44

A solution of the phosphonate **193** (0.013 g, 0.015 mmol) in dichloromethane (0.5 mL) was treated with trifluoroacetic acid (0.1 mL) and triethylsilane (0.2 mL). the reaction mixture was stirred at room temperature under an inert atmosphere for 20 minutes. The volatiles were removed *in vacuo* with toluene. The solid was triturated in diethylether/hexane to afford the product **194** (0.010 g, 80%) as a TFA salt: ¹H NMR (CDCl₃) δ 8.95 (dd, 1H), 8.15 (dd, 1H), 7.55 (dd, 1H), 7.35 (m, 2H), 7.3-7.1 (m, 5H), 7.05 (t, 2H), 5.0 (m, 1H), 4.75 (s, 2H), 4.35 (s, 2H), 4.2 (m, 2H), 3.8-3.6 (m, 4H), 3.4-3.0 (m, 6H), 2.5-2.7 (m, 2H), 1.6 & 1.4 (d, 3H), 1.25 (t, 3H); ³¹P NMR (CDCl₃) δ 23.39, 21.67; MS: 721 (M+1).

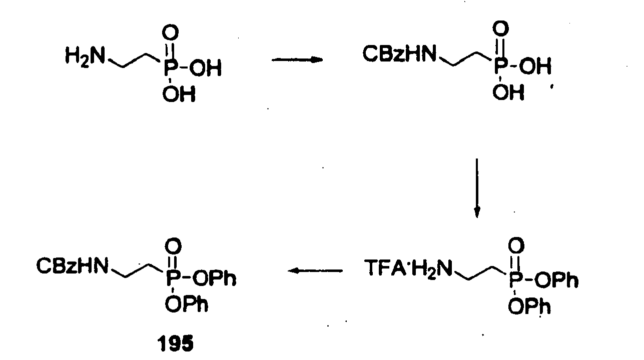

### Example 45

To a solution of 2-aminoethylphosphonic acid (1.26 g, 10.1 mmol) in 2N NaOH (10.1 mL, 20.2 mmol) was added benzyl chloroformate (1.7 mL, 12.1 mmol). After the reaction mixture was stirred for 2 d at room temperature, the mixture was partitioned between Et₂O and water. The aqueous phase was acidified with 6N HCl until pH = 2. The resulting colorless solid was dissolved in MeOH (75 mL) and treated with Dowex 50WX8-200 (7 g). After the mixture was stirred for 30 minutes, it was filtered and evaporated under reduced pressure to give carbobenzoxyaminoethyl phosphonic acid (2.37 g, 91 %) as a colorless solid.

To a solution of carbobenzoxyaminoethyl phosphonic acid (2.35 g, 9.1 mmol) in pyridine (40 mL) was added phenol (8.53 g, 90.6 mmol) and 1,3-dicyclohexylcarbodiimide (7.47 g, 36.2 mmol). After the reaction mixture was warmed to 70°C and stirred for 5 h, the mixture was diluted with CH₃CN and filtered. The filtrate was concentrated under reduced pressure and diluted with EtOAc. The organic phase was washed with sat. NH₄Cl, sat. NaHCO₃, and brine, then dried over Na₂SO₄, filtered, and evaporated under reduced pressure. The crude product was chromatographed on silica gel twice (eluting 40-60% EtOAc/hexane) to give diphenyl 2-aminoethyl phosphonic acid (2.13 g, 57%) as a colorless solid.

To a solution of diphenyl 2-aminoethyl phosphonic acid (262 mg, 0.637 mmol) in iPrOH (5 mL) was added TFA (0.05 mL, 0.637 mmol) and 10% Pd/C (26 mg). After the reaction mixture was stirred under H₂ atmosphere (balloon) for 1 h, the mixture was filtered through Celite. The filtrate was evaporated under reduced pressure to give diphenyl carbobenzoxyaminoethyl phosphonate **195** (249 mg, 100%) as a colorless oil.

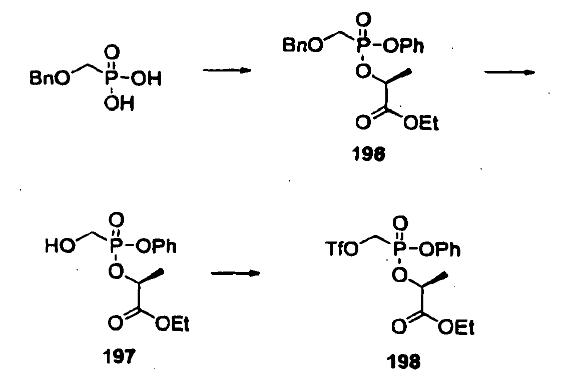

### Example 46

To a solution of benzyloxymethyl phosphonic acid (520 mg, 2.57 mmol) in CH₃CN (5 mL) was added thionyl chloride (0.75 mL, 10.3 mmol) and heated to 70°C in an oil bath. After the reaction mixture was stirred for 2 h at 70°C, the mixture was concentrated and azeotroped with toluene. To a solution of the crude chloridate in toluene (5 mL) was added tetrazole (18 mg, 0.26 mmol) at 0°C. To this mixture was added phenol (121 mg, 1.28 mmol) and triethylamine (0.18 mL, 1.28 mmol) in toluene (3 mL) at 0°C. After the reaction mixture was warmed to room temperature and stirred for 2 h, ethyl lactate (0.29 mL, 2.57 mmol) and triethylamine (0.36 mL, 2.57 mmol) in toluene (2.5 mL) were added. The reaction mixture was stirred for 16 hours at mom temperature, at which time the mixture was partitioned between EtOAc and sat. NH₄Cl. The organic phase was washed with sat. NH₄Cl, 1M NaHCO₃, and brine, then dried over Na₂SO₄, filtered, and evaporated under reduced pressure. The crude product was chromatographed on silica gel (eluting 20-40% EtOAc/hexane) to give two diastereomers (isomer A and isomer B) of 2-(benzyloxymethyl-phenoxy-phosphinoyloxy)-propionic acid ethyl ester **196** (66 mg, 109 mg, 18% total) as colorless oils.

### Example 47a

To a solution of benzyl phosphonate **196** isomer A (66 mg, 0.174 mmol) in EtOH (2 mL) was added 10% Pd/C (13 mg). After the reaction mixture was stirred under H₂ atmosphere (balloon) for 6 h, the mixture was filtered through Celite. The filtrate was evaporated under reduced pressure to give alcohol **197a** isomer A (49 mg, 98%) as a colorless oil.

### Example 47b

To a solution of benzyl phosphonate **196** isomer B (110 mg, 0.291 mmol) in EtOH (3 mL) was added 10% Pd/C (22 mg). After the reaction mixture was stirred under H₂ atmosphere (balloon) for 6 h, it was filtered through Celite. The filtrate was evaporated under reduced pressure to give alcohol **197b** isomer B (80 mg, 95%) as a colorless oil.

### Example 48a

To a solution of alcohol **197a** isomer A (48 mg, 0.167 mmol) in CH₂Cl₂ (2 mL) was added 2,6-lutidine (0.03 mL, 0.250 mmol) and trifluoromethanesulfonic anhydride (0.04 mL, 0.217 mmol) at -40°C (dry ice-CH₃CN bath). After the reaction mixture was stirred for 15 min at -40°C, the mixture was warmed to 0°C and partitioned between Et₂O and 1M H₃PO₄. The organic phase was washed with 1M H₃PO₄ (3 times), dried over Na₂SO₄, filtered, and evaporated under reduced pressure to give triflate **198a** isomer A (70 mg, 100%) as a pale yellow oil.

### Example 48b

To a solution of alcohol **197b** isomer B (80 mg, 0.278 mmol) in CH₂Cl₂ (3 mL) was added 2,6-lutidine (0.05 mL, 0.417 mmol) and trifluoromethanesulfonic anhydride (0.06 mL, 0.361 mmol) at -40°C (dry ice-CH₃CN bath). After the reaction mixture was stirred for 15 min at -40°C, the mixture was warmed to 0°C and partitioned between Et₂O and 1M H₃PO₄. The organic phase was washed with 1M H₃PO₄ (3 times), dried over Na₂SO₄, filtered, and evaporated under reduced pressure to give triflate **198b** isomer B (115 mg, 98%) as a pale yellow oil.

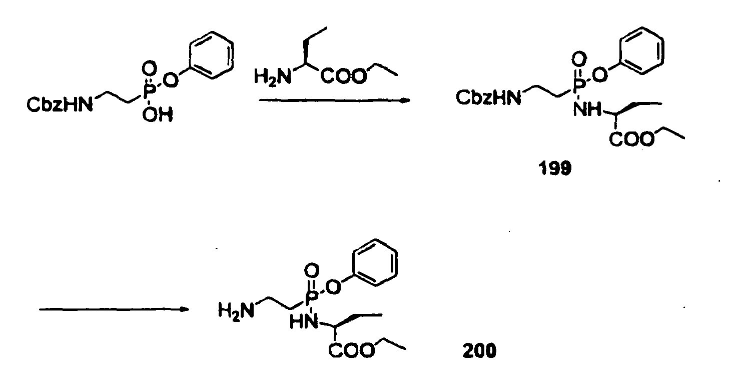

### Example 49

To a stirred solution of phenyl 2-carbobenzoxyaminoethyl phosphonate (1 g, 3 mmol) in 30 mL of acetonitrile at room temperature under N₂ was added thionyl chloride (0.67 mL, 9 mmol). The resulted mixture was stirred at 60-70 °C for 0.5 h. After cooled to room temperature, the solvent was removed under reduced pressure, and the residue was added 30 mL of DCM, followed by DIEA (1.7 mL, 10 mmol), L-alanine butyric acid ethyl ester hydrochloride (1.7 g, 10 mmol) and TEA (1.7 mL, 12 mmol). After 4h at room temperature, the solvent was removed under reduced pressure, and the residue was diluted with DCM and washed with brine and water, dried over Na₂SO₄, filtered and concentrated. The residue was purified by chromatography on silica gel (Hexane/EtOAc 1:1) to give 199 (670 mg, 50%) as a yellow oil. ¹H NMR (CDCl₃) δ 7.33-7.11 (m, 10H), 5.70 (m, 1H), 5.10 (s, 2H), 4.13-3.53 (m, 5H), 2.20-2.10 (m, 2H), 1.76-1.55 (m, 2H), 1.25-1.19 (m, 3H), 0.85-0.71 (m, 3H); ³¹P NMR (CDCl₃) δ 30.2 and 29.9; MS (ESI) 471 (M+Na).

### Example 50

A solution of compound **199** (450mg) was dissolved in 9 mL of EtOH, then 0.15 mL of acetic acid and 10 % Pd/C (90 mg) was added. The resulted mixture was stirred under H2 atmosphere (balloon) for 4 h. Alter tiltration through Celite, the filtered was evaporated under reduced pressure to afford the compound **200** (300mg, 95%) as a colorless oil. ¹H NMR (CDCl₃) δ 7.29-7.12 (m, 5H), 4.13-3.53 (m, 5H), 2.20-2.10 (m, 2H), 1.70-1.55 (m, 2H), 1.24-1.19 (m, 3H), 0.84-0.73(m, 3H); ³¹P NMR (CDCl₃) δ 29.1 and 28.5; MS (ESI) 315 (M+1).

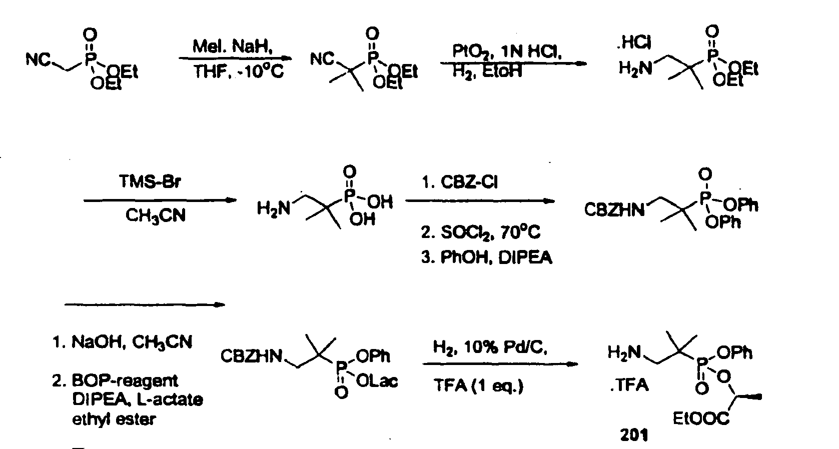

### Example 51

A THF solution (30 mL) ofNaH (3.4 g of 60% oil dispersion, 85 mmol) was cooled to -10°C, followed by the addition of diethyl (cyanomethyl)phosphonate (5g, 28.2 mmol) and iodomethane (17 g, 112 mmol). The resulting solution was stirred at -10°C for 2 hr, then 0°C for 1 hr, was worked up, and purified to give diethyl (cyano(dimethyl)methyl) phosphonate (5 g, 86 %).

Diethyl (cyano(dimethyl)methyl) phosphonate was reduced to the amine derivative by the described procedure (J. Med. Chem. 1999, 42, 5010-5019) whereby a solution of ethanol (150 mL) and 1N HCl aqueous solution (22 mL) of diethyl (cyano(dimethyl)methyl) phosphonate (2.2 g, 10.7 mmol) was hydrogenated at 1 atmosphere in the presence of PtO₂ (1.25 g) at room temperature overnight. The catalyst was filtered through a Celite pad. The filtrate was concentrated to dryness, to give crude diethyl 2-amino-1,1-dimethyl-ethyl phosphonate (2.5g, as HCl salt).

Crude diethyl 2-amino-1,1-dimethyl-ethyl phosphonate (2.5 g) in 30 mL CH₃CN was cooled to 0°C, and treated with TMSBr (8 g, 52 mmol) for 5 hr. The reaction mixture was stirred with methanol for 1.5 hr at room temperature, concentrated, recharged with methanol, concentrated to dryness to give crude 2-Amino-1,1-dimethyl-ethyl phosphonic acid which was used for next reaction without further purification.

2-Amino-1,1-dimethyl-ethyl phosphonic acid was protected with CBZ, followed by the reaction with thionyl chloride at 70°C. The CBZ protected dichloridate was reacted with phenol in the presence of DIPEA. Removal of one phenol, follow by coupling with ethyl L-lactate gave N-CBZ-2-amino-1,1-dimethylethyl phosphonate derivative. Hydrogenation ofN-CBZ derivative at 1 atmosphere in the presence of 10 % Pd/C and 1 eq. of TFA gave lactate phenyl (2-amino-1,1-dimethyl-ethyl)phosphonate **201** as the TFA salt.

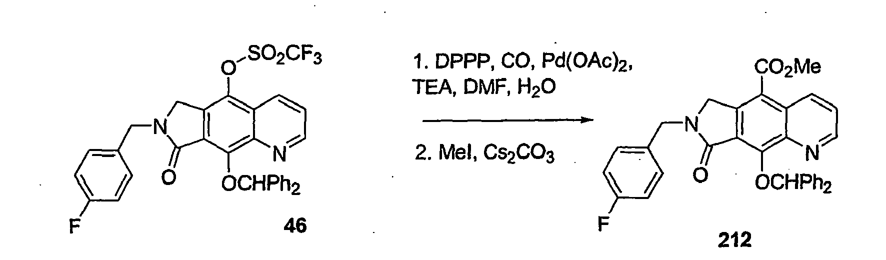

### Example 52

To trifluoro-methanesulfonic acid 9-benzhydryloxy-7- (4-fluoro-benzyl)-8-oxo-7, 8-dihydro-6H-pyrrolo[3,4-g]quinolin-5-yl ester **46** (1.48 g, 2.39 mmol) and 1,3-bis(diphenylphosphino)propane (DPPP) (295 mg, 0.7 mmol) in DMF (20 mL) and water (1 mL) in a two-necked round bottom flask were added Pd(OAc)₂ (107 mg, 0.48 mmol). The solution was degassed under high vacuum and flushed with carbon monoxide from a balloon. The flushing was repeated five times. TEA (0.733 mL, 3.26 mmol) was introduced. The mixture was heated under CO atmosphere for 2.5 hours and cooled down to the room temperature. MeI (0.74 mL, 12 mmol) and Cs₂CO₃ were added and stirring was continued under a nitrogen atmosphere for 45 minutes. The mixture was diluted with EtOAc (300 mL), washed with water, 1N aqueous HCl and brine, dried over MgSO₄ and concentrated. The crude product was purified by chromatography on a silica gel column eluting with 15% to 35% of EtOAc in hexane to afford 9-benzhydryloxy-7-(4-fluoro-benzyl)-8-oxo-7,8-dihydro-6H-pyrrolo[3,4-g]quinoline-5-carboxylic acid methyl ester **212**, (0.9g, 1.69 mmol, 70%) as a yellow solid. ¹H NMR (CDCl₃): δ9.25 (d, 1H), 9.05 (m, 1H), 7.80 (d, 4H), 7.56 (dd, 1H), 7.0-7.4 (m, 11H), 4.85 (s, 2H), 4.55 (s, 2H), 3.95 (s, 3H); MS: 555 (M+Na).

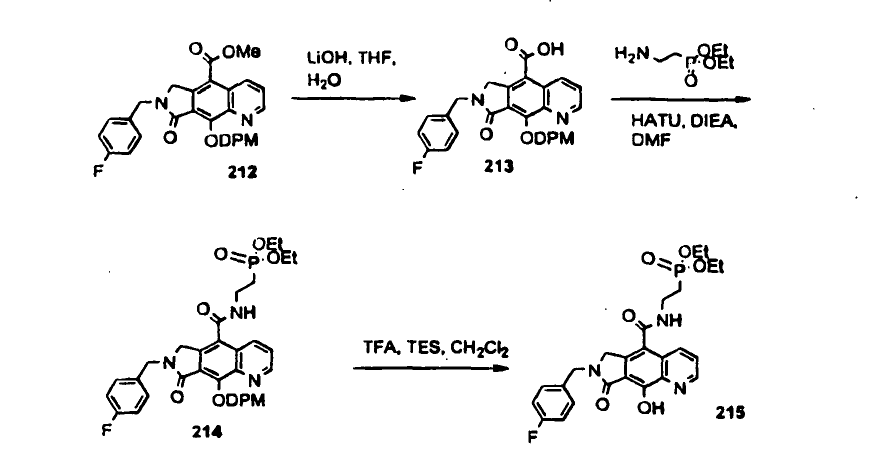

### Example 53

A solution of 9-benzhydryloxy-7-(4-fluoro-benzyl)-8-oxo-7,8-dihydro-6H-pyrrolo[3,4-g]quinoline-5-carboxylic acid methyl ester **212** (54 mg, 0.10 mmol) in 1.0 mL of a 1:1:1 mixture of THF: MeOH: H₂O was stirred with LiOH (9.7 mg, 0.41 mmol) overnight when the starting materials were completely consumed as judged by TLC (DPM = benzhydryl, Ph₂CH-). The reaction mixture was dried under reduced pressure and the residue was dissolved in EtOAc. The organic layer was stirred with saturated aqueous NH₄Cl for 30 minutes. The aqueous layer was checked by TLC to assure complete transfer of the products to the organic layer. The organic layer was dried *in vacuo* to yield 45.5 mg (87%) of 9-benzhydryloxy-7-(4-fluoro-benzyl)-8-oxo-7,8-dihydro-6H-pyrrolo[3,4-g]quinoline-5-carboxylic acid **213** as a white solid. The product was carried on without purification. MS (*m*/*z*) 519.2 [M+H]⁺, 541.2 [M+Na]⁺.

Alternatively, methyl ester **212** (0.071g, 0.1334mmol) was dissolved in 2.4 mL of tetrahydrofuran and 0.6 mL of DI H₂O. To this was added LiOH (0.013g, 0.5338mmol) and mixture stirred at room temperature. After 15 hours, starting material consumed. Diluted with dichloromethane, washed with 1M HCl solution, dried (Na₂SO₄), concentrated to give **213** (0.068g, 0.1313 mmol, 98%.) ¹H NMR (CD₃SOCD₃) δ 9.25 (d, 1H), 9.12 (dd, 1H), 8.17 (s, 1H), 7.75 (d, 5H), 7.37 (dd, 2H), 7.24 (m, 6H), 4.82 (s, 2H), 4.59 (s, 2H.) MS: 517 (M-1.)

### Example 54

A solution of the oxalate salt (HO₂CCO₂) of diethyl(aminoethyl)phosphonate (12 mg, 0.042 mmol) in 0.21 mL of DMF was mixed with DIEA (15 µL, 0.084 mmol) until the reaction became clear. To this solution was added **213** (11 mg, 0.021 mmol) and O-(7-azabenzotriazol-1-yl)-N,N,N',N'-tetramethyluronium hexafluorophosphate (HATU) (16 mg, 0.042 mmol). This mixture was stirred at room temperature for 2 hours when it was warmed to 60 °C with a heat gun for I minute. LCMS analysis demonstrated complete consumption of the starting materials. The reaction mixture was directly loaded onto a silica gel column and the product was quickly eluted with a gradient of EtOAc-10% MeOH/ EtOAc to provide 12.7 mg (88%) of the product **214.** ¹H NMR (300 MHz, CD₃OD) δ 1.29 (t, 6, *J*= 7 Hz), 2.18 (dt, 2H, *J*= 7, 18 Hz), 3.53-3.65 (m, 2H), 4.08 (septet, 4H, *J*= 7 Hz), 4.46 (s, 2H), 4.83 (s, 2H), 7.06- 7.25 (m, 8H), 7.40 (dd, 2H, *J*= 5, 9 Hz), 7.61- 7.68 (m, 6H), 8.04 (s, 1H), 8.44 (d, 1H, *J*= 7 Hz), 9.04-9.09 (m, 1H); ³¹P (121.4 MHz, CD₃OD) δ 29.5; MS (*m*/*z*) 682.1 [M+H]⁺, 704.2 [M+Na]⁺.

### Example 55

A solution of **214** (12.7 mg, 0.019 mmol) in 0.19 mL of CH₂Cl₂ was stirred with TFA (144 µL, 1.9 mmol) and TES (304 µL, 1.9 mmol) for 45 minutes under a N₂ atmosphere. TLC and LCMS analysis indicated complete reaction at that time. The reaction was worked up by removing the solvent under reduced pressure. The residue was purified by crystallization from EtOAc-Hex to yield 8.6 mg (71%) of (2-{[7-(4-fluoro-benzyl)-9-hydroxy-8-oxo-7,8-dihydro-6H-pyrrolo[3,4-g]quinoline-5-carbonyl]-amino}-ethyl)-phosphonic acid diethyl ester 215 as a yellow solid. ¹H NMR (500 MHz, CD₃OD) δ 1.33 (t, 6H, *J*= 7 Hz), 2.24 (dt, 2H, *J*= 19, 7 Hz), 3.70 (septet, 2H, *J*= 8 Hz), 4.09- 4.17 (m, 4H), 4.61 (s, 2H), 4.78 (s, 2H), 7.10 (t, 2H, *J*= 9 Hz), 7.41 (dd, 2H, *J*= 6, 8 Hz), 7.76 (br d, 1H, *J*= 5 Hz), 8.71 (d, 1H, *J=* 9 Hz), 8.95 (br s, 1H); ³¹P (121.4 MHz, CD₃OD) δ 29.5; MS (*m*/*z*) 516.3 [M+H]⁺, 1030.9 [2M]⁺, 1053.0 [2M+Na]⁺.

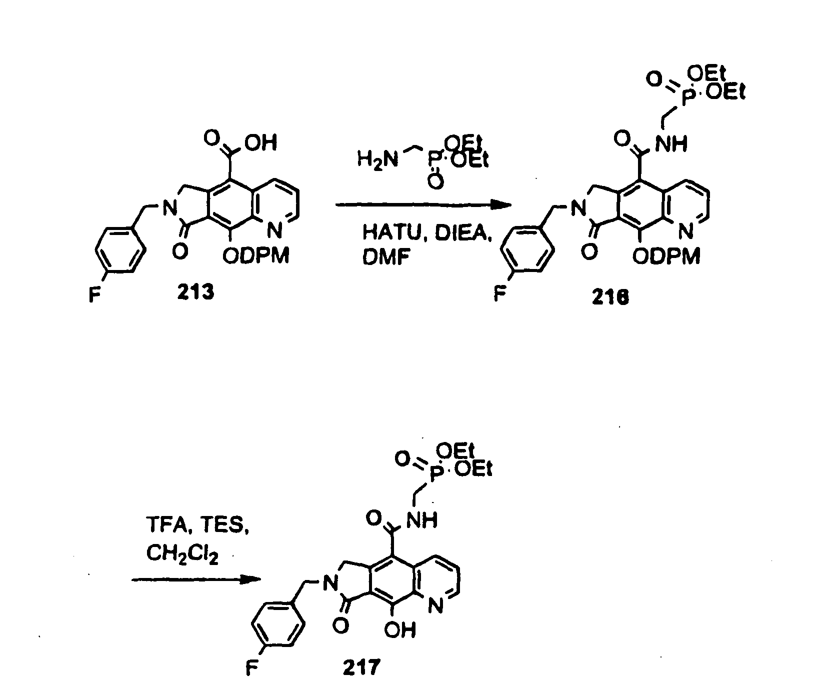

### Example 56

A solution of oxalate salt of diethyl(aminomethyl)phosphonate (8 mg, 0.031 mmol) in 0.31 mL of DMF and DIEA (22 µL, 0.124 mmol) was added to **213** (16 mg, 0.031 mmol) and HATU (24 mg, 0.062 mmol). The solution was stirred at ambient temperature for 2 hours when another batch of the amine and the coupling reagent equivalent to the above amounts were added. The reaction was heated with a heat gun to 60 °C for 1 minute and the reaction was analyzed by LCMS. The reaction mixture was loaded onto a flash column and ({[9-benzhydryloxy-7-(4-fluoro-benzyl)-8-oxo-7,8-dihydro-6H-pyrrolo[3,4-g]quinoline-5-carbonyl]-amino}-methyl)-phosphonic acid diethyl ester **216** was eluted with EtOAc- 10% MeOH to provide 20 mg (97%) of a clear oil. MS (*m*/*z*) 668.1 [M+H]⁺, 690.3 [M+Na]⁺.

### Example 57

A solution of **216** (20 mg, 0.030 mmol) in 0.30 mL of CH₂Cl₂ was stirred with TFA (231 µL, 3.00 mmol) and TES (479 µL, 3.00 mmol) for 30 minutes when the starting materials were completely consumed as judged by TLC and LCMS. The reaction was worked up by removal of the solvent *in vacuo* and crystallizing the product from EtOAc- Hex to provide 10 mg (66%) of ({[7-(4-Fluoro-benzyl)-9-hydroxy-8-oxo-7,8-dihydro-6H-pyrrolo[3,4-g]quinoline-5-carbonyl]-amino}-methyl)-phosphonic acid diethyl ester **217** as a yellow solid. ¹H NMR (300 MHz, CD₃OD) δ 1.32 (t, 6H, *J*= 7 Hz), 3.96 (d, 2H, *J*= 12 Hz), 4.16 (septet, 4H, *J*= 7 Hz), 4.56 (s, 2H), 4.79 (s, 2H), 7.10 (t, 2H, *J*= 9 Hz), 7.39 (dd, 2H, *J*= 9 Hz), 7.76 (br s, 1H), 8.66 (d, 1H, *J*= 8 Hz), 8.95 (br s, 1H); ³¹P (121.4 MHz, CD₃OD) δ 23.2; ¹⁹F NMR (282.6 MHz, CD₃OD) δ -76.2, 59.9; MS (*m*/*z*) 502.5 [M+H]⁺, 1003.0 [2M]⁺, 1025.1 [2M+Na]⁺.

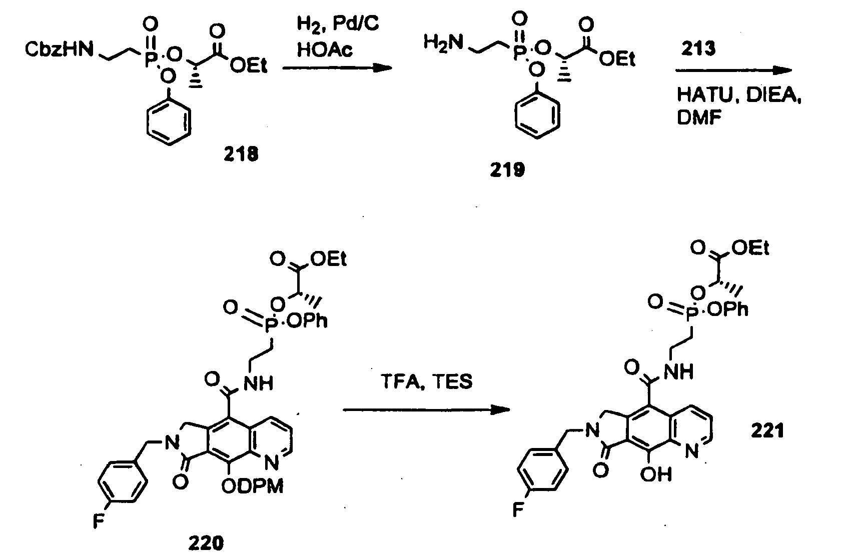

### Example 58

*S*-lactate ester **218** was prepared from phenyl 2-carbobenzoxyaminoethyl phosphonate by the coupling and hydrogenation procedures described in Example 201.

### Example 59

A solution of 2-[(2-benzyloxycarbonylamino-ethyl)-phenoxy-phosphinoyloxy]-propionic acid ethyl ester **218** (240 mg, 0.551 mmol) with approximately 50% purity and a ratio of 2:1 of diastereomers was dissolved in 5.5 mL of ethanol with acetic acid (63 µL, 1.10 mmol). To this solution was added 36 mg of 10% Pd/C and the solution was degassed under a hydrogen atmosphere three times. The solution was vigorously stirred at room temperature for 3 hours when TLC showed complete consumption of the starting materials. The mixture was filtered through a pad of Celite and dried to provide 174 mg (87%) of 2-[(2-amino-ethyl)-phenoxy-phosphinoyloxy]-propionic acid ethyl ester; compound with acetic acid **219** as a clear oil.

### Example 60

A solution of 13.5 mg of **213** in 0.13 mL of DMF was stirred with HATU (20 mg, 0.052 mmol) at room temperature for 10 minutes. To this solution was added a premixed solution of **219** (28 mg, 0.078 mmol) of approximately 50% purity in 0.130 mL of DMF and DIEA (13.4 mg, 0.104 mmol). The reaction mixture was gently heated with a heat gun for 30 seconds and then the reaction was allowed to proceed at room temperature for 2 hours when LCMS demonstrated complete consumption of the carboxylic acid. The reaction mixture was loaded onto a silica gel column and purified with EtOAc- 10% MeOH to provide 9.5 mg of 3-[(2-{[9-Benzhydryloxy-7-(4-fluoro-benzyl)-8-oxo-7,8-dihydro-6H-pyrrolo[3,4-g]quinoline-5-carbonyl]-amino}-ethyl)-phenoxy-phosphinoyl]-2-methyl-propionic acid ethyl ester **220** which was carried on to the next step.

### Example 61

A solution of **220** (9.5 mg, 11.8 µmol) was stirred with 0.12 mL of dry dichloromethane with trifluoroacetic acid (93 µL, 1.18 mmol) and triethylsilane (189µL, 1.18 mmol) for 1 hour at room temperature when TLC showed complete consumption of the starting materials. The reaction mixture was dried *in vacuo* and azeotroped from dichloromethane three times. The solid product was triturated with EtOAc- Hex to get 6 mg of 2-[(2-{[7-(4-Fluoro-benzyl)-9-hydroxy-8-oxo-7,8-dihydro-6H-pyrrolo[3,4-g]quinoline-5-carbonyl]-amino}-ethyl)-phenoxy-phosphinoyloxy]-propionic acid ethyl ester **221** as a pale yellow solid. The NMR of the two diastereomers in CDCl₃ is broad and indicates presence of rotamers. VT NMR in DMSO at 85 °C resulted in drastic sharpening of the peaks. ¹H NMR (300 MHz, DMSO- *d*6, 85 °C) δ 1.15-1.26 (m, 3H), 1.35 and 1.47 (d, 3H, *J*=7 Hz), 2.23- 2.45 (m, 2H), 3.58- 3.57 (m, 2H), 4.08- 4.19 (m, 2H), 4.56 (s, 2H), 4.69 (s, 2H), 4.93- 5.04 (m, 1H), 7.14 (t, 2H, *J*= 9 Hz), 7.18- 7.23 (m, 3H), 7.35- 7.42 (m, 4H), 7.65 (dd, 1H, *J*= 4, 8 Hz), 8.42 (br s, 1H), 8.55 (d, 1H, *J*= 9 Hz), 8.92 (d, 1H, *J*=4H); ³¹P (121.4 MHz), DMSO-*d*6, 85 °C) δ 26.1, 28.3; MS (*m*/*z*) 636.5 [M+H]⁺.

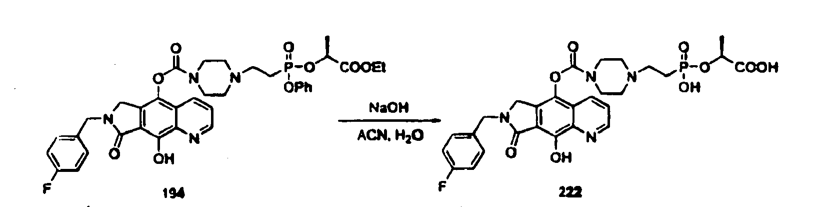

### Example 62

A solution of the trifluoroacetate salt of 4-{2-[(1-ethoxycarbonyl-ethoxy)-phenoxy-phosphoryl]-ethyl}-piperazine-1-carboxylic acid 7-(4-fluoro-benzyl)-9-hydroxy-8-oxo-7,8-dihydro-6H-pyrrolo[3,4-g]quinolin-5-yl ester **194** (0.045 g, 0.054 mmol) in acetonitrile (ACN, 0.68 mL) and water (0.68 mL) was treated with an aqueous solution of NaOH (0.162 mL, 1M). The reaction mixture was stirred at room temperature for 3 hours. The mixture was cooled to 0° C, then acidified with a 2N aqueous solution of HCl to pH=1. Acetonitrile was removed *in vacuo* then purified by reversed phase HPLC to afford the trifluoroacetate salt of 4-{2-[(1-carboxy-ethoxy)-hydroxy-phosphoryl]-ethyl}-piperazine-1-carboxylic acid 7-(4-fluoro-benzyl)-9-hydroxy-8-oxo-7,8-dihydro-6H-pyrrolo[3,4-g]quinolin-5-yl ester, compound with trifluoro-acetic acid **222** (0.032 g, 80%): ¹H NMR (CD₃OD) δ 9.0 (d, ¹H), 8.5 (d, 1H), 7.75 (dd, 1H), 7.4 (dd, 2H), 7.1 (t, 2H), 4.8 (s, 2H), 4.45 (s, 2H), 4.3-3.7 (m, 4H), 3.7-3.35 (m, 6H), 2.2 (m, 2H), 1.55 (d, 3H); ³¹P NMR (CDCl₃) δ 19.8; MS: 617 (M+1).

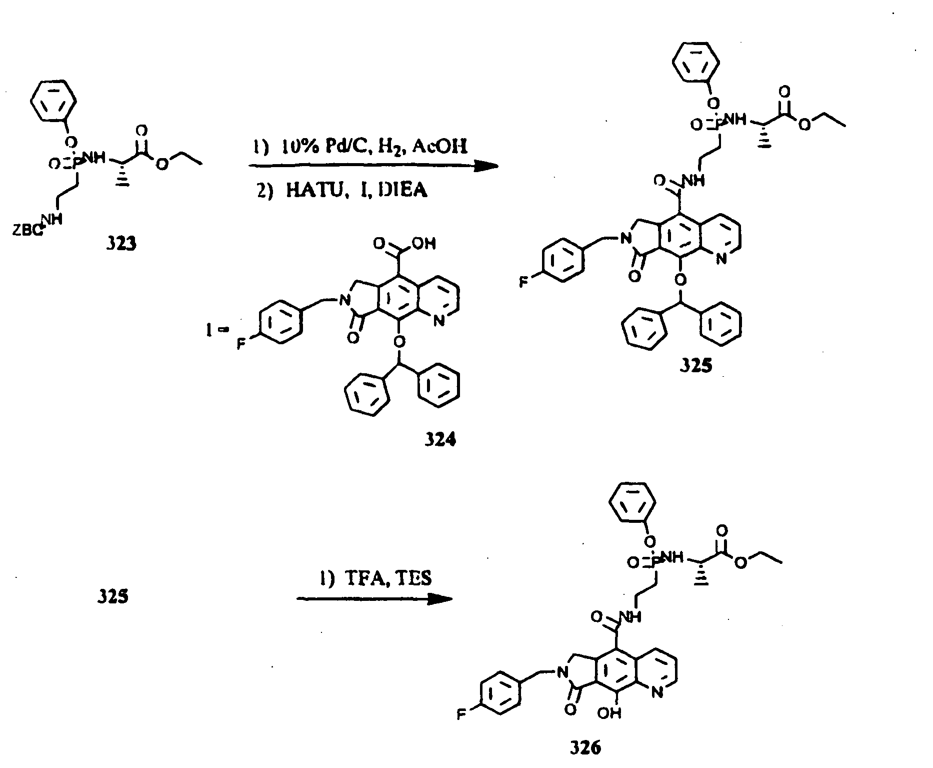

### Example 63

400 mg (0.9mmol) of **323** dissolved in 3 ml of ethanol and 216 ul of acetic acid was allowed to react with 100 mg of 10% palladium on carbon and H₂ (1 atm) for 30 minutes. The reaction was then filtered through celite and concentrated *in vacuo.* The residue was then dissolved in DCM, washed once with saturated Na₂CO₃, and concentrated *in vacuo.* The residue was then added to I (155 mg, 0.3 mmol) which had been reacting with HATU (220 mg, 0.6 mmol) in DMF for 10 minutes. This mixture was then treated with DIEA (155.1 mg, 1.2 mmol) and stirred at room temperature for one hour. The mixture was diluted with ethyl acetate, washed with saturated NaHCO₃ (twice), water (twice) and brine (twice), dried (Na₂SO₄), and concentrated. The residue was purified by silica gel chromatography (2% methanol in ethyl acetate) to afford 2 (213 mg, 80 % yield)

**325** (0.1 g, 0.12 mmol) in methylene chloride (0.9 mL) was treated with trifluoracetic acid (0.045 mL) and triethylsilane (0.075 mL). The reaction mixture was stirred at room temperature under an inert atmosphere for 40 minutes. The volatiles were removed *in vacuo* with toluene. The product was triturated ( 1/3 - diethyl ether/hexane with sonicaton to afford **326** ( 75 mg, 83 % yield) as the monotriflouroacetate salt. ¹H NMR (CD₃OD) δ 8.89 (bs, 1H), 8.70 (bs, 0.5 H), 8.65 (d, 1.0), 7.67 (bs, 1 H), 7.35 (m, 4H), 7.22 (t, 3H), 7.10 (t, 3H), 4.74 (bs, 2H), 4.59 (bs, 2H), 4.10 (m, 2H), 3.97 (m, 1H), 3.84 (bs, 2H), 2.39 (m, 2H). ³¹P NMR (CDCl₃) δ 30.10, 29.16. MS = 635.1 (M + 1).

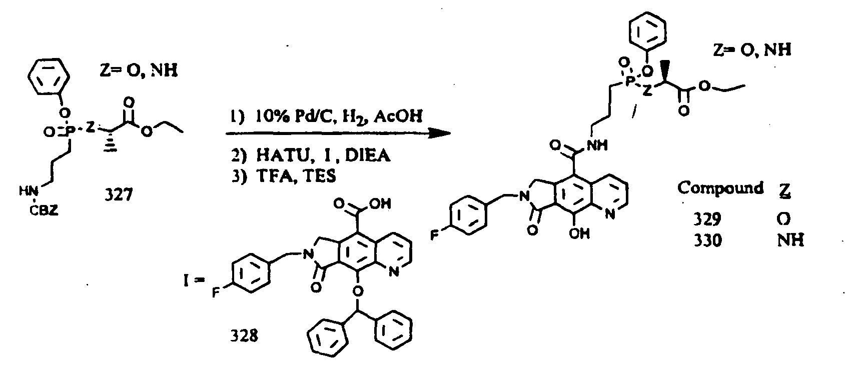

### Example 64

The syntheses of **329** and **330** from starting materials of type **327** were carried out in a manner similar to that stated previously. **329** ¹H NMR (CD₃OD) δ 8.90 (bs, 1H), 8.57 (b, 1H), 7.70 (b, 1H), 7.37 (m, 4H), 7.24 (t, 3H), 7.10 (t, 3H), 4.73 (b, 2H), 4.57 (b, 2H), 4.15 (q, 2H), 3.59 (b, 2H), 2.13 (m, 4H), 1.53 (d, 2H), 1.39 (d, 2H), 1.22 (q, 3H). ³¹P NMR (CDCl₃) δ 30.75, 29.47. MS: 651 (M+1). **330** ¹H NMR (CD₃OD) δ 8.90 (bs, 1 H), 8.75 (bs, 0.5 H), 8.60 (bs, 1H), 7.75 (m, 1H), 7.37 (m, 4H), 7.24 (t, 3H), 7.10 (t, 3H), 4.80 (bs, 2H), 4.60 (bs, 2H), 4.10 (q, 2H), 3.95 (m, 1H), 3.58 (bs, 2H), 2.10 (m, 2H), 1.28 (d, 2H), 1.20 (m, 4H). ³¹P NMR (CDCl₃) δ 34.18, 33.40. MS: 649 (M+1).

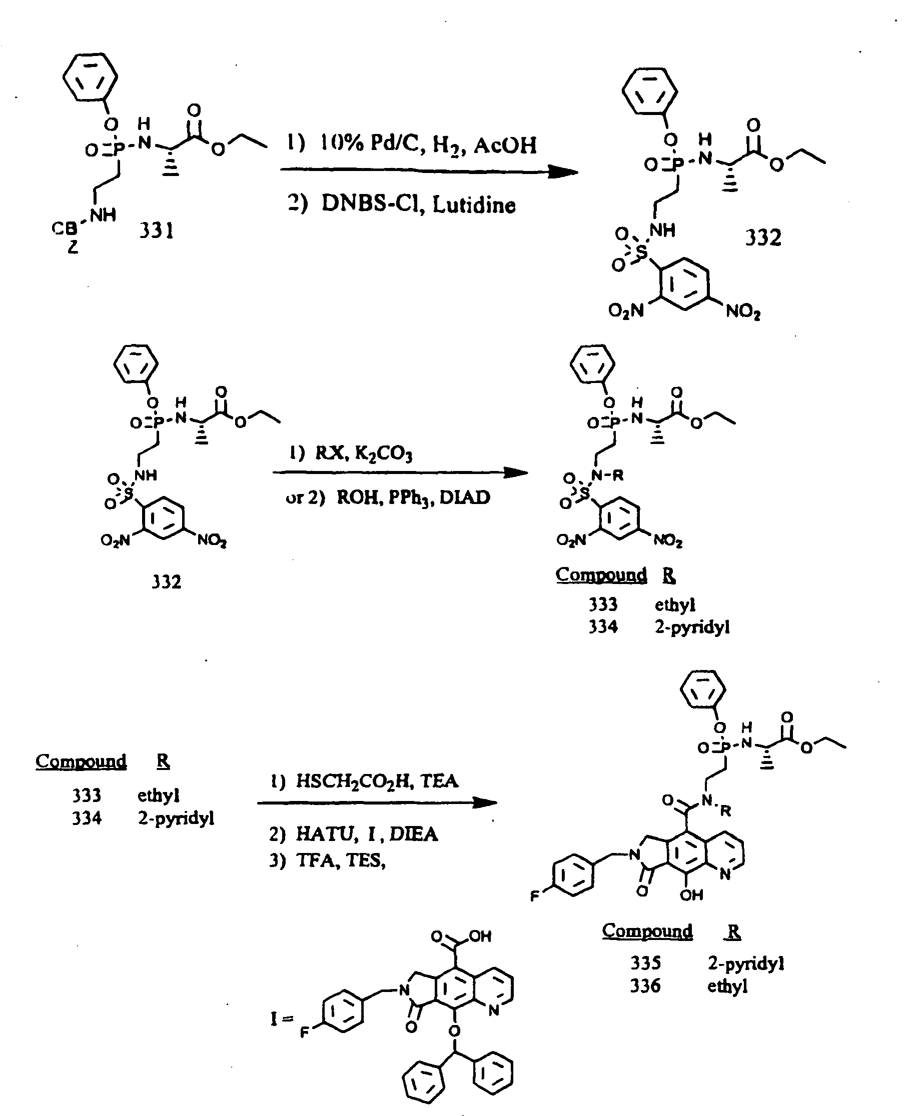

### Example 65

990 mg (2.3 mmol) of **331** dissolved in 10 ml of ethanol and 0.3 ml of acetic acid was treated with 300 mg of 10% palladium on carbon and placed under a hydrogen balloon. After 1 hour, the reaction was filtered through celite and concentrated *in vacuo*. The residue was then partitioned between DCM and saturated Na₂CO₃. The organic layer was then collected and concentrated *in vacuo.* The residue was then dissolved in DCM and lutidine (0.801 ml, 6.9 mmol). To this reaction mixture was added 1.8 g (6.9 mmol) of Dinitrobenzenesulfonyl chloride. After stirring for 30 minutes at room temperature, the reaction was quenched with 1.2 ml of acetic acid and concentrated *in vacuo.* The residue was then diluted with ethyl acetate, washed once with saturated NH₄Cl, once with water, dried over magnesium sulfate, and concentrated *in vacuo.* The residue was then purified by silica gel chromatography ( neat ethyl acetate) to give **795** mg (52 % yield) of **332**.

**332** was then alkylated either by treatment with an alkyl halide in the presence of K₂CO₃ or by treatment with an alkyl alcohol in the presence of PPh₃ and DIAD. For alkylation by treatment with and alkyl halide, the following procedure was followed:
2 (80 mg, 0.19 mmol) dissolved in DMF was allowed to react with iodoethane and K₂CO₃ for 1 hour. The reaction was then diluted with ethyl acetate and washed once with saturated ammonium chloride, once with 2.5 % aqueous LiCl, once with water, once with brine, dried over Mg₂SO₄, and concentrated *in vacuo.* The residue was then purified by silica gel chromatography ( neat ethyl acetate ) to afford 60 mg (52 % yield) of **333**.

For alkylation by treatment with an alkyl alcohol, the following procedure was followed: 2 ( 100 mg, 0.19 mmol ) dissolved in DCM was allowed to react with 2-pyridyl-carbinol (62.2 mg, 0.57 mmol), PPh3 (150 mg, 0.57 mmol), and DIAD (115.2 mg, 0.57 mmol) for one hour at room temperature. The reaction was then concentrated and purified by silica gel chromatography ( neat ethyl acetate ) to afford 94 mg ( 80% yield) of **334**.

Compounds **333** and **334** were then converted to compounds **336** and **335** respectively using the following representative procedure: **334** (47 mg, 0.08 mmol) dissolved in DCM was allowed to react with mercaptoacetic acid ( 14,7 mg, 0.16 mmol) and TEA ( 16.2 mg, 0.16 mmol) for 30 minutes. The reaction was then diluted with DCM, washed twice with saturated Na₂CO₃, and concentrated *in vacuo.* Amide bond formation between the crude residue and I was then performed in a similar manner as stated previously to give **335**. **335** ¹H NMR (CD₃OD) δ 8.91 (m, 1H), 8.52 (m, 1H), 7.95 (m, 1H), 7.71 (m, 2H), 7.54 (m, 2H), 7.39 (m, 4H), 7.24 (m, 311), 7.12 (m, 3H), 6.88 (m, 1H), 4.71 (m, 1H), 4.52 (m, 4H), 4.01 (m, 2H), 3.98 (m, 1H), 3.64 (m, 2H), 2.30 (m, 2H), 1.32 (m, 6H). ³¹P NMR (CDCl₃) δ 31.363, 31.132, 30.483, 30.237, 29.112, 29.091, 28.598, 28.428. MS: 726 (M+1). **336** ¹H NMR (CD₃OD) δ 8.96 (bs, 1H), 8.30 (m, 1H), 7.74 (b, 1H), 7.38 (m, 4H), 7.24 (m, 3H), 7.10 (m, 3H), 6.83 (m, 1 H), 4.69 (t, 1H), 4.43 (b, 1H), 4.07 (m, 4H), 3.65 (m, 2H), 3.20 (m, 2H), 2.28 (m, 2H), 1.17 (m, 10H). ³¹P NMR(CDCl₃) δ 31.06, 30.10,28.95, 28.14. MS: 663 (M+1).

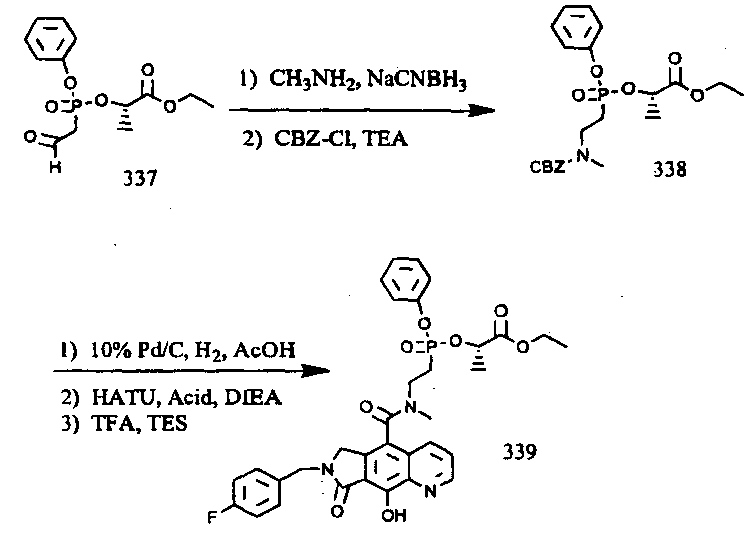

### Example 66

337 (200 mg, 0.67 mmol) dissolved in DMF was allowed to react with acetic acid (0.264 ml, 4.5 mmol) and 2.0 M CH₃NH₂ in THF ( 2.0 ml, 4.0 mmol) at room temperature for 2 hours. TFA (0.31 ml, 4.5 mmol) and NaCNBH₃ (140 mg, 2.2 mmol) were then added and the reaction was stirred for another hour. The reaction was then diluted with ethyl acetate, washed twice with saturated sodium carbonate, dried over magnesium sulfate, and concentrated *in vacuo.*

The crude residue was then diluted with DCM and allowed to react with TEA ( 0.6 ml, 4.3 mmol) and CBZ-CL (0.366 ml, 2.6 mmol) for 12 hours at room temperature. The reaction was then concentrated *in vacuo,* diluted with ethyl acetate, washed once with 5 % citric acid (aqueous), once with water, once with brine, dried over magnesium sulfate, and concentrated *in vacuo.* The residue was then purified by silica gel chromatography ( neat ethyl acetate ) to afford 7 (168.8 mg, 56 % yield ).

**338** was then converted to **339** in a manner similar to that stated previously. ¹H NMR (CD₃OD) δ 8.91 (m, 1H), 8.31 (m, 1H), 7.68 (m, 1H), 7.36 (m, 4H), 7.23 (m, 3H), 7.07 (m, 3H), 6.81 (m, 1H), 4.94 (m, 1H), 4.79 (m, 1H), 4.63 (m, 3H), 4.41 (m, 2H), 4.17 (m, 2H), 3.96 (m, 1H), 3.55 (m, 1H), 3.17 (s, 1.5 H), 2.84 (s, 1.5 H), 2.59 (m, 1H), 1.53 (m, 1H), 1.37 (m, 1H), 1.2 (m, 4H), 1.03 (d, 1H). ³¹P NMR (CD₃OD) δ 27.70, 27.65, 25.90, 25.75. MS: 650 (M+1).

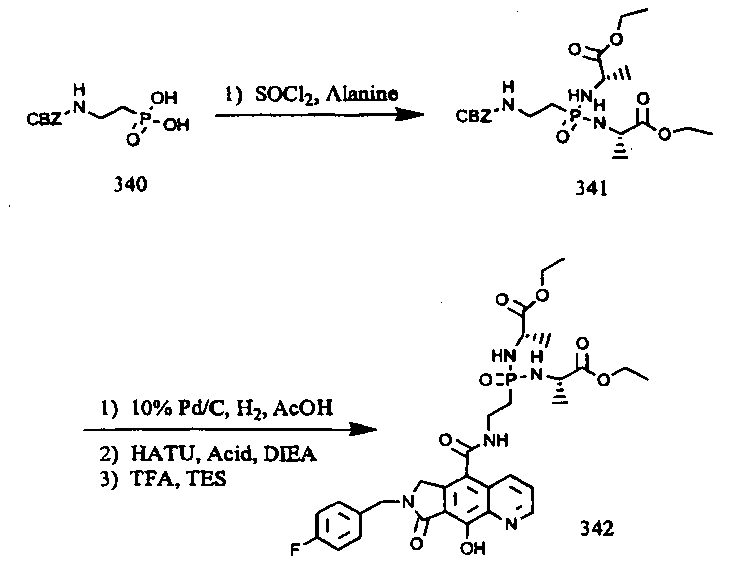

**340** ( 200 mg, 0.73 mmol) dissolved in toluene was allowed to react with thionyl chloride ( 348 mg, 2.93 mmol) for one hour at 65 C. The mixture was then concentrated *in vacuo* and azeotroped three times with toluene. The residue was then dissolved in DCM, cooled to 0 C, and allowed to react with L-Alanine ethyl ester (432 mg, 3.66 mmol) for one hour. The reaction was then quenched by addition of acetic acid ( 0.6 ml) and concecntrated *in vacuo.* The residue was then diluted with ethyl acetate, washed once with water, once with brine, dried over magnesium sulfate, and concentrated *in vacuo.* The residue was then purified by silica gel chromatography (5 % methanol in ethyl acetate) to afford **341** (103 mg, 30 % yield). **341** was then converted into **342** in a manner similar to that stated previously. ¹H NMR (CD₃OD) δ 8.88 (d, 1H), 8.61 (t, 1H) 8.55 (d, 2H), 7.68 (m, 1H), 7.36 (m, 2H), 7.05 (t, 2H), 4.73 (s, 2H), 4.54 (s, 2H), 4.26 (q, 1H), 4.12(q, 2H), 4.03 (m, 2H), 3.90 (m, 2H), 3.47 (bs, 2H), 1.84 b(m, 2H), 1.49 (d, 1H), 1.33 (t, 7H), 1.21 (m, 7H). ³¹P NMR (CD₃OD) δ 31.6. MS: 672 (M+1).

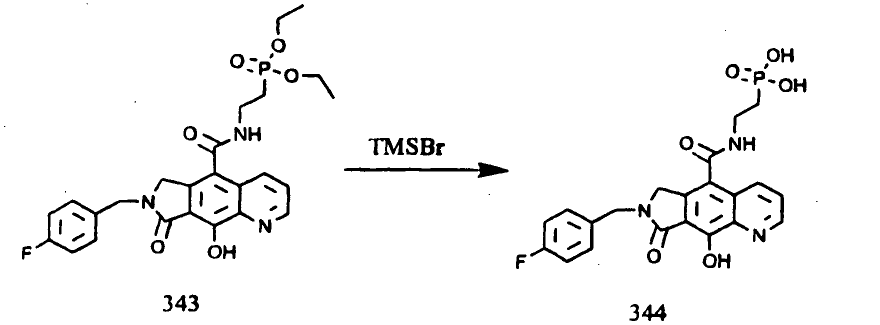

### Example 67

Following the procedure in Example 17, compound **343** was converted to compound **344**. ¹H NMR (CD₃OD) δ 8.96 (d, 1H), 8.76 (d, 1H), 7.79 (m, 1H), 7.42 ( q, 2H), 7.11 (t, 2H), 4.79 (s, 2H), 4.61 (s, 2H), 3.71 (dt, 2H), 2.13 (dt, 2H). ³¹P NMR (CD₃OD) δ 25.834. MS: 460 (M+1).

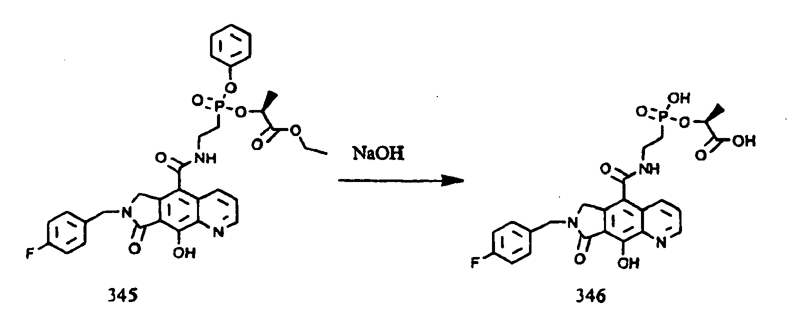

### Example 68

Following the procedure in Example 62, compound **345** was converted to compound **346**. ¹H NMR (CD₃OD) δ 8.96 (d, 1H), 8.83 (d, 1H), 7.81 (m, 1H), 7.42 (q, 2H), 7.10 (t, 2H), 4.79 (d, 2H), 4.63 (s. 2H), 3.77 (m, 2H), 2.23 (dt, 2H), 1.49 (d, 3H), ³¹P NMR (CD₃OD) δ 27.588. MS: 532 (M+1).

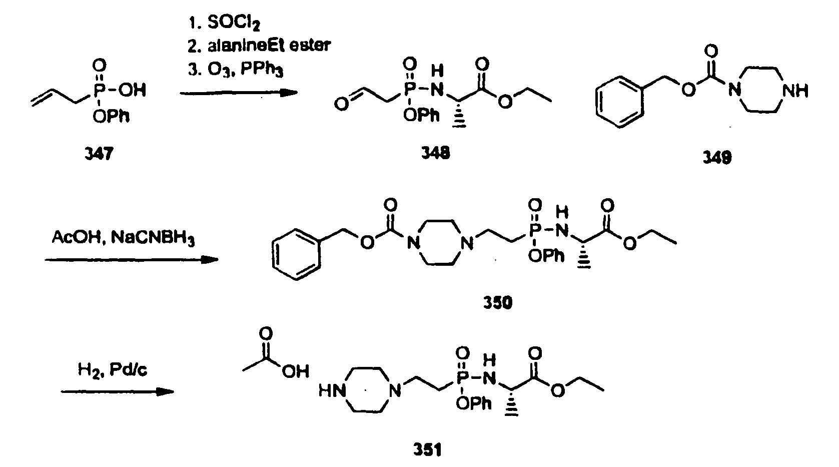

### Example 69

**348:** A solution of **347** (1.17 g, 5.9 mmol) in toluene (7.4 mL) was treated with thionyl chloride (1.29 mL, 17.7 mmol) and N,N-Dimethylformamide (DMF) (0.040 mL).

The reaction mixture was heated to 65° C under nitrogen atmosphere and stirred for 2 hours at which point the reaction was complete as shown by ³¹P NMR (CDCl₃) δ 35.8. The reaction mixture was concentrated as such to afford the intermediate mono-chloridate as an oil which was immediately dissolved in methylene chloride (19.7 mL) and cooled to -20° C. L-Alanine ethyl ester hydrochloride was partitioned between methylene chloride and saturated Na₂CO₃. The organic phase was dried (NaSO₄), filtered then concentrated *in vacuo* to afford the freed base L-Alanine ethyl ester (2.0 g, 17.7 mmol) which was added to the reaction mixture. The mixture was stirred at -20° C under nitrogen atmosphere for 1 hour and then was concentrated *in vacuo*. The residue was purified by chromatography on silica gel (2/1 - ethyl acetate/hexane) to afford the desired allyl phosphonate mono-amidate intermediate (0.92 g, 52%) as an oil. To a solution of the allyl phosphonate mono-amidate (0.92 g, 3.1 mmol) dissolved in methylene chloride 10.2 mL) cooled to -78° C was bubbled ozone. After the reaction was saturated and the solution turned a blue color, oxygen was bubbled to remove excess ozone, triphenyl phosphine (1.21 g, 4.61 mmol) was added and the reaction mixture stirred for 1 hour while warming to room temperature. The mixture was concentrated *in vacuo* without further purification to afford a mixture of the aldehyde product **348** and triphenyl phosphine oxide (2.5g, 100%).

**350:** To a solution of the crude aldehyde **348** (0.91 g, 3.1 mmol) and Benzyl 1-piperazine-1-carboxylate **349** (0.740 g, 3.36 mmol) dissolved in ethanol (30.6 mL) was added 4 angstrom molecular sieves (0.300 g) and acetic acid (0.699 mL, 12.22 mmol). The reaction mixture was stirred at room temperature under nitrogen atmosphere for 1.5 hours then sodium cyanoborohydride (0.387 g, 6.15 mmol) was added. The reaction mixture stirred at room temperature for 1 hour and was concentrated *in vacuo* then redissolved in ethyl acetate. The mixture was washed with saturated NaHCO₃ and brine, dried (NaSO₄), filtered and concentrated. The residue was purified by chromatography on silica gel (2/98 - methanol/ethyl acetate) to afford the desired product **350** (0.759 g, 49%) as an oil.

**351:** To a solution of the phosphonate **350** (0.759 g, 1.5 mmol) dissolved in ethanol (15.0 mL) was added palladium (on carbon). The reaction was purged under a vacuum then submitted to hydrogen gas (via balloon attached to the reaction vessel). After several purges between gas and vacuum the reaction mixture was stirred at room temperature for 2 hours. The mixture was filtered with celite and concentrated *in vacuo* to afford the amine **351** (0.700 g, 100%) as an acetate salt with a 1:1.3 mixture of diastereomers without further purification: ¹H NMR (CDCl₃) δ 7.4-7.1 (m, 10H), 4.2-4.0 (m, 3H), 3.3-3.1 (m, 4H), 2.9-2.6 (m, 6H), 2.3-2.0 (m, 2H), 1.4 & 1.3 (d, 3H), 1.25 (t, 3H); ³¹P NMR (CDCl₃)δ 30.82,30.33; MS: 370 (M+1).

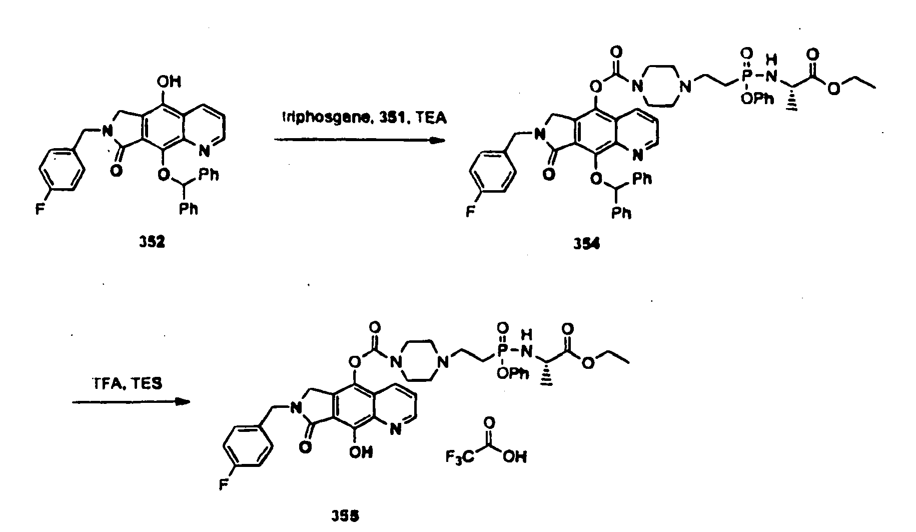

### Example 70

**354:** A solution of the phenol intermediate **352** (0.115 mmol) in methylene chloride (1.2 mL) was treated with triethylamine (0.065 mL, 0.461 mmol) and cat. 4-dimethylaminopyridine. The reaction mixture was cooled to 0° C then triphosgene (0.068 g, 0.23 mmol) in a 1M solution of methylene chloride was added. The mixture stirred at room temperature under nitrogen atmosphere for 1 hour, then amine **351** (0.150 g, 0.346 mmol) in a 1M solution of methylene chloride treated with triethylamine (0.065 mL, 0.461 mmol) was added to the reaction, and the mixture was stirred overnight. The mixture was partitioned between methylene chloride and water. The organic phase was washed with brine (twice), dried (MgSO₄), filtered and concentrated *in vacuo.* The residue was purified by silica gel chromatography (2/98-methanol/ethyl acetate) to afford the product **354** (0.030 g, 30%).

**355:** A solution of the phosphonate **354** (0.020 g, 0.025 mmol) in methylene chloride (0.5 mL) was treated with trifluoracetic acid (0.1 mL) and triethylsilane (0.2 mL). The reaction mixture was stirred at room temperature under an inert atmosphere for 20 minutes. The volatiles were removed *in vacuo* with toluene. The solid was triturated in diethyl ether/hexane then purified by reversed phase HPLC to afford the desired product **355** (0.014 g, 80%) as a TFA salt with a 1:1.6 mixture of diastereomers: ¹H NMR (CDCl₃) δ 9.0 (dd, 1H), 8.15 (dd, 1H), 7.6 (dd, 1H), 7.35 (m, 2H), 7.4-7.1 (m, 5H), 7.1 (t, 2H), 4.75 (s, 2H), 4.35 (s, 2H), 4.3-3.2 (m, 15H), 2.7-2.4 (m, 2H), 1.4-1.2 (m, 6H); ³¹P NMR (CDCl₃) δ 26.01,25.38; MS: 720 (M+1).

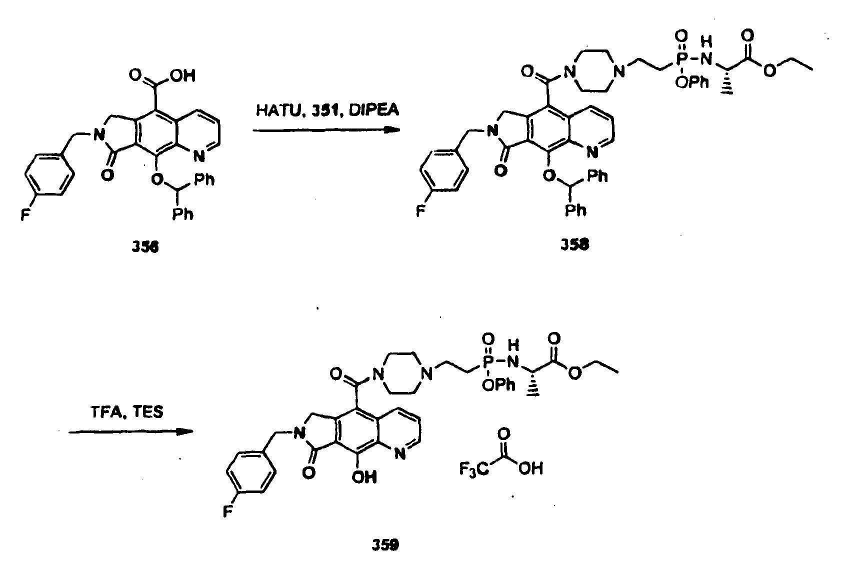

### Example 71

**358:** The acetate salt **351** was partitioned between methylene chloride and saturated Na₂CO₃. The organic phase was dried (NaSO₄), filtered then concentrated *in vacuo* to afford the freed base amine (0.260 g, 0.703 mmol) which was dissolved in DMF (1 mL) and treated with diisopropylethylamine (0.20 mL, 1.12 mmol). This mixture was added to a solution of carboxylic acid **356** (0.145 g, 0.281 mmol) in DMF (1.15 mL) that had been stirred with HATU (0.214 g, 0.562 mmol). The reaction mixture was stirred overnight then diluted with ethyl acetate, washed with saturated NH₄Cl, brine (twice), and aqueous LiCl (twice), dried (NaSO₄) filtered and concentrated. The residue was purified by chromatography on silica gel (5/95 - methanol/ethyl acetate) to afford the desired product **358** (0.160 g, 65%) as a solid.

**359:** This compound is synthesized in a similar fashion as compound **355** without need for purification by reversed phase HPLC to afford the desired product **359** (100%) as a TFA salt with a 1:1.5 mixture of diastereomers: ¹H NMR (CDCl₃) δ 9.0 (dd, I H), 8.15 (dd, I H), 7.65 (dd, 1H), 7.35 (m, 2H), 7.4-7.1 (m, 5H), 7.05 (t, 2H), 4.9 (m, 1H), 4.5 (s, 2H), 4.3-3.2 (m, 12H), 3.0 (m, 2H), 2.6-2.3 (m, 2H), 1.4-1.2 (m, 6H); ³¹P NMR (CDCl₃) δ 26.21, 25.56; MS: 704 (M+1).

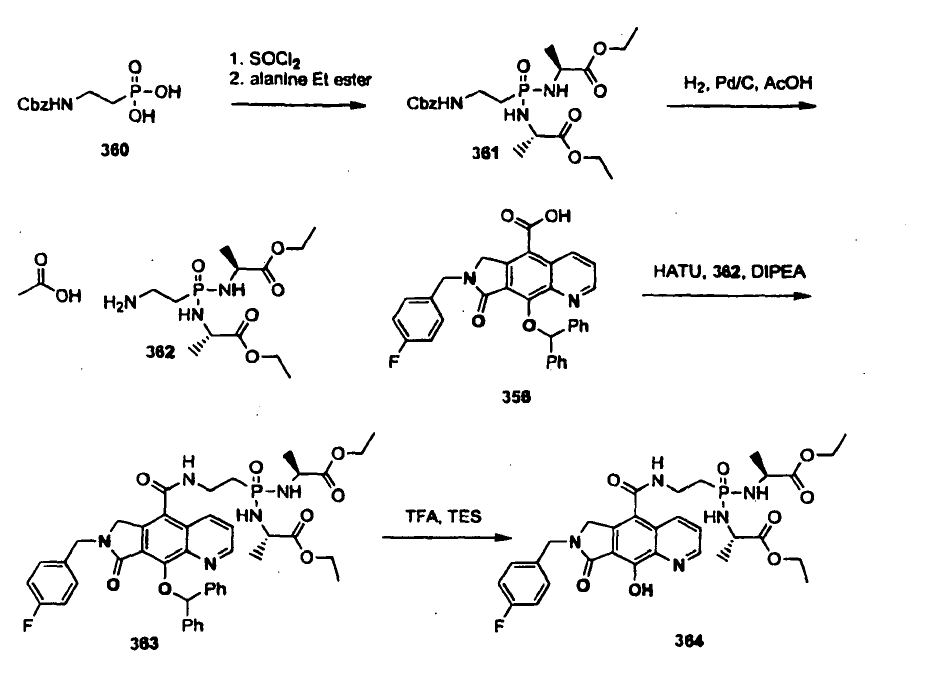

### Example 72

**361:** A solution **of 360** (1.05 g, 4.05 mmol) in toluene (20.3 mL) was treated with thionyl chloride (1.18 mL, 16.2 mmol) and DMF (0.040 mL). The reaction mixture was heated to 65° C under nitrogen atmosphere and stirred for 3 hours at which point the reaction was complete as shown by ³¹P NMR (CDCl₃) δ 46.5. The reaction mixture was concentrated as such to afford the intermediate mono-chloridate as an oil which was immediately dissolved in methylene chloride (13.5 mL) and cooled to -20° C. L-Alanine ethyl ester hydrochloride was partitioned between methylene chloride and saturated Na₂CO₃. The organic phase was dried (NaSO₄), filtered then concentrated *in vacuo* to afford the freed base L-Alanine ethyl ester (2.37 g, 20.3 mmol) which was added to the reaction mixture. The mixture was stirred at -20° C under nitrogen atmosphere for 1 hour and then was concentrated *in vacuo.* The residue was purified by chromatography on silica gel (3/97 - methanol/ethyl acetate) to afford the desired bis-amidate intermediate (1.03 g, 56%) as an oil.

**362:** The compound was synthesized in a similar fashion as amine **351** to afford the desired amine **362** (100%) as an acetate salt without further purification ¹H NMR (CDCl₃) δ 4.2-4.0 (m, 3H), 4.0 (m, 2H), 3.3 (m, 2H), 2.4-1.9 (m, 2H), 1.4 (m, 6H), 1.3 (t, 6H); ³¹P NMR (CDCl₃) δ 27.30; MS: 324 (M+1).

**363:** The compound was made in a similar fashion as compound **358** to afford phosphonate **363.**

**364:** A solution of the phosphonate **363** (0.135 g, 0.164 mmol) in methylene chloride (0.550 mL) was treated with trifluoracetic acid (0.063 mL, 0.82 mmol) and triethylsilane (0.052 mL, 0.328 mmol). The reaction mixture was stirred at room temperature under an inert atmosphere for 20 minutes. The volatiles were removed *in vacuo* with toluene. The solid was triturated in diethyl ether/hexane then purified by reversed phase HPLC in neutral conditions to afford the desired product **364** (0.014 g, 50%): ¹H NMR (CDCl₃) δ 8.8 (dd,1 H), 8.6 (dd, 1H), 7.9 (dd, 1H), 7.5 (m, 1H), 7.3 (m, 2H), 7.0 (t, 2H), 4.6 (s, 2H), 4.5 (s, 2H), 4.2-3.8 (m, 8H), 2.15 (m, 2H), 1.4 (m, 6H), 1.25 (m, 6H); ³¹P NMR (CDCl₃) δ 28.33; MS: 658 (M+1).

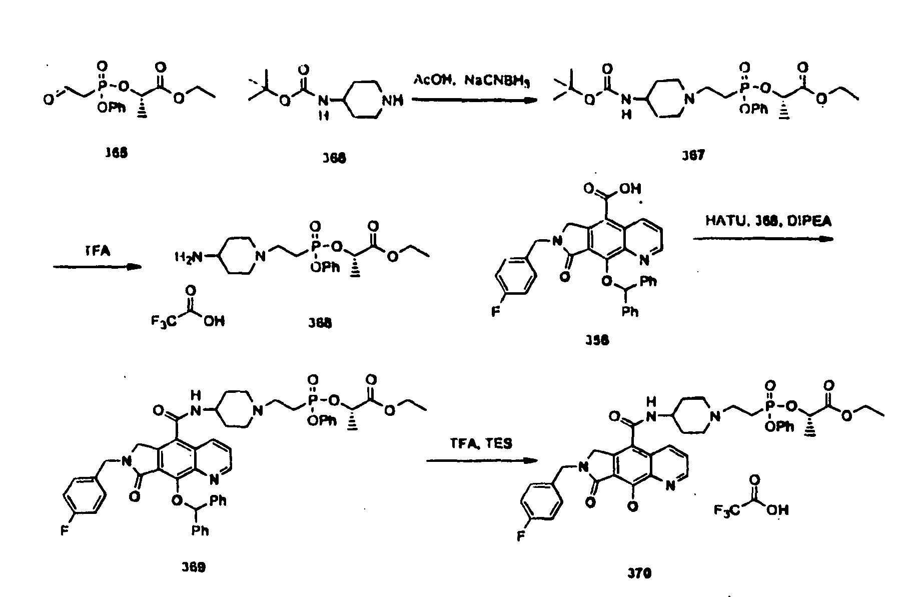

### Example 73

**367:** To a solution of aldehyde **365** as a 1:1 mixture of DMSO (0.250 g, 0.667 mmol) and 4-BOC aminopiperidine **366** (0.147 g, 0.733 mmol) dissolved in ethanol (6.67 mL) was added 4 angstrom molecular sieves (0.300 g) and acetic acid (0.699 mL, 12.22 mmol). The reaction mixture was stirred under nitrogen atmosphere at room temperature for 1.5 hours then sodium cyanoborohydride (0.387 g, 6.15 mmol) was added. The reaction mixture stirred at room temperature overnight then concentrated *in vacuo.* The residue was redissolved in ethyl acetate then washed with saturated NaHCO₃ and brine, dried (NaSO₄), filtered and concentrated. The residue was purified by chromatography on silica gel (2/98 - methanol/ethyl acetate) to afford the desired product **367** (0.173 g, 54%) as an oil.

**368:** A solution of the phosphonate **367** (0.173 g, 0.357 mmol) in methylene chloride (2.4 mL) was treated with trifluoracetic acid (0.275 mL, 3.57 mmol). The reaction mixture was stirred at room temperature under an inert atmosphere overnight. The volatiles were removed *in vacuo* with toluene to afford the free piperazine linker phosphonate **368** (0.190 g, 100%) as a TFA salt.

**369:** A solution of the amine **368** (0.105 g, 0.213 mmol) in DMF (0.5 mL) was treated with diisopropylamine (0.075 mL, 0.426 mmol). This mixture was added to a solution of carboxylic acid **356** (0.0275 g, 0.053 mmol) in DMF (0.53 mL) that had been stirred with HATU (0.040 g, 0.106 mmol). The reaction mixture was stirred overnight then diluted with ethyl acetate, washed with saturated NH₄Cl, brine (twice), and aqueous LiCl (twice), dried (NaSO₄), filtered and concentrated. The residue was purified by chromatography on silica gel (5/95 - methanol/methylene chloride) to afford the desired product **369** (0.044 g) approximately 75% pure.

**370:** This compound is synthesized in a similar fashion as compound **355** to afford the desired product (70%) as a TFA salt with a 1.2:1 mixture of diastereomers: ¹H NMR (CD₃OD) δ 8.9 (dd, 1H), 8.55 (dd, 1H), 7.7 (dd, 1H), 7.35 (m, 2H), 7.5-7.0 (m, 9H), 5.0 (m, 1H), 4.8-4.4 (m, 2H), 4.3-4.1 (m, 2H), 3.9-3.5 (m, 4H), 2.9-2.6 (m, 2H), 2.5-2.2 (m, 2H), 1.9 (m, 1H), 1.5 & 1.4 (m, 3H), 1.3 (t, 3H); ³¹P NMR (CD₃OD) δ 23.9, 25.37; MS: 719 (M+1).

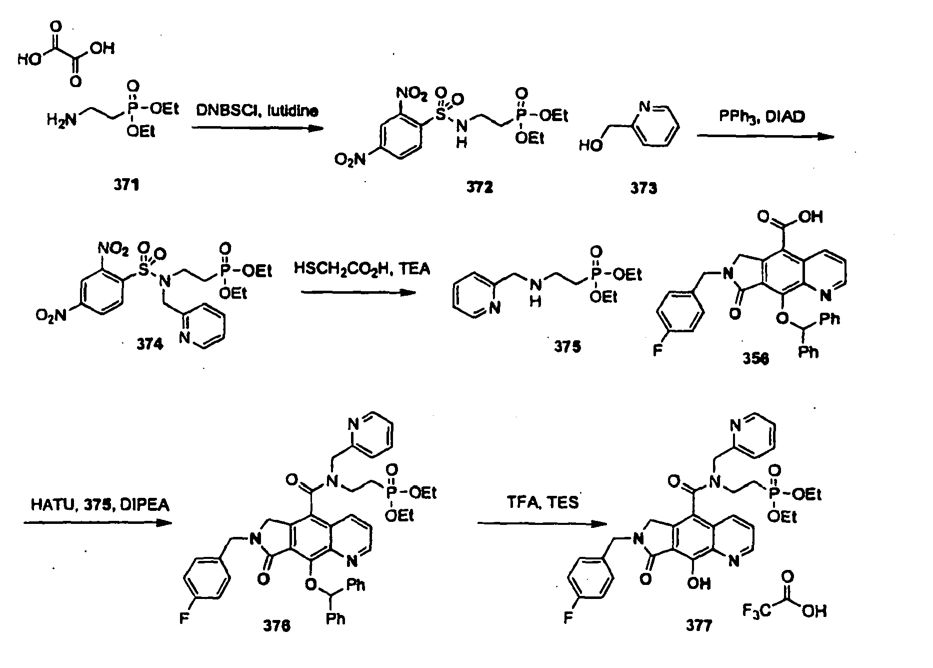

### Example 74

**372:** Commercially available Diethyl(aminoethyl)phosphonate oxalate **371** was partitioned between methylene chloride and saturated Na₂CO₃. The organic phase was dried (MgSO₄) then concentrated *in vacuo* to afford the freed amine (0.115 g, 0.635 mmol) which was dissolved in methylene chloride (1.3 mL). The solution was treated with 2,6-Lutidine (0.148 mL, 1.27 mmol) and 2,4-Dintrobenzene-sulfonyl chloride (0.254 g, 0.953 mmol) and stirred at room temperature for 1 hour. The reaction was quenched with acetic acid (0.3 mL), diluted with ethyl acetate then washed with saturated NH₄Cl and brine (twice), dried (NaSO₄), filtered and concentrated. The residue was purified by chromatography on silica gel (4/1 - ethyl acetate/hexane) to afford the desired product **372** (0.200 g, 77%) as a solid.

**374:** To a solution of the protected amine **372** (0.99 g, 0.24 mmol) dissolved in methylene chloride (0.600 mL) was added triphenyl phosphine (0.190 g, 0.73 mmol) and 2-pyridyl carbinol **373** (0.079 g, 0.73 mmol). The reaction mixture was cooled to 0° C under nitrogen atmosphere then Azodicarboxylic acid diisopropyl ester (DIAD) (0.155 mL, 0.73 mmol) was added. The reaction was stirred at room temperature for 2 hours then diluted with ethyl acetate, washed with saturated NH₄Cl (twice) and brine (twice), dried (NaSO₄), filtered and concentrated. The residue was purified by chromatography on silica gel (100% - ethyl acetate) to afford the desired product **374** (0.096 g, 60%) as a solid.

**375:** To a solution of compound **374** (0.095 g, 0.19 mmol) in methylene chloride (0473 mL) was added triethylamine (0.053 mL, 0.38 mmol) and mercaptoacetic acid (0.026 mL, 0.38 mmol). The reaction mixture was stirred under nitrogen atmosphere for 30 minutes then diluted with ethyl acetate, washed with saturated NaHCO₃ (twice), dried (NaSO₄), filtered and concentrated *in vacuo* without further purification to afford the product **375** (0.057 g, 100%).

**376:** This compound is synthesized in a similar fashion as compound **369** to afford the desired product **376** (69%).

**377:** This compound is synthesized in a similar fashion as compound **364** without need for purification by reversed phase HPLC to afford the desired product **377** (90%): ¹H NMR (CDCl₃) δ 8.95 (dd, 1H), 8.6-8.4 (m, 1H), 8.05 (dd, 1H), 7.6 (m, 1H), 7.4 (m, 1H), 7.3 (m, 2H), 7.05 (m, 3H), 6.75 (d, 1H), 5.1-4.3 (m, 6H), 4.15 (q, 4H), 3.6-3.4 (m, 2H), -2.5-1.6 (m, 2H), 1.35 (m, 6H); ³¹P NMR (CDCl₃) δ 28.68, 26.39; MS: 607 (M+1).

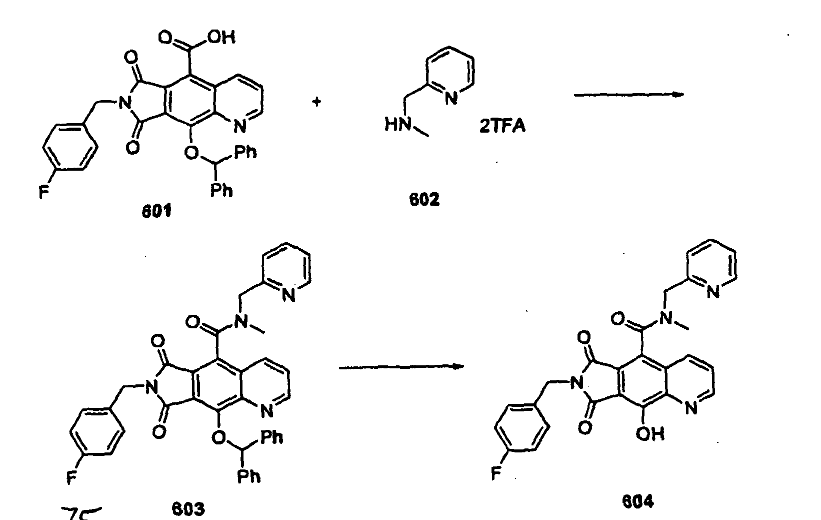

### Example 75

**Step 1:** To a solution of crude 9-benzhydrylox-7-(4-fluoro-benzyl)-6,8-dioxo-7,8-dihydro-6H-pyrrolo[3,4-g]quinoline-5-carboxylic acid **601** (839 mg, 0.188 mmol) dissolved in DMF (4 mL) was added N, N-diisopropylethylamine (0.4 mL, 1.5 mmol), *O*-(7-azabenzotriazol-1-yl)-*N,N,N'*,*N'*-tetramethyluronium hexafluorophosphate (HATU) (150 mg, 0.376 mmol). The reaction mixture was stirred at room temperature for 30min. It was then added amine **602** (0.2g, 0.564 mmol) and stirred overnight under nitrogen. The reaction was quenched by addition of 1N HCl (10 mL) and extracted with EtOAc (2X30mL). The organic phases were combined, washed with brine, dried over MgSO₄ and concentrated *in vacuo.* The crude product was purified by HPLC to give the mixture of **603** and **604,** 55mg.

**Step 2:** This mixture was dissolved in dichloromethane (2 mL), and trifluoroacidic acid (200 µl) and triethylsilane (400 µl) were added. The reaction mixture was stirred at room temperature for 1/2 hours under an inert atmosphere then concentrated *in vacuo.* The residue was purified by HPLC to afford 7-(4-Fluorobenzyl)-9-hydroxy-6,8-dioxo-7,8-dihydro-6H-pyrrolo[3,4-g]quinoline-5-carboxylic acid methyl-pyridin-2-ylmethyl-amide **604,** TFA salt, (25.6 mg, 0.037 mmol, 20%) as a yellow solid: ¹H NMR (CDCl₃) δ 9.0 (d, 1H), 8.8 (d, 1H), 8.4 (d, 1H), 8.3 (d, 1H), 3.2 (t, 1H), 7.7 (m, 2H), 7.6 (m, 1H), 7.4 (dd, 2H), 7.0 (dd, 2H), 5.6 (d, 1H), 5.1 (d, 1H), 4.8 (s, s, 2H), 2.9 (s, 3H); MS: 471 (M+1). HPLC conditions: mobile phase A was 0.1% TFA in water, mobile phase b was 0.1 % TFA in CH₃CN; gradient from 5% to 60% B in 20 min; flow rate was 20 mL/min; column was Phenomenex, luna 5µ, C18(2), 150mm x 21.1mm.

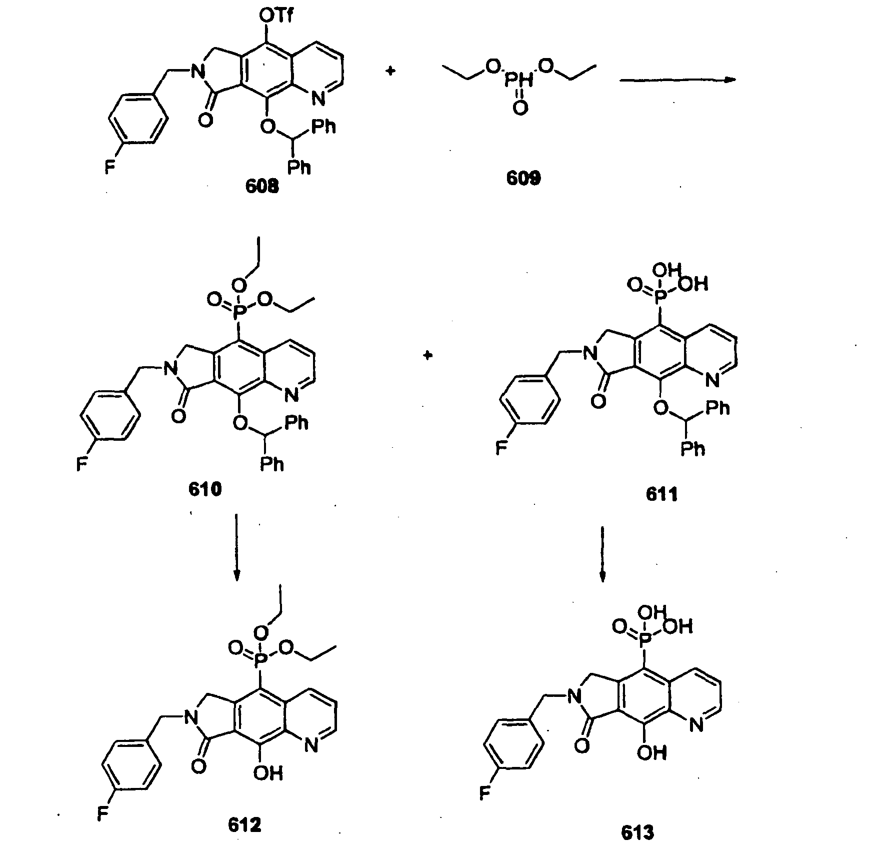

### Example 76

**Step 1:** To a solution of triflate **608** (50 mg, 0.08 mmol) dissolved in anhydrous CH₃CN (mL) was added N-methylmorpholine (11 µl, 0.104 mmol), diethylphosphite **609** (14 µl, 0.104). The mixture was flashed with argon three times. Tetrakis-(triphenylphosphine)-palladium (0) (3 mg, 0.024mmol) was then added. The reaction mixture was heated to 75°C under argon for 24 hours. No product was found by LC/MS. After cooling to room temperature, TEA (0.5mL) and diethylphosphine (200ul) were added. The mixture was degassed again and heated to 75°C under argon for 24 hours. Cooling to room temperature, it was diluted with EtOAc (20mL) and washed with 1N HCl, sat'd NaHCO₃ and brine. The organic phase was dried (MgSO₄). The crude product was purified by HPLC to afford intermediates **610** (2mg) and **611** (4mg).

**Step 2:** The experimental was carried out the same as in Example 388. After HPLC purification, it generated product 612, TFA salt, (0.4 mg), and product **613,** TFA salt, (0.8 mg) separately.

¹H NMR (CDCl₃) for **612**, δ 9.8 (d, 1H), 8.9 (d, 1H), 7.7 (m, 1H), 7.4 (m, 2H), 7.1 (t, 2H), 4.6 (s, 2H), 4.5 (s, 2H), 4.2 (m, 4H), 1.3 (m, 6H); MS: 445 (M+1).

¹H NMR (CDCl₃) for **613**, δ 9.0 (d, 1H), 8.6 (d, 1H), 7.6 (m, 1H), 7.3 (m, 2H), 7.1 (t, 2H), 4.8 (s, 2H), 4.6 (s, 2H), 3.8 (s, 2H); MS: 411 (M+23).

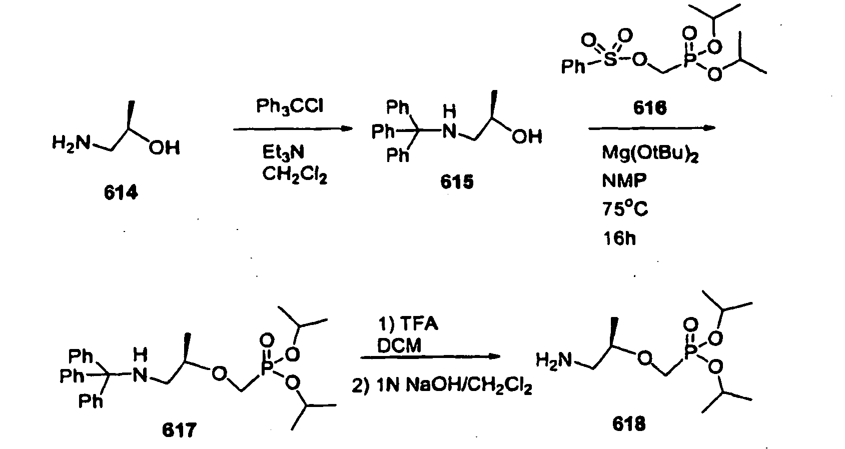

### Example 77

**Step1:** To a solution of amino alcohol **614** (4 g, 0.053 mol) dissolved in dichloromethane (60 mL) was added TEA (15mL) followed by trytl-Cl (14.84 g, 0.053 mol). The reaction mixture warmed up due to the reaction heat generating. The reaction was done in 2h at room temperature. Filtered off the precipitation through a pile of Celite, the filtrate was concentrated and the residue was purified by flash chromatography on silica gel with EtOAc/Hexane (1/9 to 3/7). It yielded 16.8g of compound **615**, 99.4%.

**Step 2:** To a solution of **615** (16.8g, 5.29 mmol) dissolved in NMP (80 mL) was added Mg(OtBu)₂ (18.1g, 10.6 mmol), followed by **616** (21.4 g, 6.36 mmol). The mixture was heated to 75°C for 16h. After cooling to room temperature, water (about 150 mL) was added. The precipitate was collected by filtration (very slow). The sticky solid was dissolved in MeOH/ CH₂Cl₂ (1/1) then concentrated. The crude mixture was purified by flash chromatography on silica gel with EtOAc/Hexane (1/9 to 1/1). It yielded 24 g of compound **617**, 91%.

**Step 3:** Compound **617** (7.7g, 15.5 mmol) was dissolved in 300 mL of 10% TFA/CH₂Cl₂ at room temperature. The reaction was done in 30 min. It was concentrated *in vacuo* and co-evaporated with CH₂Cl₂ two times which gave the TFA salt of **618**. This salt was dissolved in 100 mL of CH₂Cl₂, then added 62 mL of 1N NaOH. The reaction mixture was stirred at room temperature for 1/2 hours. The layer was separated. The organic layer was concentrated to give the free amine **618**.

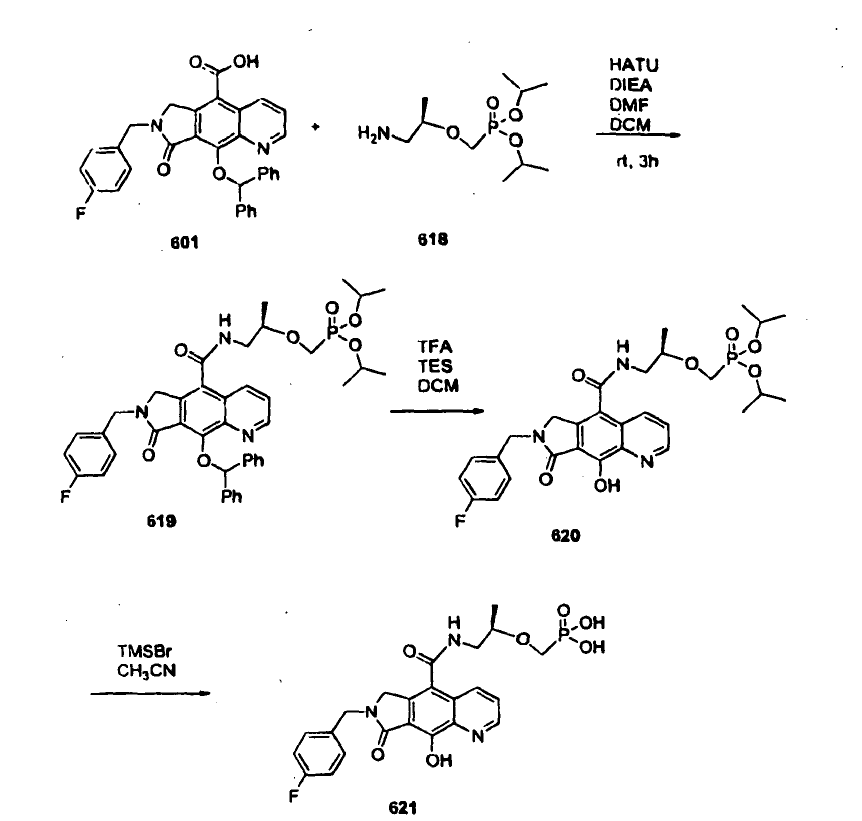

### Example 78

Compound **620** was made from coupling of acid **601** with amine 618 in the same manner as in Example 388. ¹H NMR (CD₃OD) δ 9.0 (d, 1H), 8.8 (d, 1H), 8.0 (s, 1H), 7.8 (m, 1H), 7.3 (m, 2H), 7.0 (t, 2H), 4.8 (dd, 2H), 4.7 (m, 2H), 4.6(dd, 2H), 3.9 (m, 2H), 3.8 (m, 1H), 3.8 (m, 2H), 1.3-1.2 (m, 15H); ¹³P NMR: 20.7ppm, s; MS: 588 (M+1).

### Example 79

Compound **620** (35mg, 0.05 mmol) was dissolved in CH₃CN (1 mL) and cooled to 0°C. TMSBr (1 mL) was added slowly. The reaction was warmed to room temperature, and finished in 18h. It was concentrated to give a crude residue, which was purified by HPLC (condition as in Example 388). Yield: 29mg of compound **621**, 94%. ¹H NMR (CD₃OD) δ 9.0 (d, 1H), 8.8 (d, 1H), 8.0 (s, 1H), 7.8 (m, 1H), 7.4 (m, 2H), 7.1 (t, 2H), 4.8 (s. 2H), 4.6 (s, 2H), 3.9-3.6 (m. 4H), 3.4 (m, 1H), 1.2 (s,s, 3H); ¹³P NMR: 19.9ppm, s; MS: 504 (M+1).

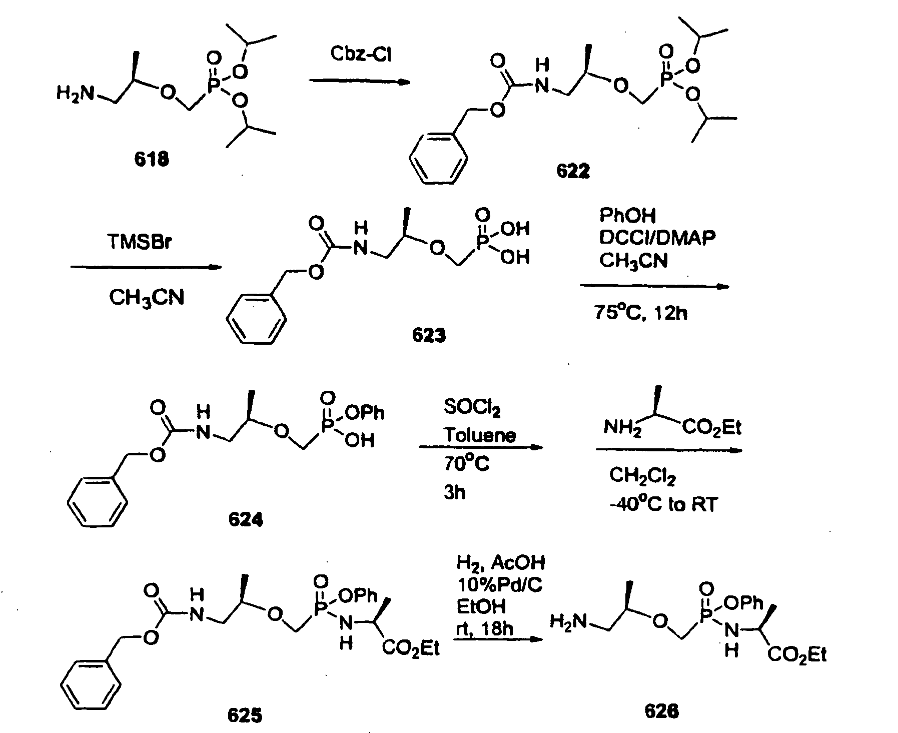

### Example 80

**Step 1:** To a solution of free amine **618** in the mixture of CH₂Cl₂ and 1N NaOH (from Example 391) was added Cbz-Cl (4.0g, 23.25 mmol, 1.5eq). The reaction was done in 18h at room temperature. The layers were separated. The aqueous layer was extracted with CH₂Cl₂ twice. The organic layers were combined and dried (Na₂SO₄) and concentrated. The crude mixture was purified by flash chromatography on silica gel with EAOAc/Hexane (3/7) to afford pure **622**, 2.6 g.

**Step 2:** To a solution of **622** (2.6g, 6.7 mmol) dissolved in CH₃CN (30 mL) was added TMSBr (7 mL, 53.7 mmol) slowly at 0°C. The reaction mixture was stirred at 0°C to room temperature over 3 hours under an inert atmosphere then concentrated *in vacuo.* The residue was dissolved in dichloromethane (100 mL) and 1N NaOH (150 mL). After stirring for 10 min, the layers were separated. The aqueous layer was added 1N HCl (175 mL), to pH = 1. It was extracted with EtOAc three times. The organic layers were combined and dried (MgSO₄) and concentrated to give clean phosphonic acid **623**, 2.1g.

**Step 3:** To a solution of phosphonic acid **623** (1.8 g, 5.94 mmol) dissolved in CH₃CN (50 mL) was added PhOH (1.0 g, 10.7 mmol), DMAP (367 mg, 3 mmol) and DCC (1.5 g, 7.1 mmol). The reaction mixture was heated to 110°C for 12 hours under an inert atmosphere then concentrated *in vacuo.* The residue was dissolved in EtOAc (50 mL)/1N NaOH (20 mL), and stirred for 10 min. The layers were separated. The aqueous layer which had sodium salt of **624** was acidified with 6N HCl slowly to pH = 1. It was extracted with EtOAc three times. The organic layers were combined and dried (MgSO₄) and concentrated. The crude mixture was purified by flash chromatography on silica gel with MeOH/ CH₂Cl₂ (10%, with 0.2% AcOH) to afford pure phosphonic acid monophenyl ester **624**, 1.0g, 44%.

**Step 4:** To a solution of phosphonic acid monophenyl ester **624** (1.0 g, 2.6 mmol) dissolved in toluene (20 mL) was added thionyl chloride (20 mL), DMF (4 drops). The reaction mixture was heated to 70°C for 3 hours under an inert atmosphere then concentrated *in vacuo.* The residue was azeotropied with toluene twice to give the mono-chlorodate. This was redissolved in dichloromethane (20 mL) and cold to -40°C. The free base of alanine-ethylester dropwise. The mixture was kept at low temperature for 2h, then room temperature overnight. After concentrated, it was purified by flash chromatography on silica gel with EtOAc/Hexane (1/1, with 0.2% TEA) to afford pure **625**, 654mg, 52%.

**Step 5:** To a solution of **625** (654 mg, 1.37 mmol) dissolved in EtOH (10 mL) was added AcOH (155µl, 2.74 mmol) and 10% Pd/C (650 mg). The reaction mixture was stirred under an H₂ atmosphere for 18h at room temperature. The solid was filtered off. The filtrate was concentrated *in vacuo.* The residue was dissolved in CH₂Cl₂ (50 mL) / sat'd Na₂CO₃ (50 mL), and stirred for 10 min. The layers were separated. The aqueous layer extracted with CH₂Cl₂ once more. The organic layers were combined and dried (Na₂SO₄) and concentrated. It afforded clean amine **626**, 362 mg, 77%.

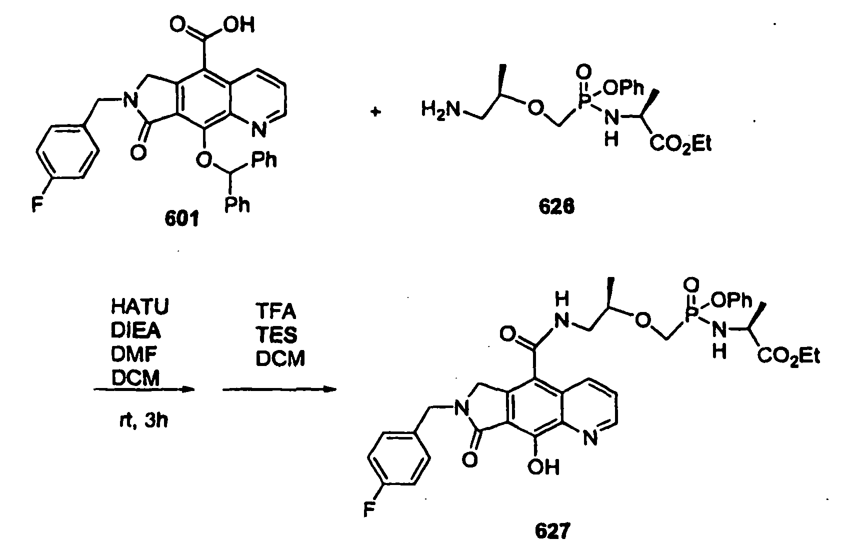

### Example 81

Compound **627** was synthesized by a method similar to Example **96**. HPLC conditions: mobile phase A was water, mobile phase b was CH₃CN; gradient from 5% to 60% B in 20 min; flow rate was 20 mL/min; column was Phenomenex, luna 5µ, C18 (2), 150mm x 21.1mm. ¹H NMR (CD₃OD) δ 8.8 (d, 1H), 8.6 (d, 1H), 7.6 (m, 1H), 7.4 (m, 2H), 7.3-7.0 (m, 7H), 5.6 (d, 1H), 4.8 (d, 2H), 4.6 (d, 2H), 4.1-3.4 (m, 8H), 1.3-1.1 (m, 9H); MS: 679 (M+1).

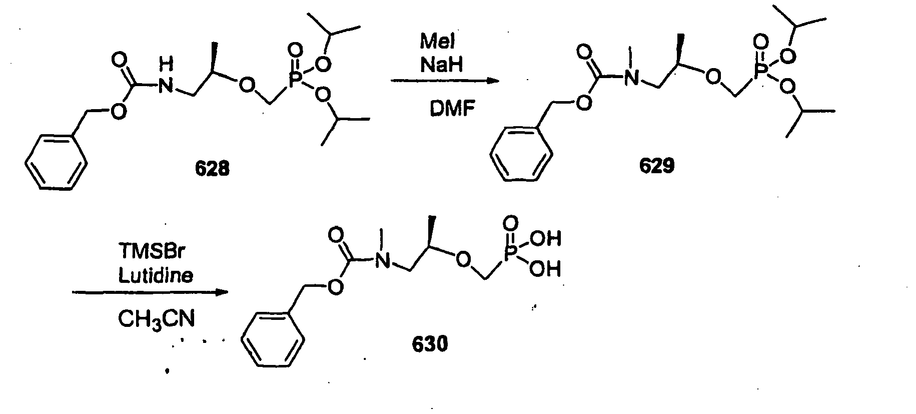

### Example 82

**Step 1:** To a solution of **628** (290 mg, 0.75 mmol) dissolved in DMF (1 mL) was added NaH (60%) (66 mg, 1.64 mmol) at 0°C. The reaction mixture was stirred for 20 min. Mel (102 µl, 1.64 mmol) was then added and kept at 0°C under argon 1.5 hours. The mixture was diluted with EtOAc (20mL) and washed with cold 1N HCl and brine. The organic phase was dried (MgSO₄), and concentrated *in vacuo.* It gave a clean compound **629**, 330 mg, >100%.

**Step 2:** To a solution of **629** (0.75 mmol) dissolved in CH₃CN (30 mL) was added 2,6-lutidine (366µl, 3.15 mmol) and cold to 0°C. TMSBr (396µl, 3.0 mmol) was added slowly. The reaction mixture was stirred at 0°C 2h then room temperature 20 hours under an inert atmosphere. After completion of the reaction, it was cold to 0°C again, then added 1N NaOH (10 mL) slowly. After stirring for 5 min, it was extracted with EtOAc. The aqueous layer was acidified with 1N HCl to pH = 1. It was extracted with EtOAc/MeOH (9/1) three times. The organic layers were combined and dried (MgSO₄) and concentrated to give clean phosphonic acid **630**, 214 mg, 89%

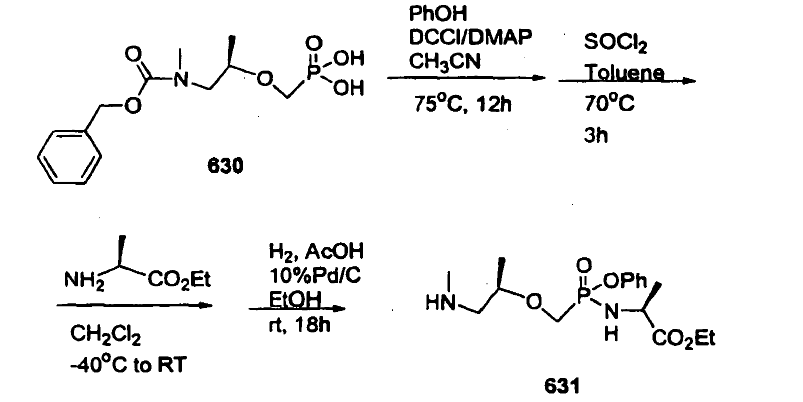

### Example 83

Conversion of compound **630** to **631** was done by the method as described in Example 394 (step 3, step 4, and step 5).

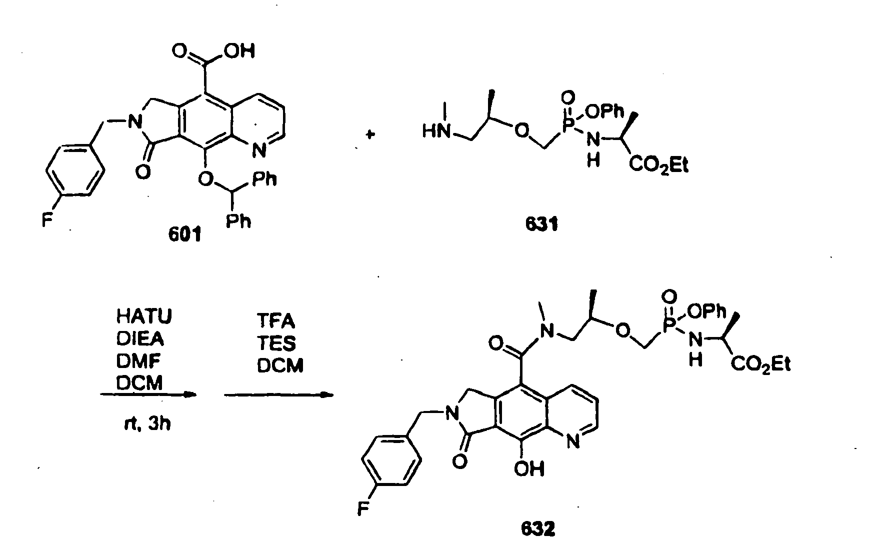

### Example 84

Compound **632** was synthesized by the method similar to Example 75. HPLC conditions: mobile phase A was water, mobile phase b was CH₃CN; gradient from 5% to 60% B in 20 min; flow rate was 20 mL/min; column was Phenomenex, luna 5µ, C18 (2), 150mm x 21.1mm. ¹H NMR (CD₃OD) δ 8.8 (d, 1H), 8.6 (d, 1H), 7.6 (m, 1H), 7.4 (m, 2H), 7.3-7.0 (m, 7H), 5.6 (d, 1H), 4.8 (d, 2H), 4.6 (d, 2H), 4.1-3.4 (m, 8H), 2.8 (3H), 1.3-1.1 (m, 9H); MS: 693 (M+1).

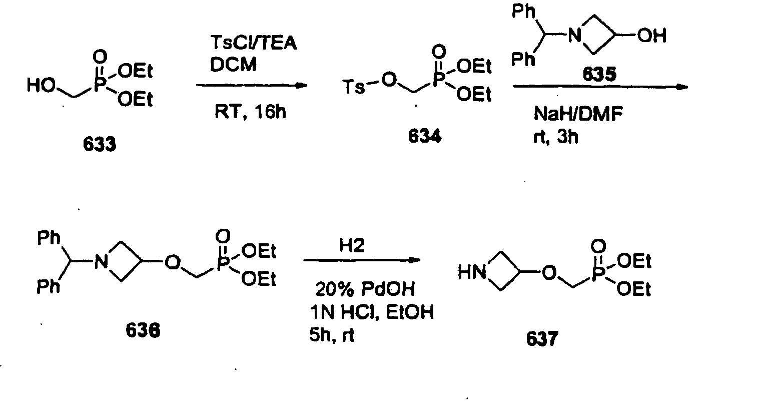

### Example 85

**Step 1:** To a solution of hydroxymethyl-phosphonic acid diethyl ester **633** (5 g, 29.7 mmol) dissolved in dichloromethane (20 mL) was added *p*-toluenesulfonyl chloride (5.66g, 29.7 mmol), followed by slow addition of TEA (5.8 mL, 41.58 mmol) under nitrogen. The reaction mixture was stirred at room temperature for 16 hours. It was quenched with addition of water. The layers were separated. The organic layer was washed with 1N HCL, sat'd NaHCO₃ and dried (MgSO₄) and concentrated. The crude mixture was purified by flash chromatography on silica gel with Acetone/CH₂Cl₂ (5%) to afford pure toluene-4-sulfonic acid diethoxy-phosphorylmethyl ester **634**, 7.5g, 78%.

**Step 2:** To a solution of 1-benzhydryl-azetidin-3-ol **635** (1 g, 4.18 mmol) dissolved in anhydrous DMF (20 mL) was added sodium hydrate (552 mg, 13.8 mmol) and stirred for 30 min at room temperature under nitrogen. Tosylate **634** (2.02g, 6.27 mmol) was then introduced by syringe. The reaction was done in 3 hours. It was quenched with cold 0.5 N HCl, extracted with EtOAc to give organic phase 1. The aqueous was treated with solid NaHCO₃ to pH 7-8, and extracted with EtOAc to give organic phase 2. The organic phases were combined and washed with brine, dried (MgSO₄) and concentrated. The crude mixture was purified by flash chromatography on silica gel with MeOH/ CH₂Cl₂ (5%) to afford pure **636**, 1.4g, 85%.

**Step 5:** To a solution of **636** (941 mg, 2.4 mmol) dissolved in EtOH (20 mL) and 1N HCl (1 mL) was added 20% PdOH/C (1 g). The reaction mixture was stirred under an H₂ atmosphere for 6h at room temperature. The solid was filtered off. The filtrate was concentrated *in vacuo* and lyophilized to afforded clean amine (HCl salt), 672 mg mg, 100%.

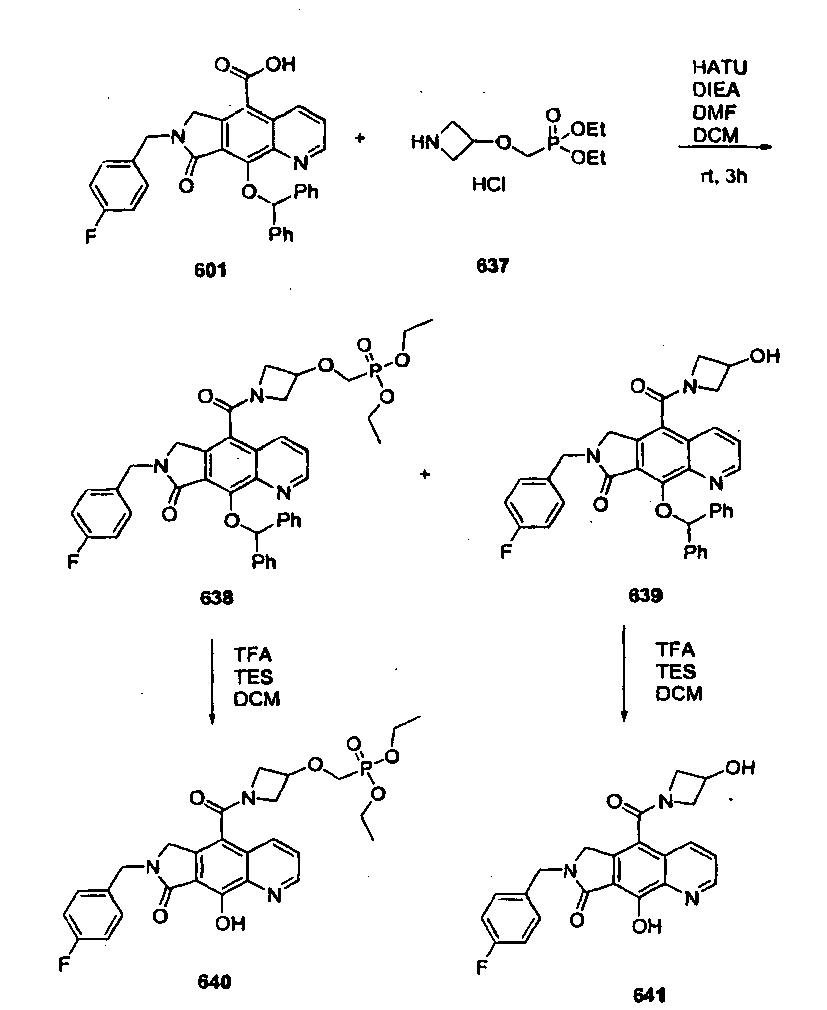

### Example 86

Compound **640** (13mg) was synthesized by a method similar to Example 75.

Compound **641** (4mg) was the by-product from this reaction.

The ratio of **638** to **639** was 24 mg to 12 mg from 42 mg of **601**.

¹H NMR of compound **640** (CD₃OD) δ 9.0 (d, 1H), 8.5 (d, 1H), 7.8 (m, 1H), 7.4 (m, 2H). 7.1 (t, 2H), 4.8 (d, 2H), 4.5 (m, 2H), 4.4 (d, 2H), 4.1 (m, 4H), 4.0-3.6 (m, 4H), 3.3 (d, 2H), 1.3 (t, 6H); ³¹P NMR: 21.4 ppm; MS: 558 (M+1).

¹H NMR of compound **641** (CD₃OD) δ 9.0 (d, 1H), 8.5 (d, 1H), 7.8 (m, 1H), 7.4 (m, 2H), 7.1 (t, 2H), 4.8 (d, 2H), 4.7-4.4 (m, 4H), 4.0 (m, 2H), 3.6 (m, 1H); MS: 408 (M+ 1).

### Example 87

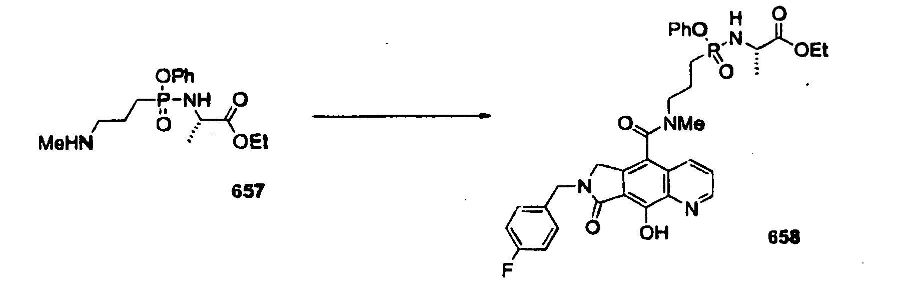

Amine **657** was obtained from the procedures similar to those described herein. Following its formation, it was then coupled to a carboxylic acid scaffold before the diphenylmethyl ether was removed to obtain **658,** which was isolated as a mixture of diastereomers and rotamers. ¹H NMR (300 MHz) CD₃OD ∂: 8.95 (s, 1H), 8.11 (m, 1H), 7.62 (s, 1H), 7.27 (m, 7H), 7.06 (m, 2H), 7.94 (bs, 1H), 2.10 (m, 4 H), etc. ¹⁹F NMR (300 MHz) CD₃OD ∂: -77.16, -114.71. ³¹P NMR (300 MHz) CD₃OD ∂: 32.24, 32,13, 32.03, 31.94, 30.96, 30.82. MS: 663.44 (M+H).

### Example 88

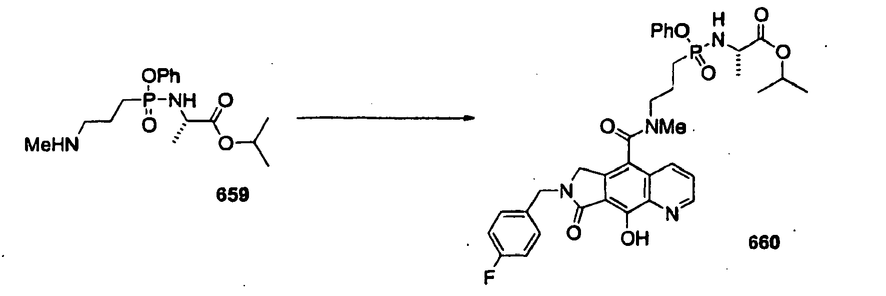

Amine **659** was obtained from the procedures similar to those described herein. Following its formation, it was then coupled to a carboxylic acid scaffold before the diphenylmethyl ether was removed to obtain 660, which was isolated as a mixture of diastereomers and rotamers. ¹H NMR (300 MHz) CD₃OD ∂: 8.97 (s, 1H), 8.10 (m, 1H), 7.57 (s, 1H), 7.27 (m, 7H), 7.06 (m, 3H), 3.21 (s, 3H, rotamer N-Me), 2.78 (s, 3H, rotamer N-Me). ¹⁹F NMR (300 MHz) CD₃OD ∂: -76.42, -114.62. ³¹P NMR (300 MHz) CD₃OD ∂:31.35, 31.23, 31.02, 30.93, 30.03, 29.92. MS: 677.25 (M+H).

### Example 89

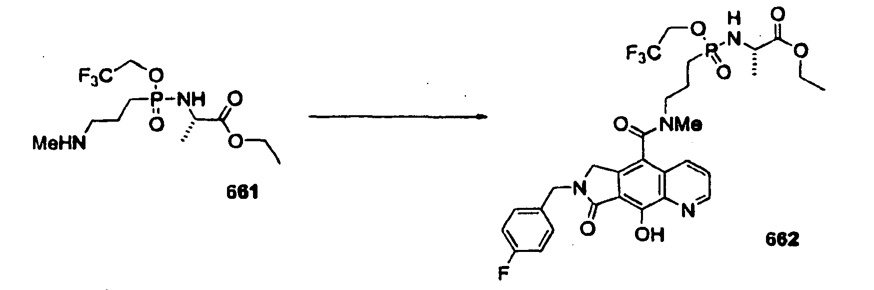

Amine **661** was obtained from the procedures similar to those described herein. Following its formation, it was then coupled to a carboxylic acid scaffold before the phenol was exposed to obtain **662,** which was isolated as a mixture of diastereomers and rotamers.
¹H NMR (300 MHz) CDCl₃ ∂: 8.97 (s, 1H), 8.10 (m, 1H), 7.60 (s, 1H), 7.27 (m, 2 H), 7.06 (m, 2H), 3.19 (s, 3H, rotamer N-Me), 2.78 (s, 3H, rotamer N-Me). ¹⁹F NMR (300 MHz) CDCl₃ ∂: -77.43, -114.66
³¹P NMR (300 MHz) CDCl₃ ∂:35.91, 35.85, 35.11, 34.73, 34.18
MS: 669.27 (M+H).

### Example 90

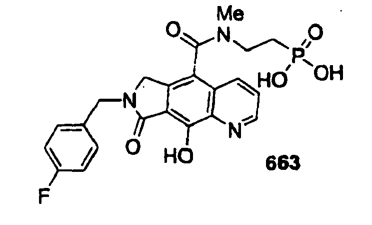

¹H NMR (300 MHz) CD₃OD ∂: 8.99 (d, 1H), 8.46 (d, 1H), 7.82 (d, 1H), 7.41 (s, 2H), 7.05 (m, 2H), 4.67 (d, 1H), 4.88 (d, 1H), 3.88 (m, 1H), 3.20 (s, N-Me rotamer, 3H), 2.86 (s, N-Me rotamer, 3H), 2.24 (m, 1H), 1.91 (m, 1H). ³¹P NMR (300 MHz) CDCl₃ ∂: 25.62, 23.88
MS: 474.27 (M+H).

### Example 91

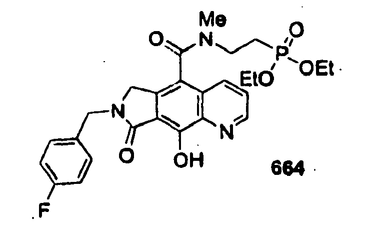

¹H NMR (300 MHz) CD₃OD ∂: 9.02 (d, 1H), 8.23 (d, 1H), 7.62 (d, 1H), 7.32 (s, 2H), 7.08 (m, 2H), 4.88 (d, 2H), 4.70 (d, 2H), 4.56 (d, 2H), 4.19 (m, 4H), 3.92 (m, 2H), 3.19 (s, N-Me rotamer, 3H), 2.83 (s, N-Me rotamer, 3H), 2.32 (m, 2H), 1.91 (m, 1H), 1.38 (m, 6H).
¹⁹F NMR (300 MHz) CDCl₃ ∂: -76.44, -114.56
³¹P NMR (300 MHz) CDCl₃ ∂:28.372, 26.24
MS: 530.28 (M+H).

### Example 92

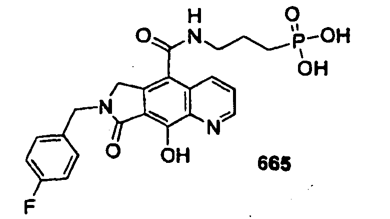

¹H NMR (300 MHz) CD₃OD ∂: 9.01 (d, 1H), 8.83 (d, 1H), 7.89 (d, 1H), 7.43 (s, 2H), 7.08 (m, 2H), 4.80 (d, 2H), 4.61 (d, 2H), 3.55 (t, 2H), 2.88 (s, 1H), 1.85 (m, 4H). ³¹P NMR (300 MHz) CD₃OD ∂: 29.05. MS: 474.27 (M+H).

### Example 93

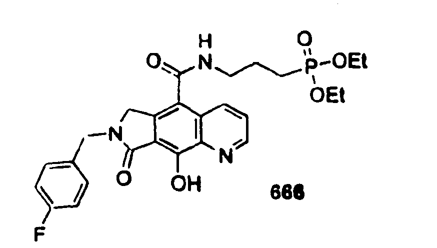

¹H NMR (300 MHz) CDCl₃ ∂: 8.86 (d, 1H), 8.58 (d, 1H), 7.60 (d, 1H), 7.24 (m,2H), 7.01 (m, 2H), 4.47 (s, 2 H), 4.42 (s, 2H), 4.15 (m, 4H), 3.69 (m, 2H), 2.01 (m, 4H), 1.29 (r, 6H). ¹⁹F NMR (300 MHz) CDCl₃ ∂: -76.35, -114.51. MS: 530.31 (M+H).

### Example 94

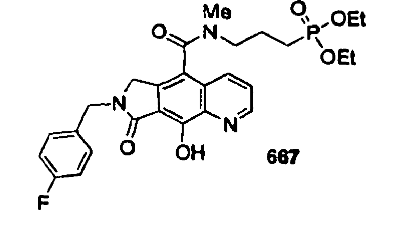

¹H NMR (300 MHz) CDCl₃ ∂: 8.98 (s, 1H), 8.10 (d, 1H), 7.62 (d, 1H), 7.23 (s, 2H), 7.09 (m, 2H), 4.88 (m, 2H), 4.70 (m, 2H), 4.23 (m, 4H), 3.92 (m, 1H), 3.73 (m, 1H) (m, 2H), 3.19 (s, N-Me rotamer, 3H), 2.78 (s, N-Me rotamer, 3H), 2.05 (m, 6H), 1.38 (m, 6H). ¹⁹F NMR (300 MHz) CDCl₃ ∂: -114.60. ³¹P NMR (300 MHz) CDCl₃ ∂: 30.95, 29.93. MS: 544.32 (M+H).

### Example 95

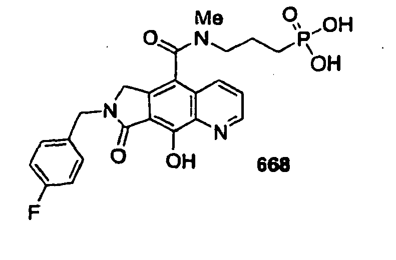

¹H NMR (300 MHz) CD₃OD ∂: 9.00 (d, 1H), 8.38 (m, 1H), 7.82 (m, 1H), 7.42 (m, 2H), 7.02 (m, 2H), 4.85 (m, 2H), 4.69 (m, 2H), 4.44 (m, 2H), 3.89 (m, 2 H), 3.31 (s, N-Me rotamer, 3H), 2.66 (s, N-Me rotamer, 3H), 2.05 (m, 1H), 1.89 (m, 1H). ¹⁹F NMR (300 MHz) CD₃OD ∂: -117.28, -78.13. ³¹P NMR (300 MHz) CD₃OD ∂: 28.79, 28.19. MS: 488.20 (M+H).

### Example 96

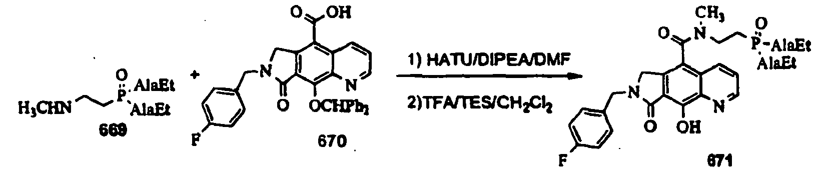

Amine 669 was prepared from procedures similar to those reported herein. Following its formation, it was then coupled to a carboxylic acid scaffold using an HATU promoted amide bond formation reaction. The diphenylmethyl ether protecting group was then removed to obtain product 671, which was isolated as a mixture of phosphorous diastereomers. ¹H NMR (300 MHz) CD₃OD Diagnostic peaks observed at δ: 8.92 (m, 1H), 8.26 (m, 1H), 2.95(s, N-Me rotamers, 3H), 1.75 (d, 3H). ³¹P NMR (121 MHz) CD₃OD δ : 36.2, minor rotamers observed at 28.0, 26.2 ppm. MS : 672.1 (M+H).

### Example 97

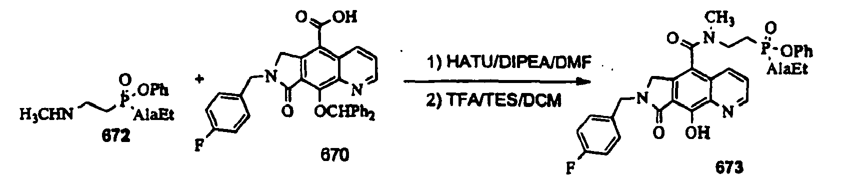

Amine 672 was prepared from procedures similar to those reported herein. Following its formation, this amine was it was then coupled to a carboxylic acid scaffold using an HATU promoted amide formation. The diphenylmethyl ether protecting group was then removed to obtain product 673, which was isolated as a mixture of phosphorous diastereomers. ¹H NMR (300 MHz) CD₃OD Diagnostic peaks at δ : 8.95 (m, 1H), 8.35 (m, 1H), 2.85-3.1 (s, N-Me rotamer, 3H), 1.42 (d, 3H). ³¹P NMR (121 MHz) CD₃OD δ 31.2, 30.0. MS : 649.4 (M+H).

### Example 98

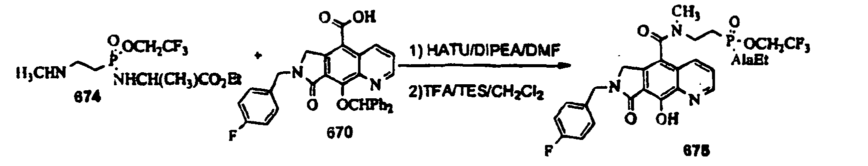

Amine 674 was prepared from procedures similar to those reported herein. Following its formation, this amine was it was then coupled to a carboxylic acid scaffold using an HATU promoted amide formation. The diphenylmethyl ether protecting group was then removed to obtain product 675, which was isolated as a mixture of phosphorous diastereomers. ¹H NMR (300 MHz) CD₃OD Diagnostic peaks at δ : 8.98 (d, 1H), 8.30-8.40 (m, 1H), 7.42 (m, 2H), 7.15 (m, 2H), 2.82 (s, N-Me rotamers). ³¹P NMR (121 MHz) CD₃OD δ 36.2, 35.0, minor rotamer observed at 34.5 . MS : 655.2 (M+H).

### Example 99 General procedure for synthesis of mono-substituted sulfamates using Cs₂CO₃ promoted alkylation

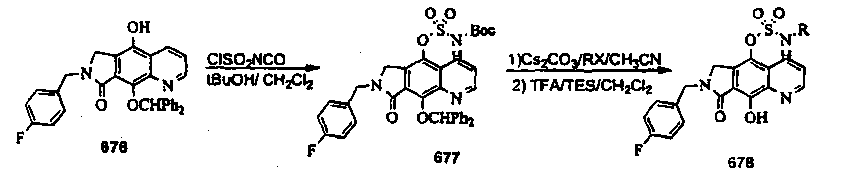

To 300 mg chlorosulfonyl isocyanate, in 10 ml CH₂Cl₂ at 0 degrees C, is added 0.3 ml of *t*-butanol. The reaction is then allowed to warm to room temperature. To a separate flask containing 1.2 g of phenol 676 in methylene chloride is added DIPEA (3 equiv), followed by 0.9 ml of the N-Boc sulfamyl chloride prepared above. The resulting *N*-Boc sulfamate 677 is used in subsequent alkylations without further purification

To sulfamate 677 in acetonitrile is added Cs₂CO₃, followed by alkylating agent. The reaction is then heated to 80 degrees C to effect alkylation. The resulting *N*-Boc *N*-alkyl sulfamate is subjected to TFA/TES treatment to remove both the diphenylmethyl ether and the t-butyl carbamate to furnish the final N-alkyl sulfamate products.

### Example 100

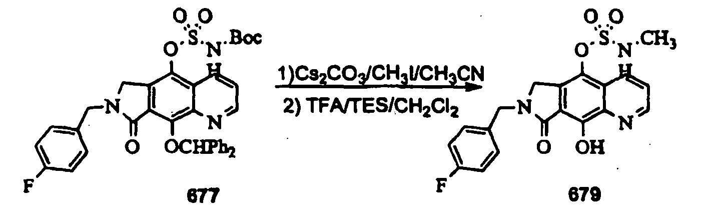

Sulfamate 674 was formed by the use of methyl iodide as alkylating agent in the general procedure described above to give N-methyl sulfamate product.
¹H NMR (300 MHz) CDCl₃ δ : 8.94 (m, 1H). 8.55 (m, 1H), 7.66 (m, 1H), 7.25-7.42 (m, 2H), 7.02-7.16 (m, 2H), 5.54 (bs, 1H), 4.85 (s, 2H), 4.62 (s, 2H), 3.08 (d, 3H). MS:418.0 (M+H).

### Example 101

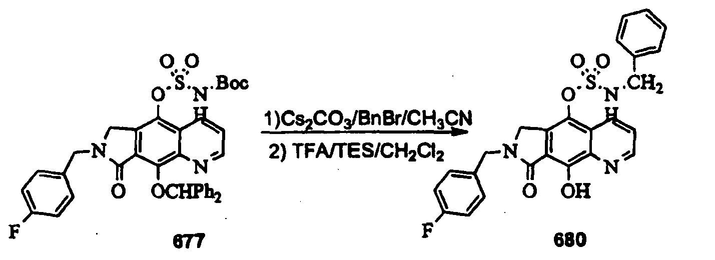

Sulfamate 680 was formed by the use of benzyl bromide as alkylating agent in the general procedure described above. ¹H NMR (300 MHz) CDCl₃ δ : 9.15 (m, 1H), 8.44 (m, 1H), 7.65 (m, 1H), 7.15-7.20 (m, 7H), 7.04 (m, 2H), 4.78 (s, 2H), 4.54 (s, 2H), 4.42 (d, 2H). MS 494.1 (M+H).

### Example 102 General procedure for the synthesis of mono-substituted sulfamates using Mitsunobu reaction

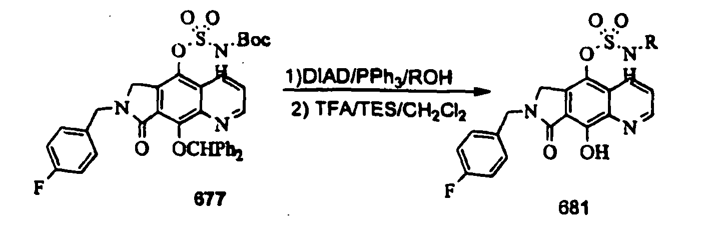

To sulfamate 677 in THF at 0 degrees C is added PPh₃, the desired alcohol, and finally DIAD. The reaction is then allowed to warm to room temperature and the resulting product isolated by direct introduction of the reaction mixture onto a silica gel column after filtration to remove triphenylphosphine oxide. The resulting *N*-Boc *N*-alkyl sulfamate is subjected to TFA/TES treatment to remove both the diphenylmethyl ether and the t-butyl carbamate to furnish the final *N*-alkyl sulfamate products.

### Example 103

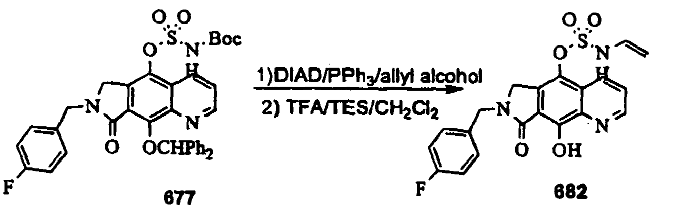

Sulfamate 682 was formed by the use of allyl alcohol as alkylating agent in the general procedure described above. ¹H NMR (300 MHz) CDCl₃ δ : 8.96 (m, 1H), 8.56 (m, 1H), 7.66 (m, 1H), 7.38 (m, 2H), 7.04 (m, 2H), 5.82-6.03 (m, 1H), 5.23-5.42 (m, 2H), 4.78 (s, 2H), 4.64 (s, 2H), 4.02 (bm, 2H). MS 444.1 (M+H).

### Example 104

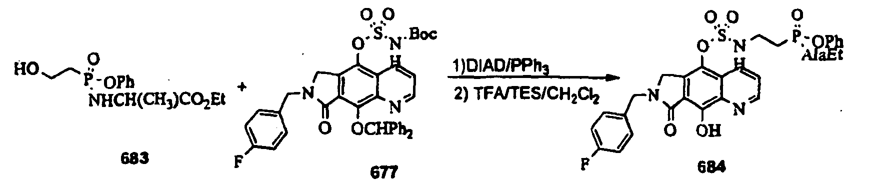

Phosphonate alcohol 683 was prepared by sodium cyanoborohydride reduction of a phosphonate aldehyde. Sulfamate 684 was formed by the use of phosphonate alcohol 683 as alkylating agent in the general Mitsunobu procedure described above to give *N*-methyl sulfamate product as a mixture of phosphorous diasteromers.
¹H NMR (300 MHz) CDCl₃ : Diagnostic peaks at δ 9.12 (m, 1H), 8.62 (m, 1H), 7.6 (m, 1H), 4.8 (s, 2H), 4.62 (d, 2H), 4.10-4.26 (m, 2H), 3.85-3.92 (m, 2H), 1.72 (d, 3H). MS : 686.1 (M+H).

### Example 105

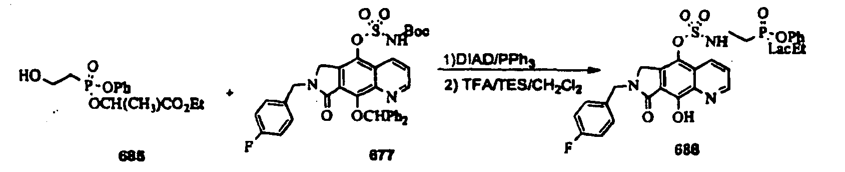

Phosphonate alcohol 685 was was prepared by sodium cyanoborohydride reduction of the corresponding phosphonate aldehyde. Sulfamate 686 was formed by the use of phosphonate alcohol 685 as alkylating agent in the general Mitsunobu procedure described above. ¹H NMR (300 MHz) CD₃OD :Diagnostic peaks observed at δ 9.25 (m, 1H), 8.64 (m, 1H), 3.62-3.75 (m, 2H), 1.82 (d, 3H), 1.20-1.43 (t, 3H).
MS: 688.0 (M+H).

## Claims

1. An HIV integrase inhibitor compound selected from:

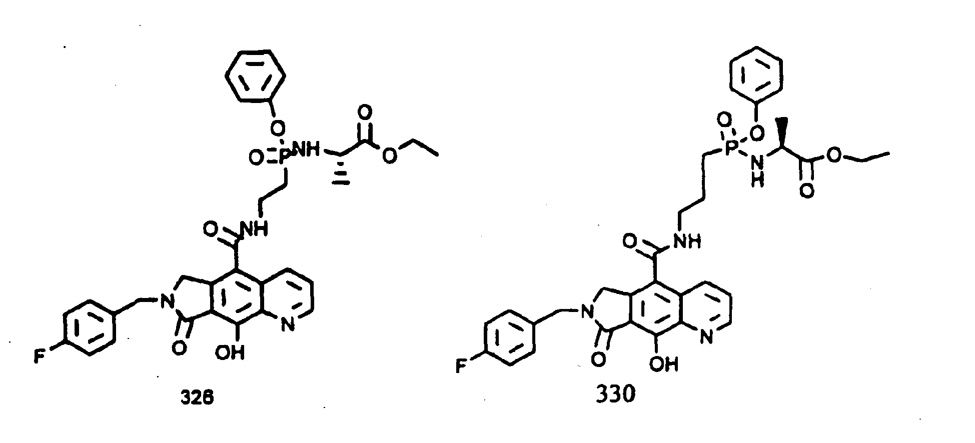

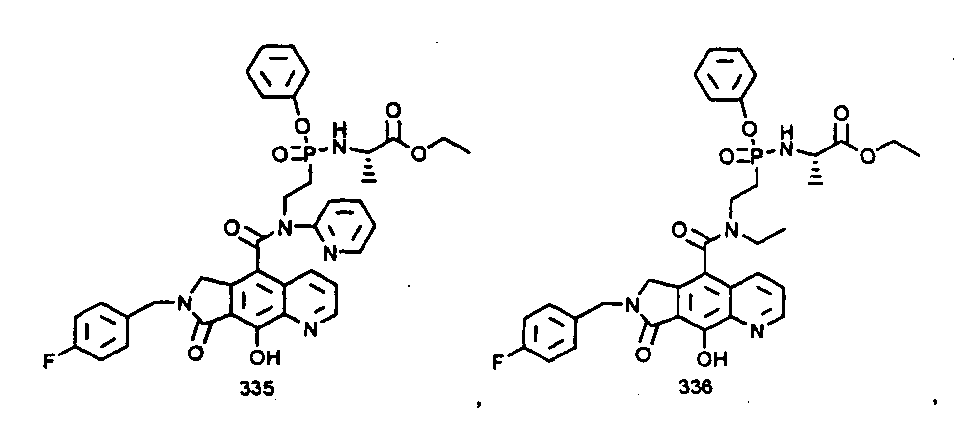

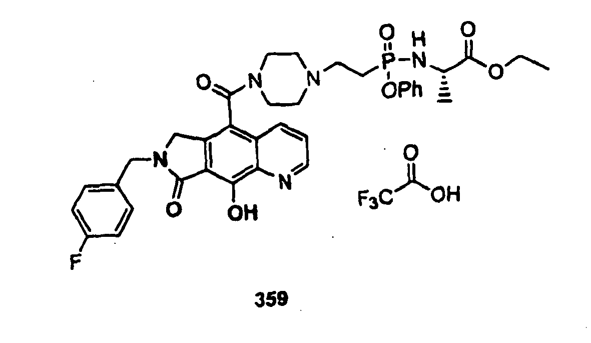

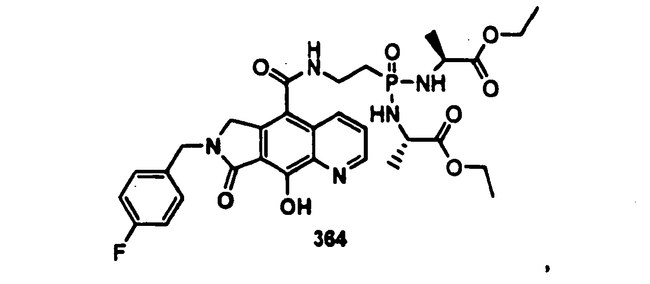

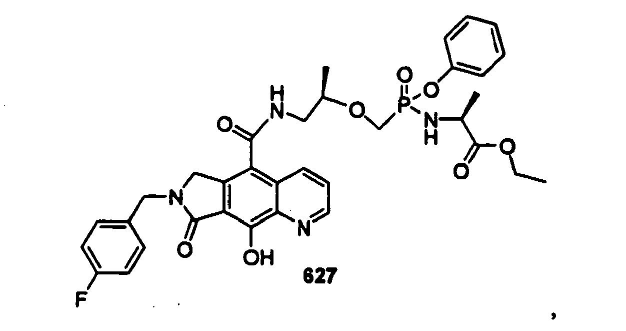

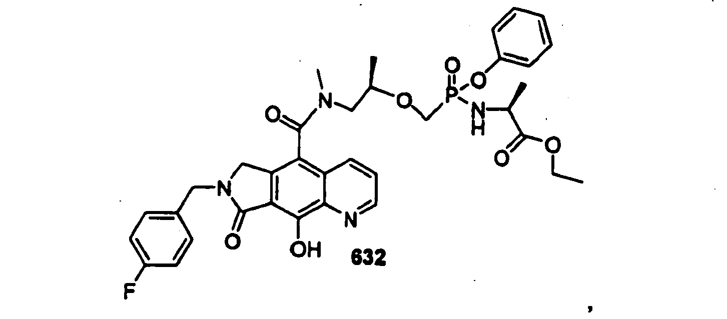

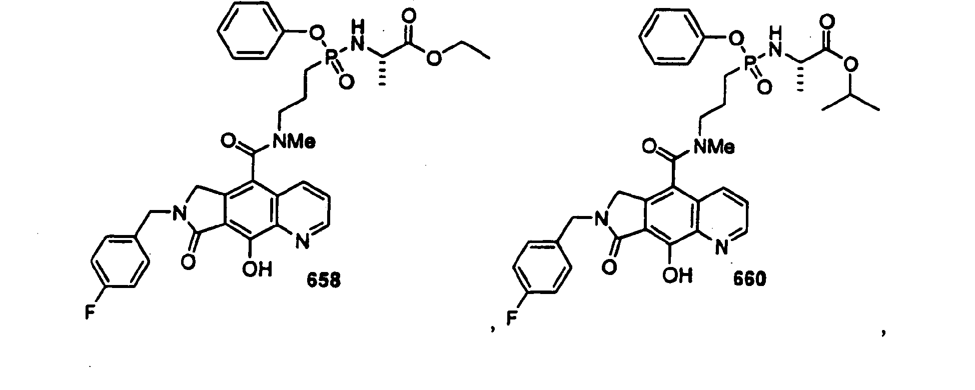

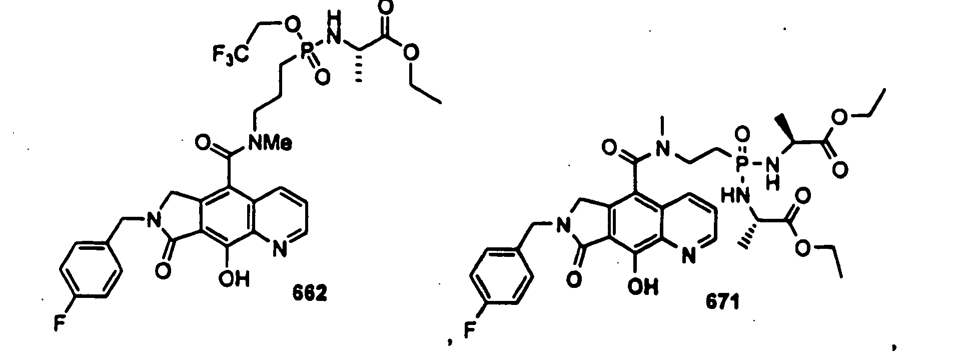

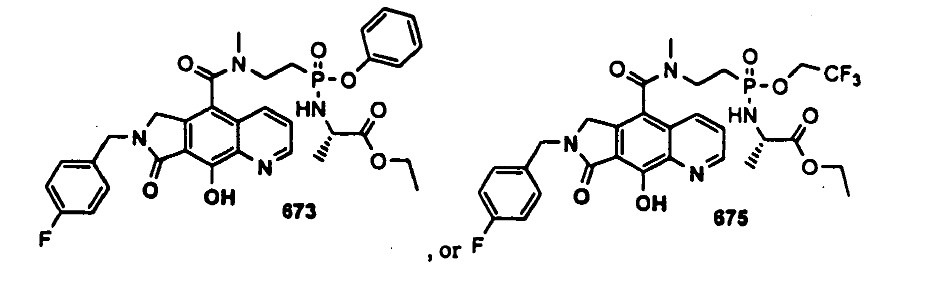

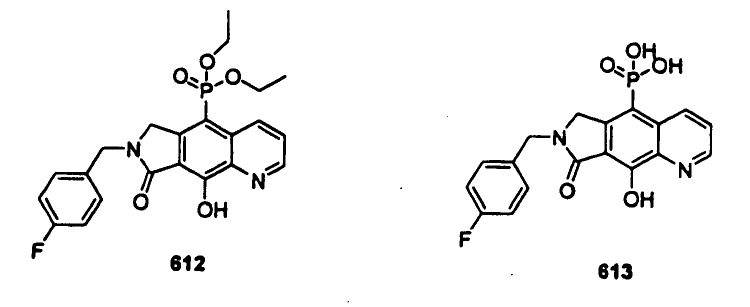

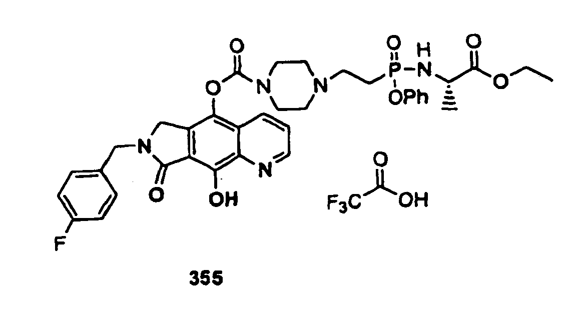

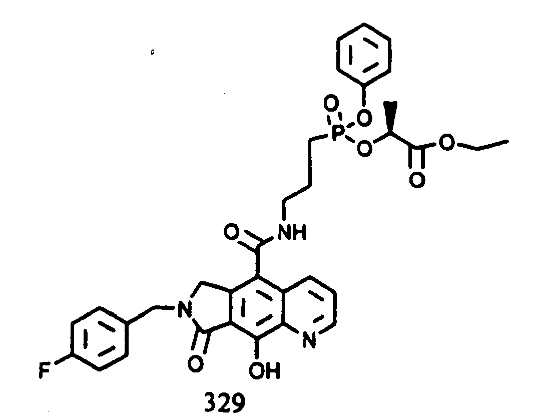

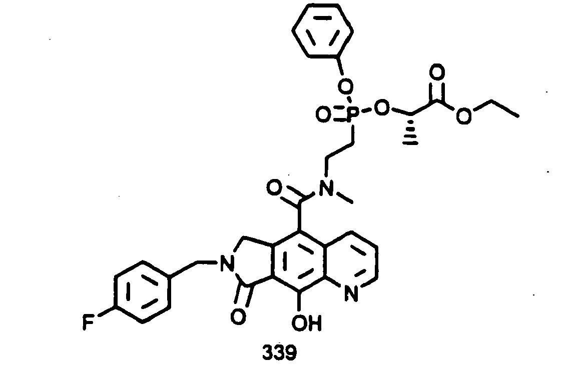

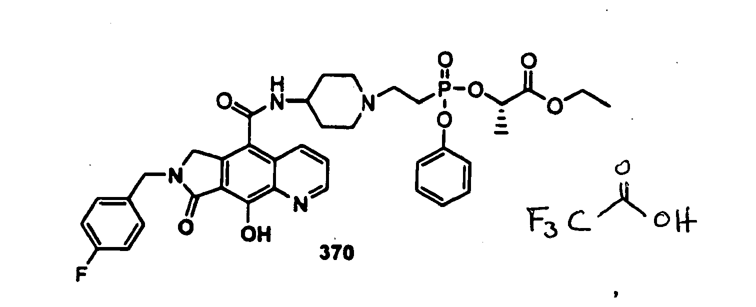

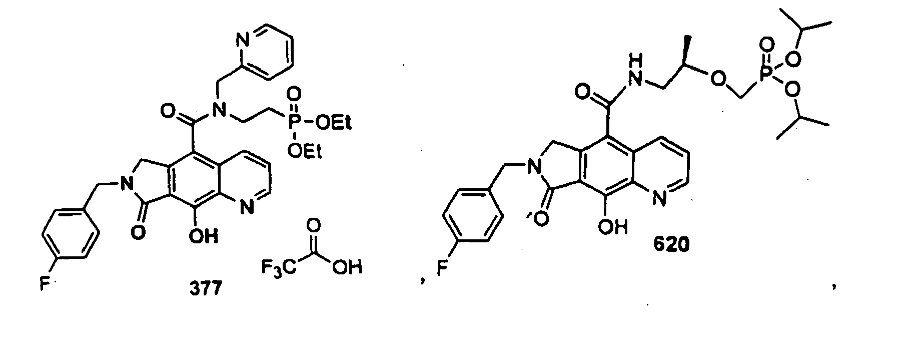

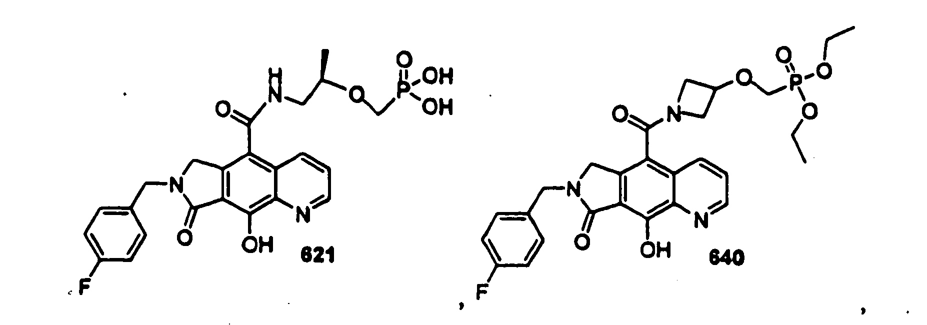

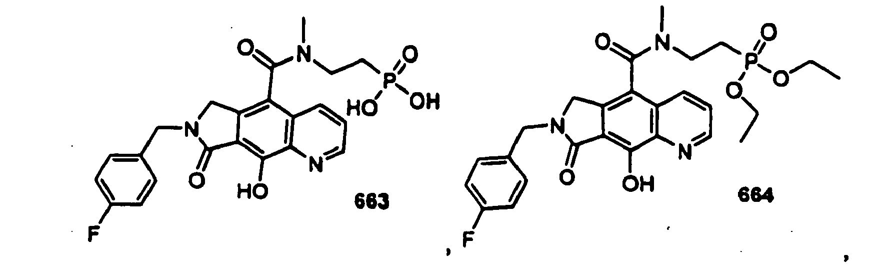

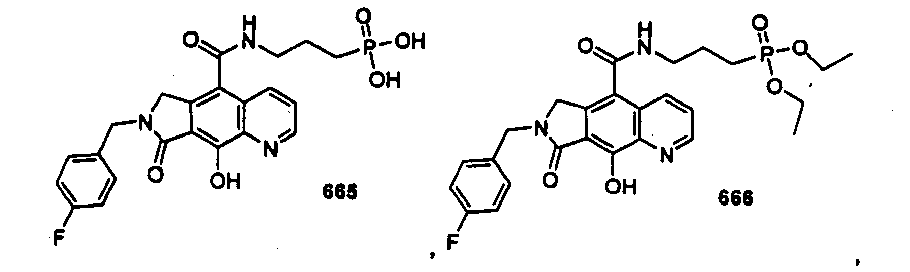

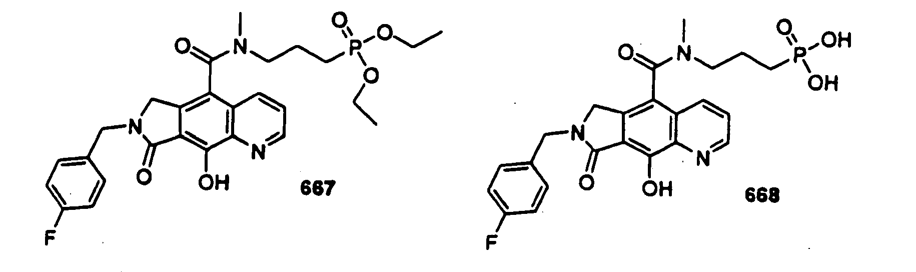

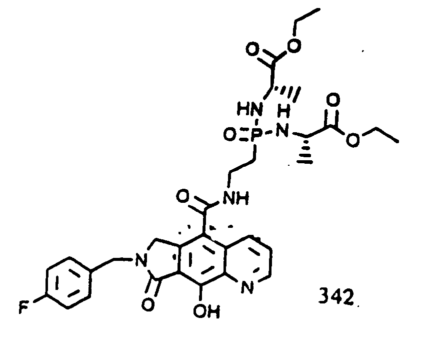

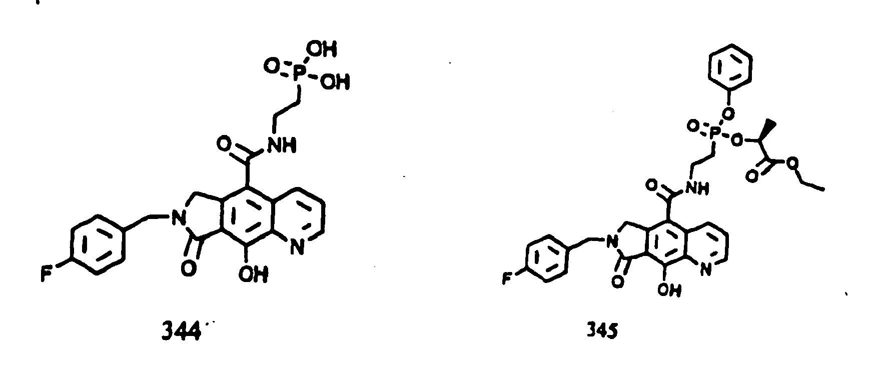

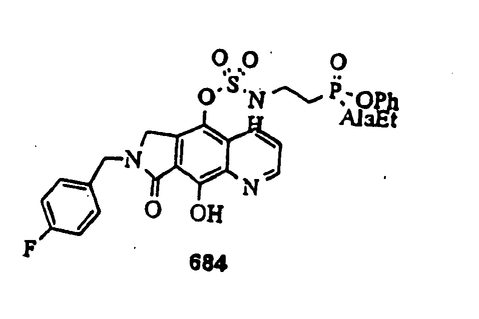

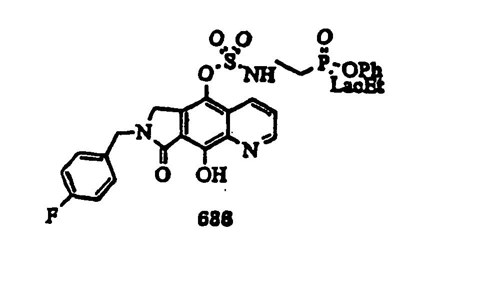

2. An HIV integrase inhibitor compound according to claim 1 which is:

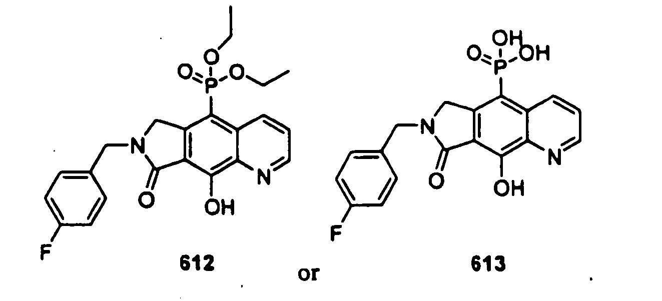

3. An HIV integrase inhibitor compound according to claim 1 that is

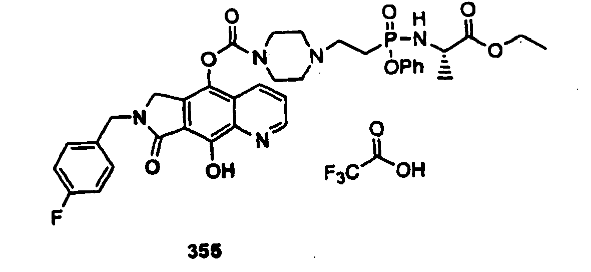

4. A pharmaceutical composition comprising an HIV integrase inhibitor compound of any one of claims 1 to 3 and a pharmaceutically acceptable carrier.

5. The pharmaceutical composition of claim 4 further comprising a therapeutically effective amount of an AIDS treatment agent selected from an HIV inhibitor agent, an anti-infective agent, and an immunomodulator.

6. The pharmaceutical composition of claim 5 wherein the HIV inhibitor agent is an HIV-protease inhibitor.

7. The composition of claim 5 wherein the HIV inhibitor agent is a nucleoside reverse transcriptase inhibitor.

8. The composition of claim 5 wherein the HIV inhibitor agent is a non-nucleoside reverse transcriptase inhibitor.

9. The use of a compound of any one of claims 1 to 3 to prepare a medicament for treatment or prophylaxis of HIV infection or for treatment of AIDS or ARC.

10. The compound of any one of claims 1 to 3 for use in medical therapy or diagnosis.

## Patentansprüche

1. HIV-Integrase-Inhibitorverbindung, ausgewählt unter

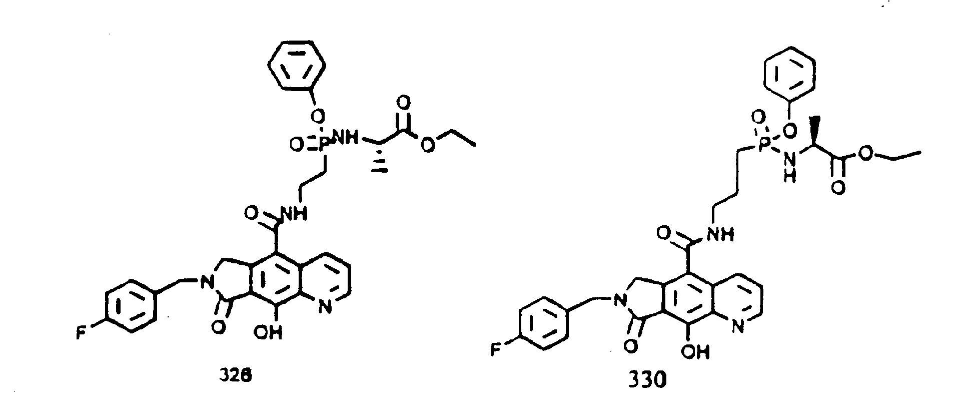

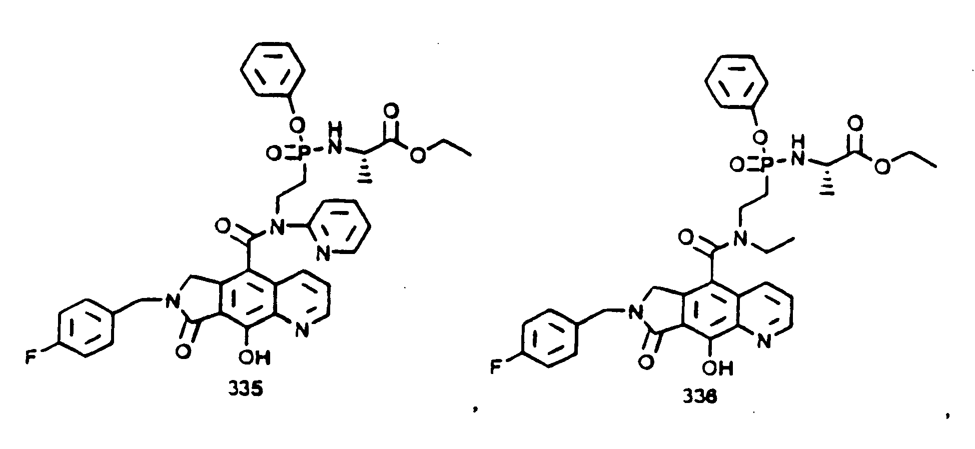

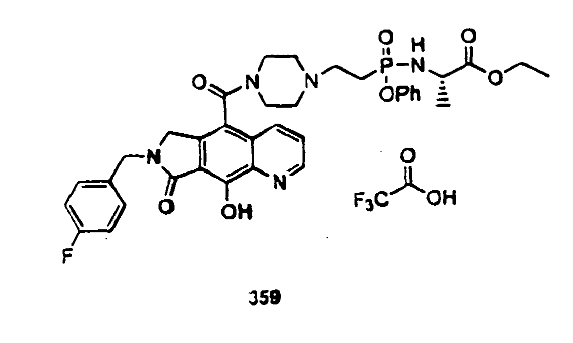

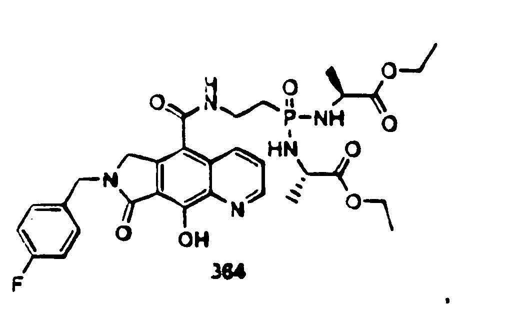

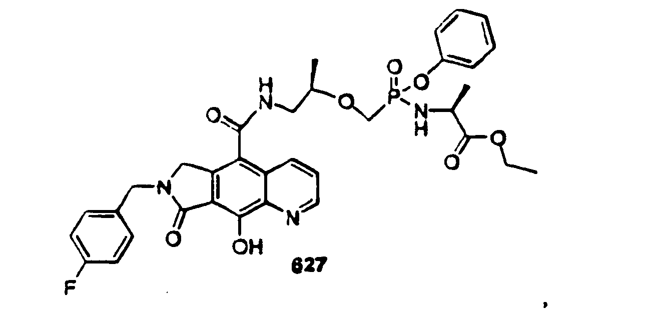

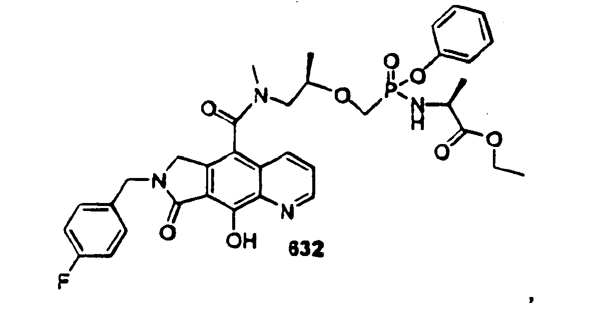

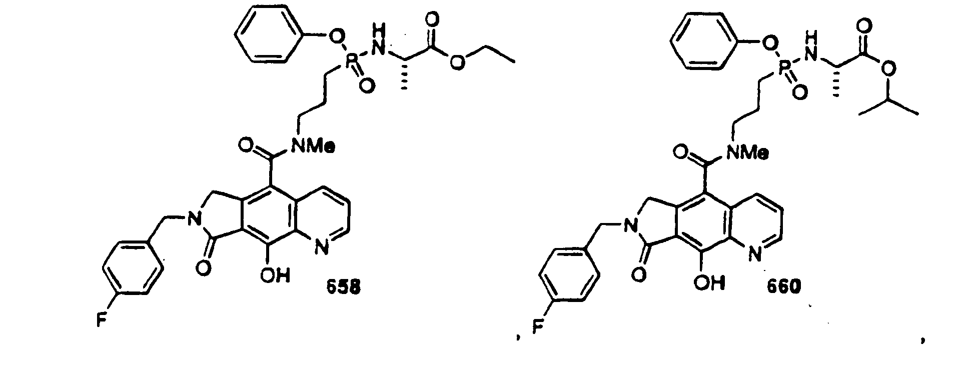

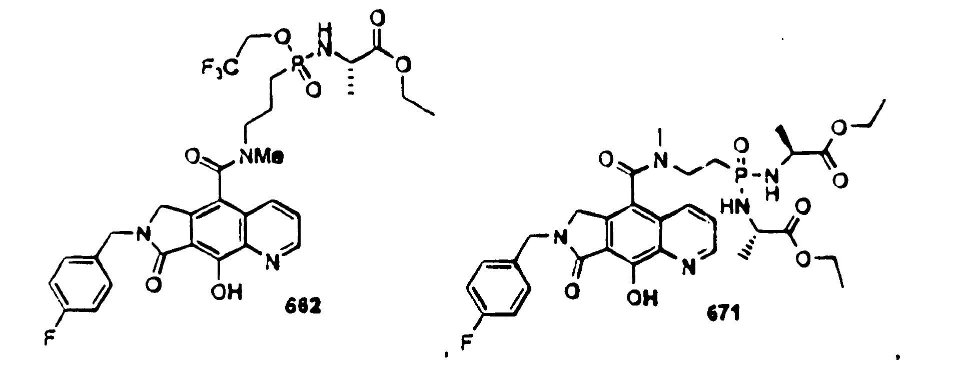

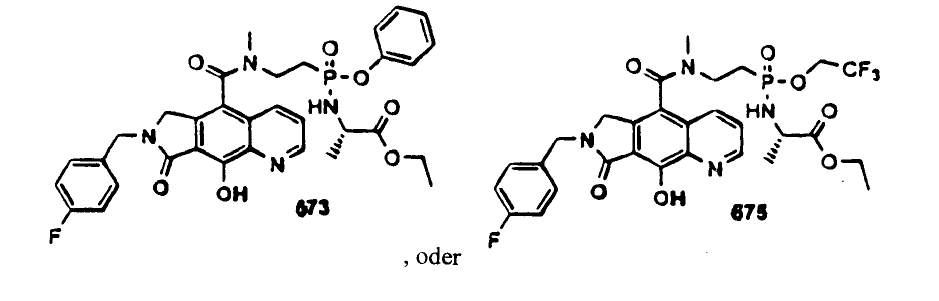

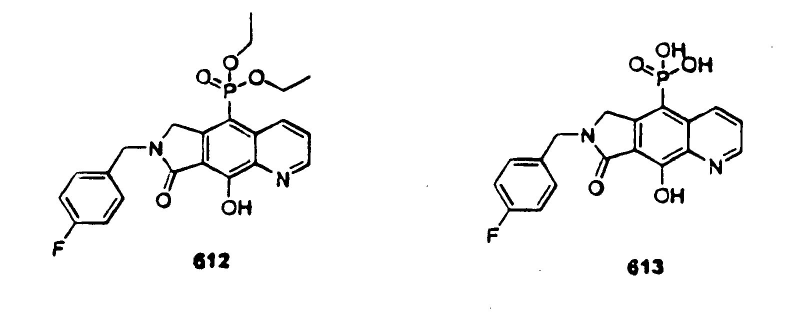

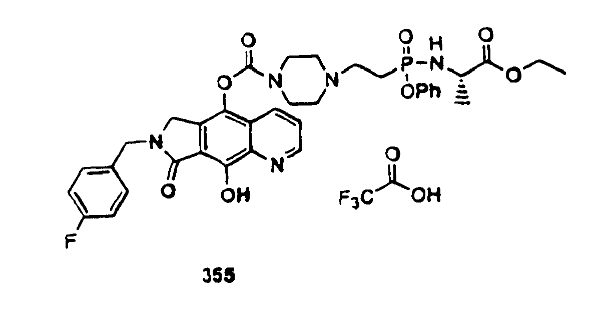

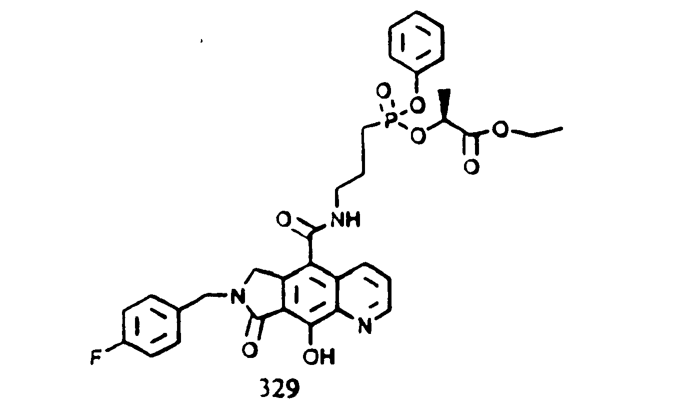

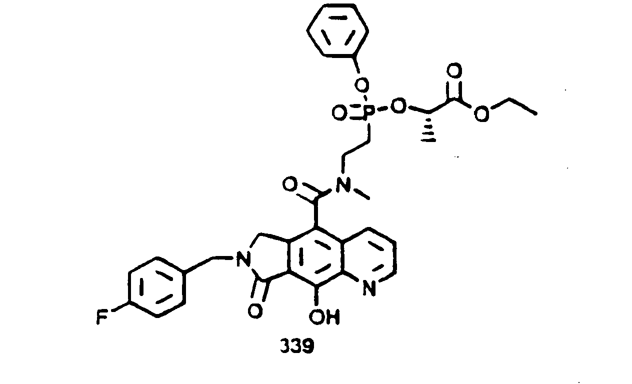

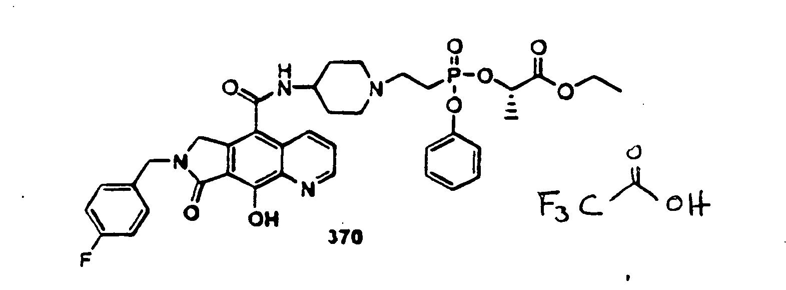

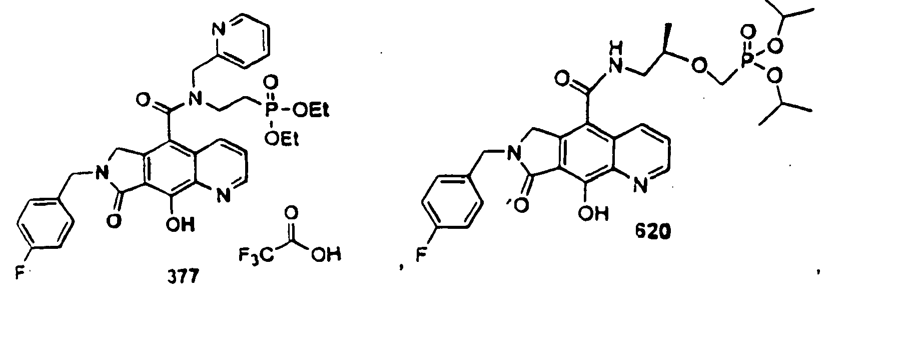

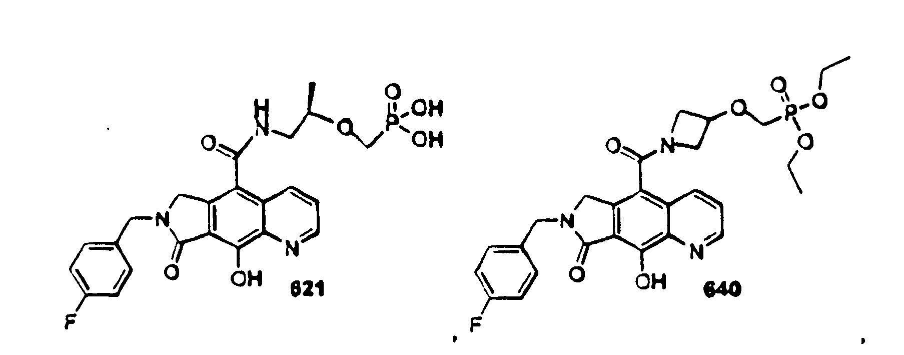

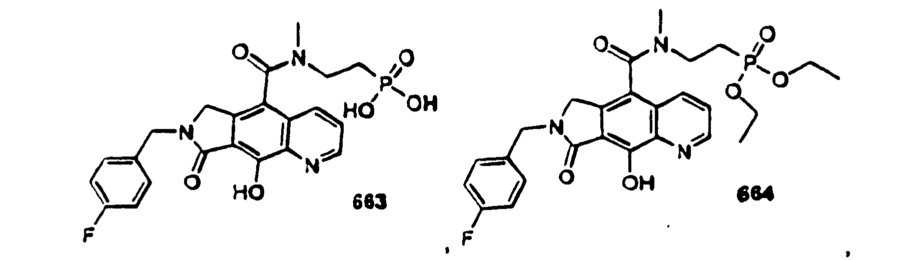

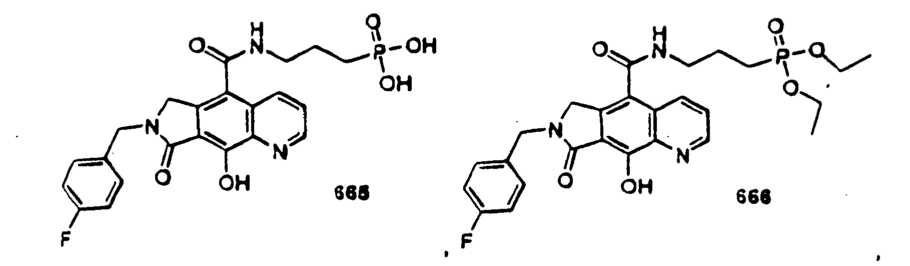

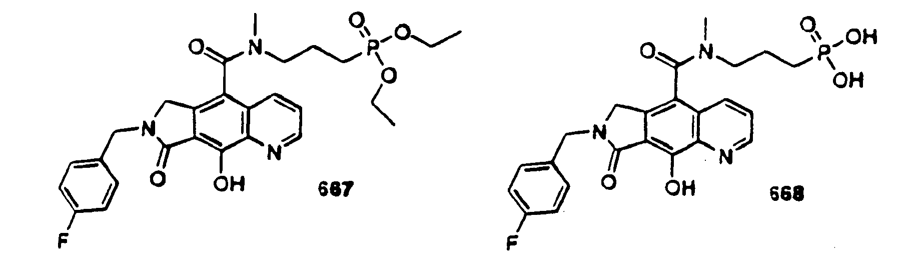

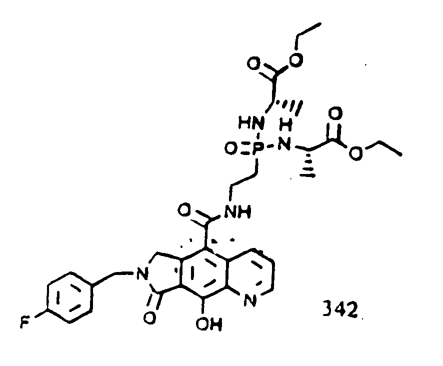

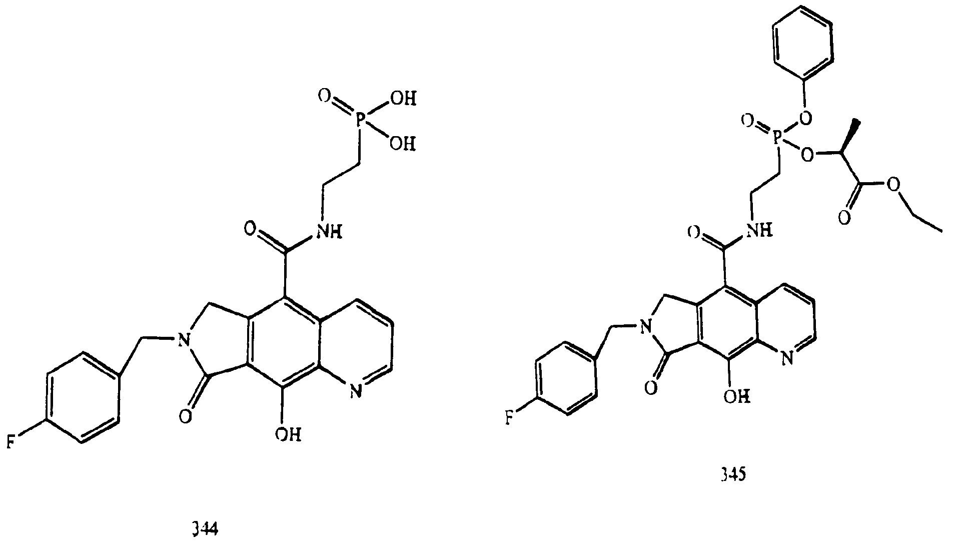

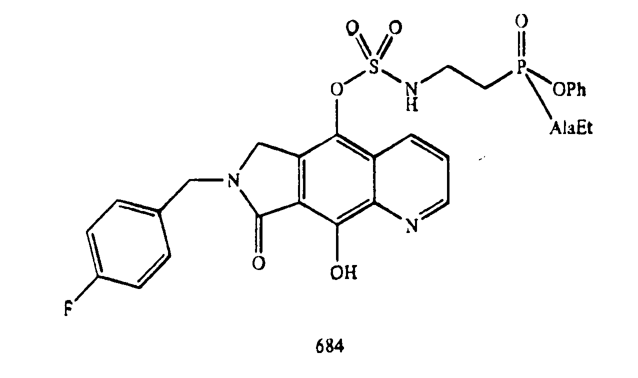

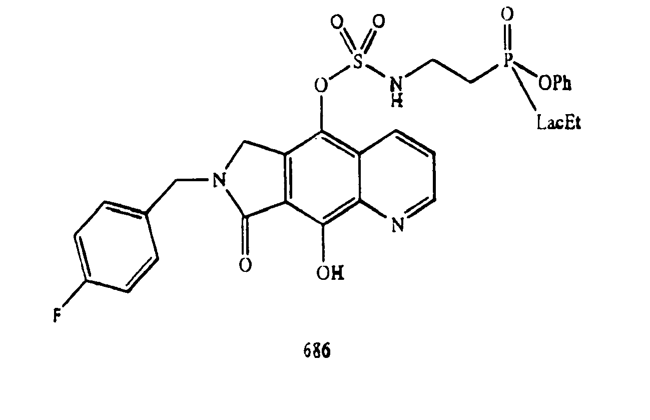

2. HIV-Integrase-Inhibitorverbindung nach Anspruch 1, nämlich

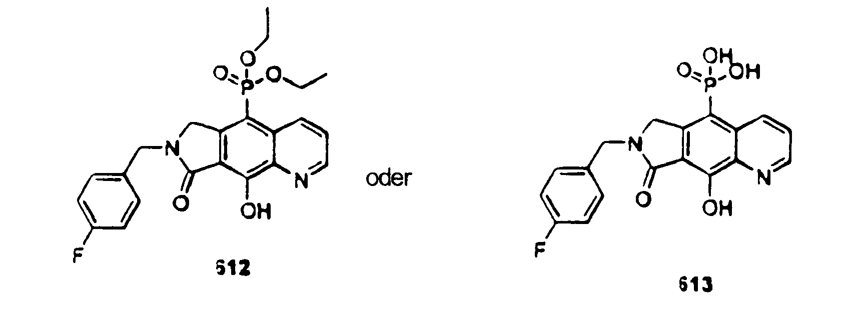

3. HIV-Integrase-Inhibitorverbindung nach Anspruch 1, nämlich

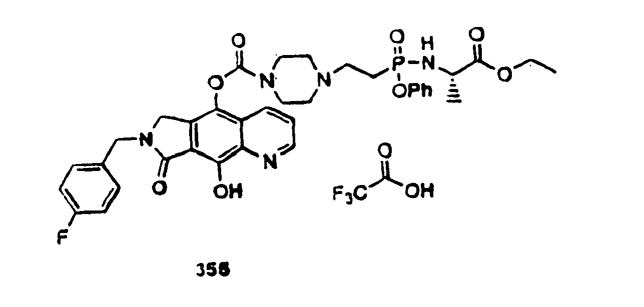

4. Pharmazeutische Zusammensetzung, umfassend eine HIV-Integrase-Inhibitorverbindung nach einem der Ansprüche 1 bis 3 und einen pharmazeutisch akzeptablen Träger.

5. Pharmazeutische Zusammensetzung nach Anspruch 4, die zusätzlich eine therapeutisch wirksame Menge eines Mittels zur Behandlung von AIDS umfasst, ausgewählt unter einem HIV-inhibitor, einem antiinfektiösen Mittel und einem Immunomodulator.

6. Pharmazeutische Zusammensetzung nach Anspruch 5, wobei es sich bei dem HIV-Inhibitor um einen HIV-Protease-Inhibitor handelt.

7. Zusammensetzung nach Anspruch 5, wobei es sich bei dem HIV-Inhibitor um einen Nukleosid-Reverse-Transkriptase-Inhibitor handelt.

8. Zusammensetzung nach Anspruch 5, wobei es sich bei dem HIV-Inhibitor um einen Nichtnukleosid-Reverse-Transkriptase-Inhibitor handelt.

9. Verwendung einer Verbindung nach einem der Ansprüche 1 bis 3 zur Herstellung eines Medikaments zur Behandlung oder Prophylaxe einer HIV-Infektion oder zur Behandlung von AIDS oder ARC.

10. Verbindung nach einem der Ansprüche 1 bis 3 zur Anwendung in der medizinischen Therapie oder Diagnose.

## Revendications

1. Composé inhibiteur de l'intégrase du VIH choisi parmi :

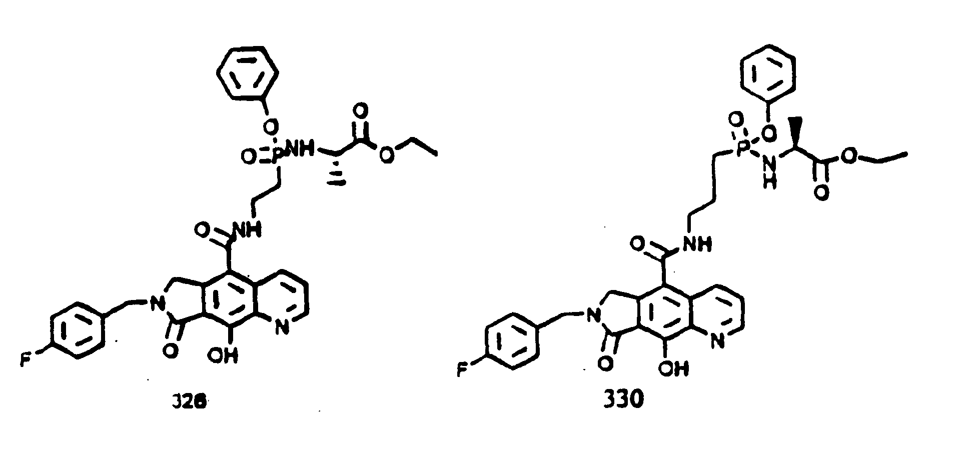

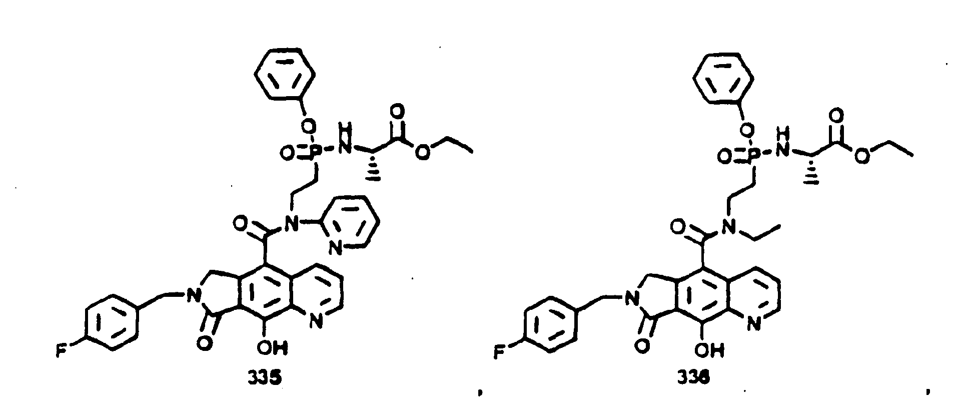

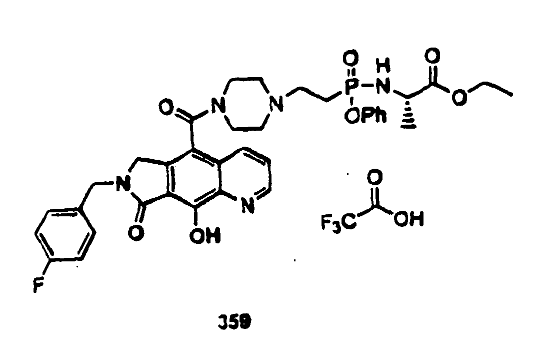

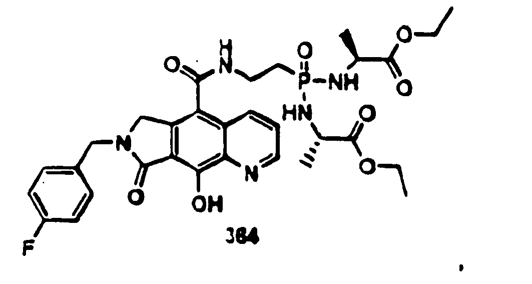

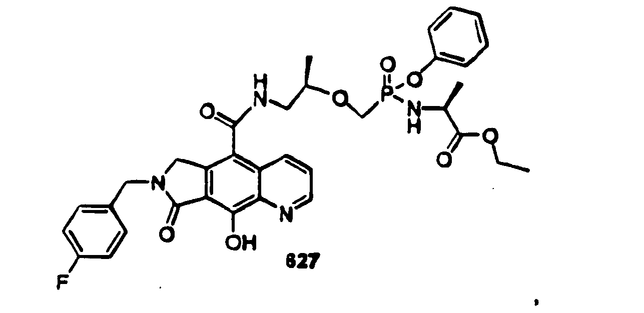

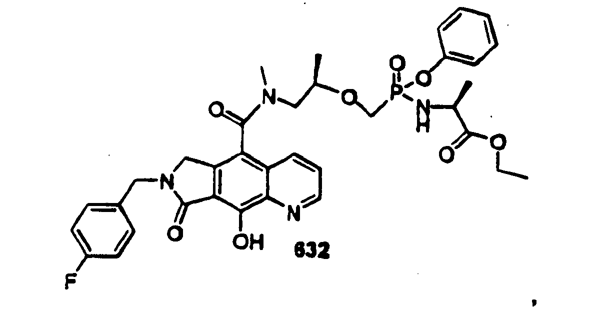

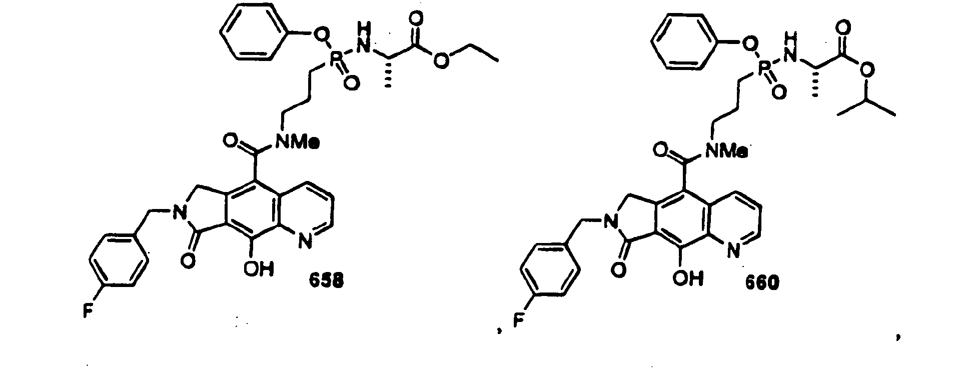

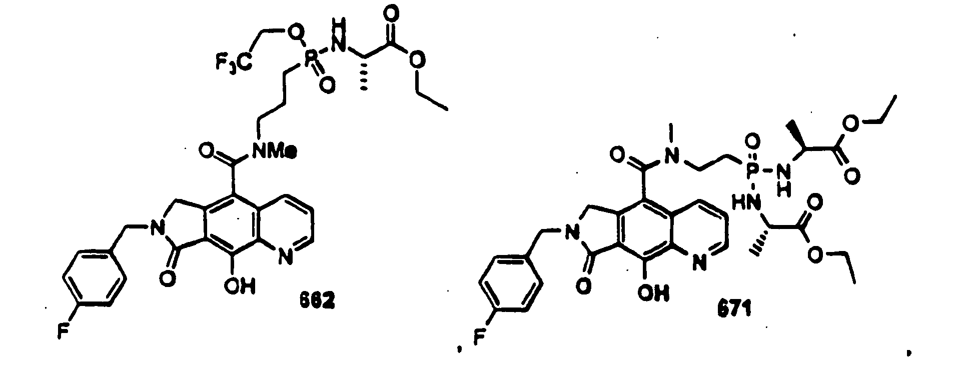

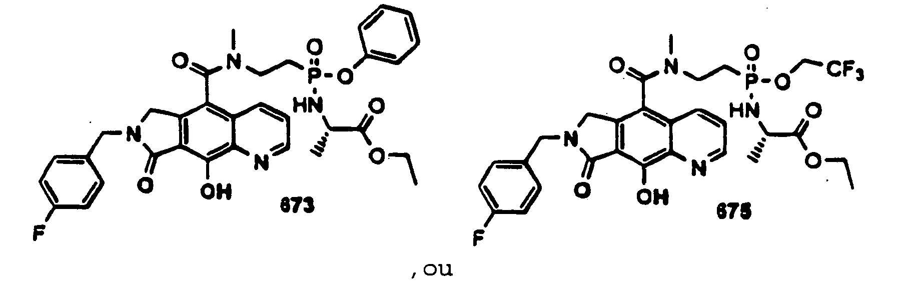

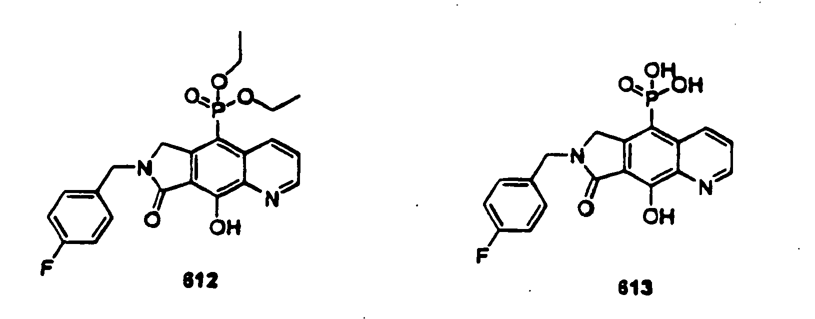

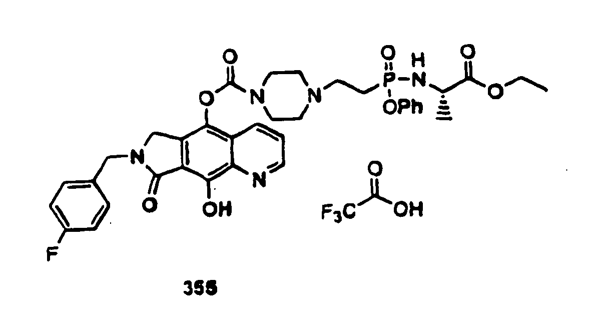

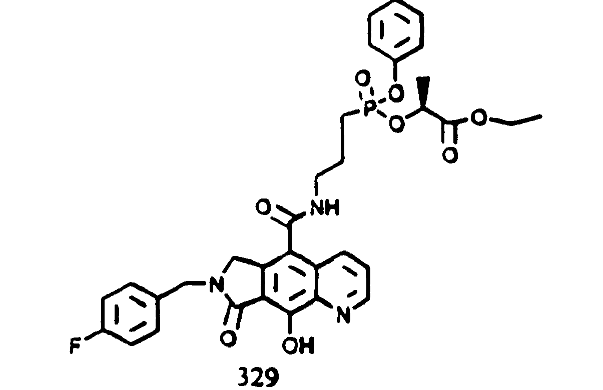

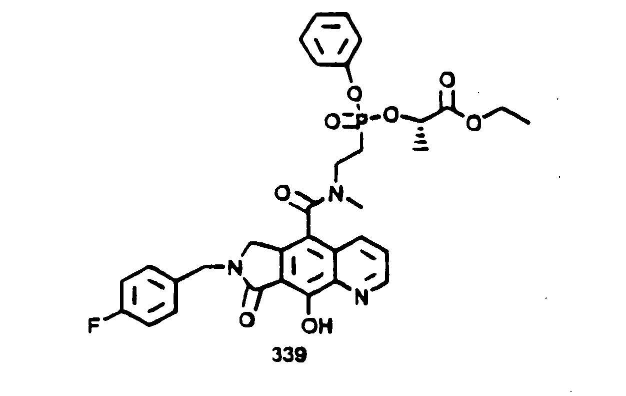

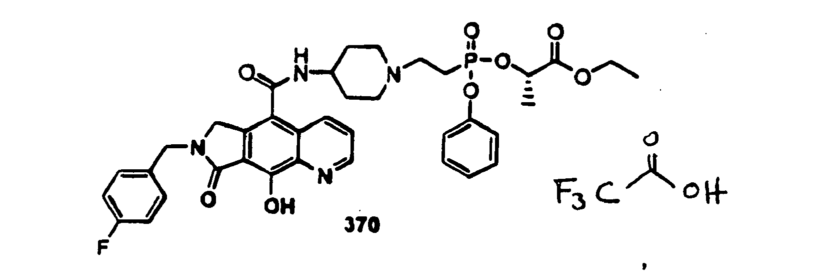

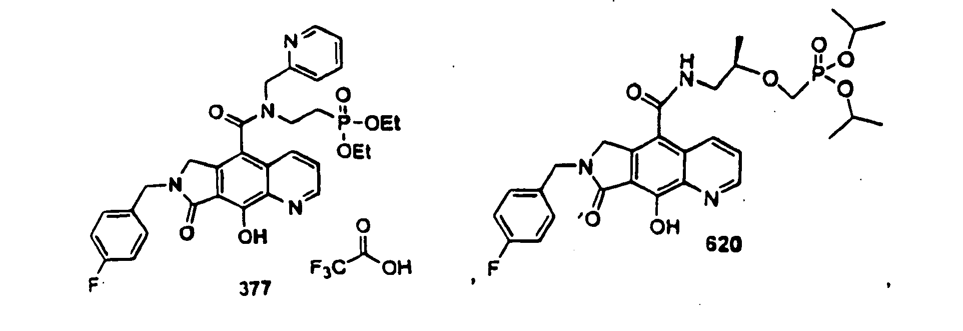

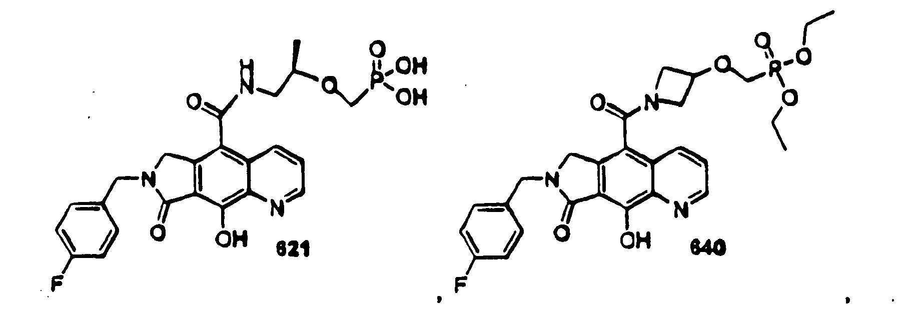

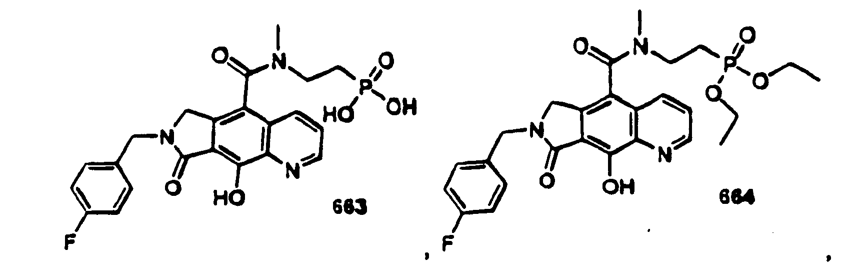

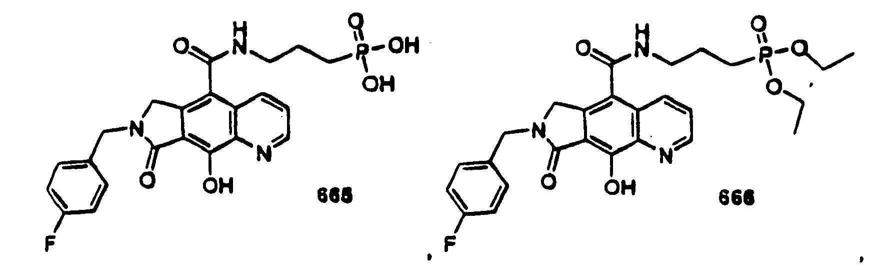

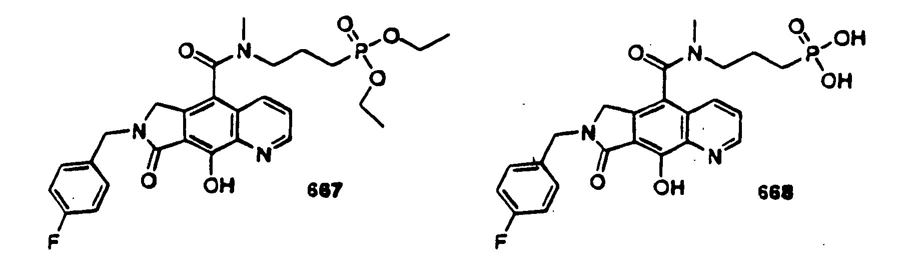

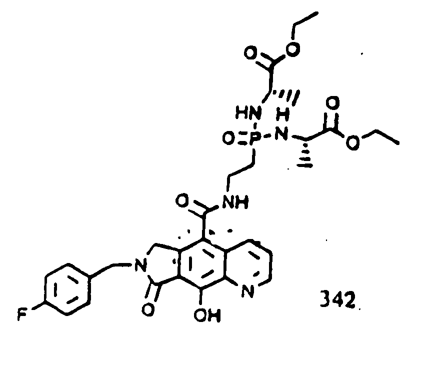

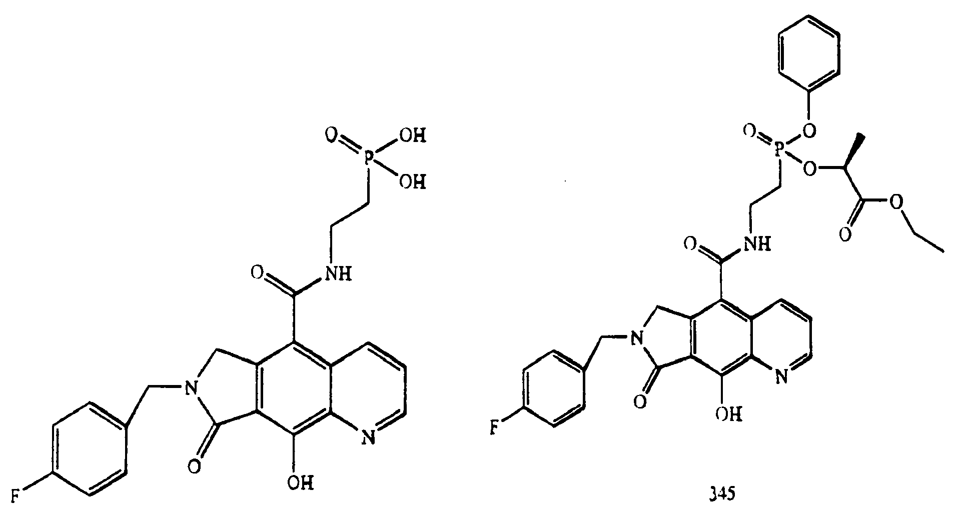

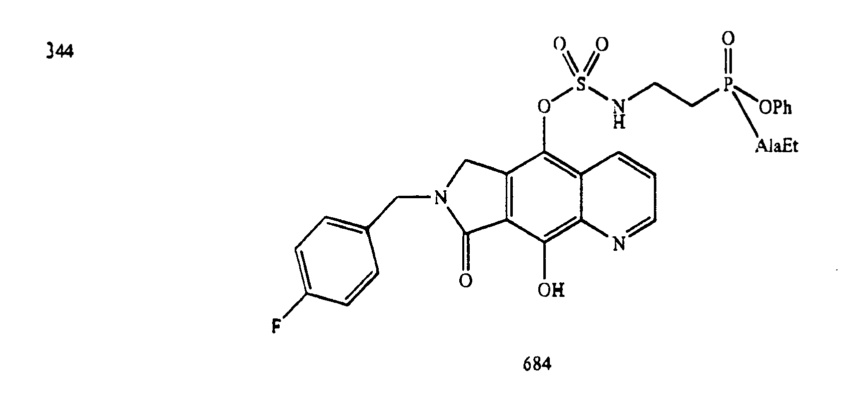

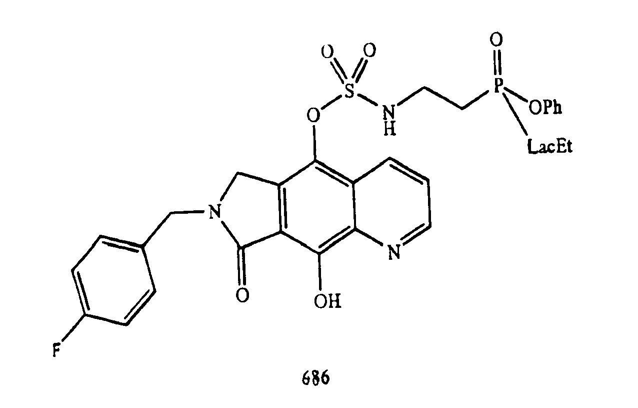

2. Composé inhibiteur de l'intégrase du VIH selon La revendication 1 qui est :

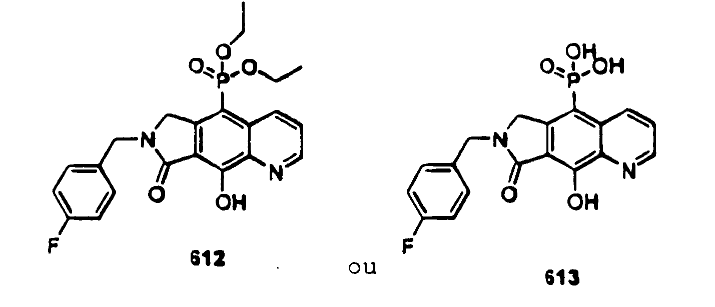

3. Composé inhibiteur de l'intégrase du VIH selon la revendication 1 qui est :

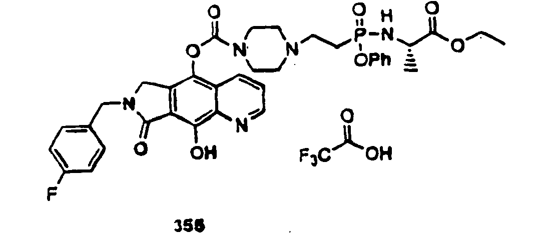

4. Composition pharmaceutique comprenant un composé inhibiteur de l'intégrase du VIH selon l'une quelconque des revendications 1 à 3 et un support pharmaceutiquement acceptable.

5. Composition pharmaceutique selon la revendication 4 comprenant en outre une quantité efficace au niveau thérapeutique d'un agent de traitement du SIDA choisi parmi un agent inhibiteur du VIH, un agent anti-infectieux et un immunomodulateur.

6. Composition pharmaceutique selon la revendication 5 dans laquelle l'agent inhibiteur du VIH est un inhibiteur de la protéase du VIH.

7. Composition selon la revendication 5 dans laquelle l'agent inhibiteur du VIH est un inhibiteur nucléosidique de transcriptase inverse.

8. Composition selon la revendication 5 dans laquelle l'agent inhibiteur du VIH est un inhibiteur non nucléosidique de transcriptase inverse.

9. Utilisation d'un composé selon l'une quelconque des revendications 1 à 3 pour préparer un médicament pour le traitement ou la prophylaxie de l'infection par le VIH ou pour le traitement du SIDA ou de l'ARC.

10. Composé selon l'une quelconque des revendications 1 à 3 pour l'utilisation dans la thérapie ou le diagnostic médical.
